(19) Europäisches Patentamt
European Patent Office
Office européen des brevets

(11) EP 0 337 731 B1

(12) EUROPEAN PATENT SPECIFICATION

(45) Date of publication and mention of the grant of the patent:
31.01.1996 Bulletin 1996/05

(51) Int Cl.[6]: **C07K 7/08**, C12P 21/04, A23K 1/16, A23K 1/17, A61K 38/00
// C12P21:04, C12R1:54

(21) Application number: 89303568.3

(22) Date of filing: 11.04.1989

# (54) Peptide antibiotics

Peptid-Antibiotika

Antibiotiques peptidiques

(84) Designated Contracting States:
AT BE CH DE ES FR GB GR IT LI NL SE

(30) Priority: 11.04.1988 US 179773
11.04.1988 US 179928
11.04.1988 US 179929
11.04.1988 US 179930

(43) Date of publication of application:
18.10.1989 Bulletin 1989/42

(73) Proprietor: ELI LILLY AND COMPANY
Indianapolis, Indiana 46285 (US)

(72) Inventors:
- Boeck, LaVerne, Dwaine
Indianapolis, Indiana 46214 (US)
- Fukuda, David Shuichi
Brownsburg, Indiana 46112 (US)
- Hoehn, Marvin Martin
Indianapolis, Indiana 46250 (US)
- Kastner, Ralph Emil
Indianapolis, Indiana 46227 (US)
- Mynderse, Jon Stuart
Indianapolis, Indiana 46208 (US)
- Papiska, Harold Rudolf
Indianapolis, Indiana 46219 (US)

(74) Representative: Tapping, Kenneth George et al
Windlesham, Surrey GU20 6PH (GB)

(56) References cited:
EP-A- 0 095 295

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 337 731 B1

## Description

This invention relates to novel lipopeptide antibiotics. In particular, it relates to a group of lipopeptides called A54145 antibiotics. The A54145 antibiotics either have, or are intermediates to compounds which have, antibiotic activity.

The A54145 antibiotics of this invention can be represented by formula 1:

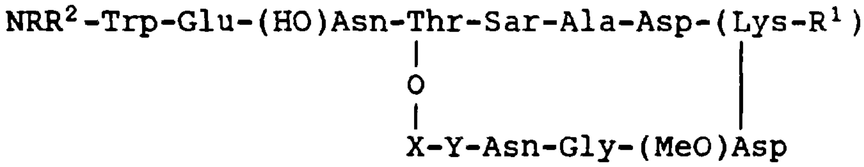

1

wherein:

- R is hydrogen, $C_8$-$C_{18}$-alkyl, optionally substituted $C_8$-$C_{18}$-alkanoyl or $C_8$-$C_{18}$-alkenoyl;
- (Lys-$R^1$) represents -$NH(CH_2)_4CH(NHR^1)CO$-;
- $R^1$ and $R^2$ are hydrogen or an amino-protecting group;
- R and $R^2$, taken together, represent a $C_8$-$C_{18}$-alkylidene group;
- X is Ile or Val; and
- Y is Glu or 3-MG;

or a salt thereof.

Background of the Invention

The A54145 antibiotics are acidic lipopeptide compounds. Previously recognized members of this class of antibiotics include the A-21978C antibiotics (see U.S. Patent 4,331,594), the A-30912 antibiotics (see U.S. Patent 4,024,245), crystallomycin, amphomycin, zaomycin, aspartocin and glumamycin [see T. Korzybski, Z. Kowszyk-Gindifer and W. Kurylowicz, "Antibiotics-Origin, Nature and Properties," Vol. I, Pergamon Press, New York, N.Y., 1967, pp. 397-401 and 404-408]; tsushimycin [J. Shoji, et al., J. Antibiotics 21, 439-443 (1968)]; laspartomycin [H. Naganawa, et al., J. Antibiotics 21, 55-62 (1968)]; brevistin [J. Shoji and T. Kato, J. Antibiotics 29, 380-389 (1976)]; cerexin A [J. Shoji, et al., J. Antibiotics 29, 1268-1274 (1976)] and cerexin B [J. Shoji and T. Kato, J. Antibiotics 29, 1275-1280 (1976)].

Although many antibacterial agents are known, the need for improved antibacterial agents continues. One reason for this need is that antibiotics differ in their effectiveness against a large variety of pathogenic organisms. A second reason is that, unfortunately, organism strains which are resistant to currently used antibiotics continually develop. Yet another reason is the fact that individual patients often suffer serious reactions to specific antibiotics, due to hypersensitivity and/or to toxic effects. There is, therefore, a continuing need for new and improved antibiotics.

In this specification, the following abbreviations, most of which are commonly known in the art, are used:

| Abbreviation | Tem |
|---|---|
| Ala: | Alanine |
| Asn: | Asparagine |
| (OH)Asn: | β-Hydroxy-asparagine |
| Asp: | Aspartic acid |
| (MeO)Asp: | β-Methoxy-aspartic acid |
| Glu: | Glutamic acid |
| Gly: | Glycine |
| Ile: | Isoleucine |
| Lys: | Lysine |
| Thr: | Threonine |
| Trp: | Tryptophan |
| Sar: | Sarcosine |

Continuation of the Table on the next page

(continued)

| Abbreviation | Tem |
|---|---|
| Val: | Valine |
| 3-MG: | 3-Methylglutamic acid |
| HPLC: | High performance liquid chromatography |
| $^1H$ NMR: | Proton nuclear magnetic resonance |
| TLC: | Thin-layer chromatography |
| IR: | Infrared |
| UV: | Ultraviolet |
| FABMS: | Fast-atom-bombardment mass spectrometry |

The term "$C_8$-$C_{18}$-alkylidenyl" refers to a group of the formula

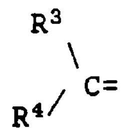

wherein $R^3$ and $R^4$ are hydrogen or an alkyl group of from 7 to 17 carbon atoms, provided that one of $R^3$ and $R^4$ must be other than hydrogen and further provided that the sum of the carbon atoms in $R^3$ and $R^4$ must be no greater than 17. Those compounds wherein R and $R^2$ represent $C_8$-$C_{18}$-alkylidenyl are known as Schiff's bases.

The term "$C_8$-$C_{18}$-alkyl" refers to a univalent saturated, straight- or branched-chain alkyl group containing from 8 to 18 carbon atoms. Those compounds wherein R is $C_8$-$C_{18}$-alkyl, referred to herein as "reduced Schiff's bases", are prepared by reduction of the corresponding compounds where R and $R^2$ represent a $C_8$-$C_{18}$-alkylidenyl group.

The term "optionally substituted $C_8$-$C_{18}$-alkanoyl and $C_8$-C-$_{18}$-alkenoyl" refer to acyl groups derived from carboxylic acids containing from 8 to 18 carbon atoms. When the R group is alkanoyl, the alkyl portion is a univalent, saturated, straight-chain or branched-chain hydrocarbon radical which can optionally bear one hydroxyl, carboxyl, or $C_1$-$C_3$-alkoxy group or from one to three halo substitutents selected from chlorine, bromine, and fluorine. When R is alkenoyl, the alkenyl portion is a univalent, unsaturated, straight-chain or branched-chain hydrocarbon radical containing not more than three double bonds. The double bond portion(s) of the unsaturated hydrocarbon chain may be either in the cis or trans configuration.

The term "amino-protecting group" refers to an art-recognized amino-protecting group which is compatible with the other functional groups in the A54145 molecule. Preferred amino-protecting groups are those which can be readily removed subsequently. Examples of suitable protecting groups can be found in "Protective Groups in Organic Synthesis" by Theodora W. Greene, John Wiley and Sons, N.Y. 1981, Chapter 7. Especially preferable amino-protecting groups are the tert-butoxycarbonyl (t-BOC) and benzyloxycarbonyl groups.

One group of the A54145 antibiotics of formula 1 are produced by submerged aerobic fermentation of a culture selected from Streptomyces fradiae strains NRRL 18158, NRRL 18159 and NRRL 18160. This group of compounds, which are called A54145 components, are the starting materials from which the other formula 1 compounds are prepared.

Eight A54145 components have been isolated from the naturally produced mixture, which is called antibiotic A54145. The A54145 components are major components A and $B_1$ and minor components B, C, D, E, F and $A_1$.

The A54145 components have general structure 2 and specific structures 2a-2h

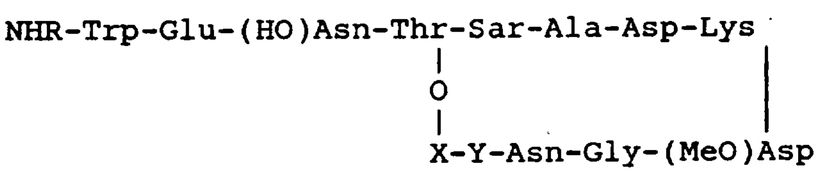

2

| Structure | Component | X | Y | R |
|---|---|---|---|---|
| 2a | A | Ile | Glu | 8-methylnonanoyl |

Continuation of the Table on the next page

(continued)

| Structure | Component | X | Y | R |
|---|---|---|---|---|
| 2b | B | Ile | 3-MG | n-decanoyl |
| 2c | C | Val | 3-MG | 8-methyldecanoyl |
| 2d | D | Ile | Glu | " |
| 2e | E | Ile | 3-MG | " |
| 2f | F | Val | Glu | 8-methylnonanoyl |
| 2g | $A_1$ | Ile | Glu | n-decanoyl |
| 2h | $B_1$ | Ile | 3-MG | 8-methylnonanoyl |

A second group of formula 1 compounds are prepared by enzymatically removing the fatty acid side chains of the A54145 antibiotics (the R group) to give the cyclic peptide unit ("nucleus"). For convenience, this cyclic peptide is called an A54145 nucleus. Thus far, four unique A54145 nuclei have been obtained. These nuclei, which have been designated the A54145A, A54145B, A54145C and A54145F nuclei, have the common structure 3 and individual structures 3a-3d:

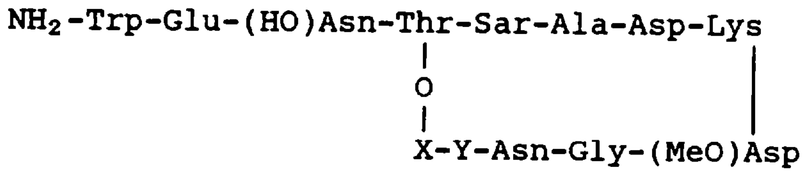

3

| A54145 | | | |
|---|---|---|---|
| Structure | Nucleus | X | Y |
| 3a | A | Ile | Glu |
| 3b | B | Ile | 3-MG |
| 3c | C | Val | 3-MG |
| 3d | F | Val | Glu |

The A54145 nuclei and blocked A54145 components and nuclei, which are called A54145 cyclic peptides, can be represented by formula 4:

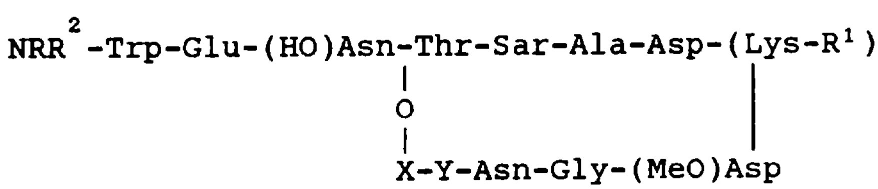

4

wherein:

R is selected from the group consisting of hydrogen, an amino-protecting group, 8-methylnonanoyl, 8-methyldecanoyl and n-decanoyl;
(Lys-$R^1$) represents $-NH(CH_2)_4CH(NHR^1)CO-$;
$R^1$ is hydrogen or an amino-protecting group;
$R^2$ is hydrogen;
X is Ile or Val; and
Y is Glu or 3-MG;

provided that:

1) when $R^1$ = H, X = Ile and Y = Glu or 3-MG, R cannot be 8-methylnonanoyl, 8-methyldecanoyl or n-decanoyl;
2) when $R^1$ = H, X = Val and Y = 3-MG, R cannot be 8-methyldecanoyl; and
3) when $R^1$ = H, X = Val and Y = Glu, R cannot be 8-methylnonanoyl.

The compounds of formula 4 wherein R is other than hydrogen or an amino-protecting group and $R^1$ is an amino-protecting group (4a compounds) are called "blocked" A54145 components.

A formula 4 compound wherein R and $R^1$ differ but are selected from hydrogen or an amino-protecting group (a formula 4b compound) is called a "blocked nucleus".

A formula 4 compound wherein R and $R^1$ are both hydrogen (a formula 4c compound) is called a "nucleus".

The formula 4 compounds are useful intermediates to the remaining group of formula 1 compounds. These compounds are prepared by acylating an A54145 nucleus (a 4c compound) or a blocked A54145 nucleus (a 4b compound). This group of formula 1 compounds have the chemical structure depicted in formula 5:

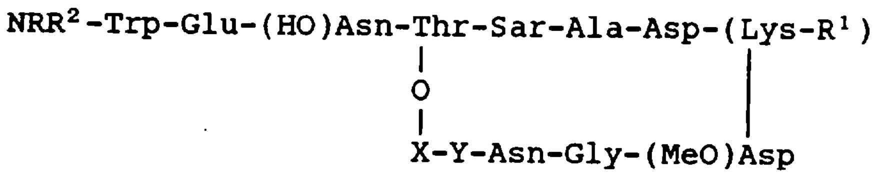

5

wherein:

R is $C_8$-$C_{18}$-alkyl, optionally substituted $C_8$-$C_{18}$-alkanoyl or $C_8$-$C_{18}$-alkenoyl;
(Lys-$R^1$) represents $-NH(CH_2)_4CH(NHR^1)CO-$;
$R^1$ is hydrogen or an amino-protecting group; and
$R^2$ is hydrogen; or
R and $R^2$, taken together, represent a $C_8$-$C_{18}$-alkylidene group;
X is Ile or Val; and
Y is Glu or 3-MG;

provided that: 1) when $R^1$ = H, X = Ile and Y = Glu or 3-MG, R cannot be 8-methylnonanoyl, 8-methyldecanoyl or n-decanoyl; 2) when $R^1$ = H, X = Val and Y = 3-MG, R cannot be 8-methyldecanoyl; and 3) when $R^1$ = H, X = Val and Y = Glu, R cannot be 8-methylnonanoyl.

Subgeneric aspects of the formula 5 compounds include the following:

(a) The compounds wherein R is alkanoyl of the formula,

$$CH_3(CH_2)_n-\overset{O}{\overset{\|}{C}},$$

wherein n is an integer from 6 to 14, provided that, when X is Ile, n cannot be 8.
(b) The (a) compounds wherein n is 6 to 10;
(c) The compounds wherein R is alkanoyl of the formula

$$CH_3(CH_2)_n\overset{CH_3}{\overset{|}{C}}H(CH_2)_m-\overset{O}{\overset{\|}{C}}-,$$

wherein n and m are each, independently, an integer from 0 to 14, provided that 1) n + m must be no less than 4 and no greater than 14; and 2) m cannot be 6 when: a) n = O and either (i) X = Ile or (ii) X = Val and Y = Glu; or b) n = 1 and either (i) X = Ile or (ii) X = Val and Y = 3-MG.
(d) The compounds wherein R is cis or trans alkenyl of the formula

$$CH_3(CH_2)_nCH{=}CH(CH_2)_m-\overset{O}{\overset{\|}{C}}-$$

wherein n and m are each, independently, an integer from 0 to 14, provided that n + m must be no less than 4 and no greater than 14;

(e) The compounds wherein R is cis or trans alkenyl of the formula

$$CH_2{=}CH(CH_2)_n\overset{O}{\overset{\|}{C}}-$$

wherein n is an integer of from 5 to 15;

(f) The compounds wherein R is alkyl of the formula $CH_3(CH_2)_n$- and n is an integer from 7 to 12; and

(g) The (a) compounds wherein R is:

$$CH_3(CH_2)_6-\overset{O}{\overset{\|}{C}}-$$

$$CH_3(CH_2)_7-\overset{O}{\overset{\|}{C}}-$$

$$CH_3(CH_2)_8-\overset{O}{\overset{\|}{C}}-$$

$$CH_3(CH_2)_9-\overset{O}{\overset{\|}{C}}-$$

$$CH_3(CH_2)_{10}-\overset{O}{\overset{\|}{C}}-$$

$$CH_2{=}CH-(CH_2)_8-\overset{O}{\overset{\|}{C}}-$$

$$CH_3(CH_2)_{11}-\overset{O}{\overset{\|}{C}}-$$

$$CH_3(CH_2)_{12}-\overset{O}{\overset{\|}{C}}-$$

$$CH_3(CH_2)_{11}-$$

$$CH_3(CH_2)_9-$$

(h) the compounds wherein R and $R_2$ represent:

$$CH_3(CH_2)_{10}CH{=}$$

$$CH_3(CH_2)_{12}CH{=}$$

$CH_3(CH_2)_8CH=$.

Table I lists illustrative formula 5a compounds of this invention.

**Table I: Formula 5a Derivatives[a]**

| Compound No. | X | Y | R | $R^1$ |
|---|---|---|---|---|
| 1 | Ile | Glu | n-undecanoyl | t-BOC |
| 2 | Ile | Glu | n-undecanoyl | H |
| 3 | Ile | Glu | n-dodecanoyl | t-BOC |
| 4 | Ile | Glu | n-dodecanoyl | H |
| 5 | Ile | Glu | n-nonanoyl | t-BOC |
| 6 | Ile | Glu | n-nonanoyl | H |
| 7 | Ile | Glu | n-undec-10-enoyl | t-BOC |
| 8 | Ile | Glu | n-undec-10-enoyl | H |
| 9 | Val | Glu | n-decanoyl | t-BOC |
| 10 | Val | Glu | n-decanoyl | H |
| 11 | Val | Glu | 8-methyldecanoyl | CBz |
| 12 | Val | Glu | 8-methyldecanoyl | H |
| 13 | Val | 3-MG | n-decanoyl | t-BOC |
| 14 | Val | 3-MG | n-decanoyl | H |
| 15 | Ile | 3-MG | n-tetradecanoyl | t-BOC |
| 16 | Ile | 3-MG | n-tetradecanoyl | H |
| 17 | Val | 3-MG | n-octanoyl | t-BOC |
| 18 | Val | 3-MG | n-octanoyl | H |
| 19 | IIe | Glu | decyl | H |
| 20 | Ile | 3-MG | n-undec-10-enoyl | t-BOC |
| 21 | Val | Glu | 8-ethyldecanoyl | H |
| 22 | Val | Glu | 8-methoxy-decanoyl | H |
| 23 | Ile | Glu | 7-methyloctanoyl | t-BOC |
| 24 | Ile | Glu | 7-methyloctanoyl | H |
| 25 | Ile | Glu | n-(tetradecanoyl) | t-BOC |
| 26 | Ile | Glu | n-(tetradecanoyl) | H |

[a]$R^2 = H$

The formula 1 compounds have both carboxyl and amino groups which can form salts such as alkali-metal, alkaline-earth-metal, amine and acid addition salts. Partial, mixed and complete salts are, therefore, contemplated as part of this invention. Such salts are useful, for example, for separating and purifying the compounds.

Representative alkali-metal and alkaline-earth-metal salts of the formula 1 compounds include the sodium, potassium, lithium, cesium, rubidium, barium, calcium and magnesium salts.

The alkali-metal and alkaline-earth-metal cationic salts of the formula 1 compounds are prepared according to procedures commonly used for the preparation of cationic salts. For example, the free acid form of the compound is dissolved in a suitable solvent such as warm methanol or ethanol; a solution containing the stoichiometric quantity of the desired inorganic base in aqueous methanol is added to this solution. The salt thus formed can be isolated by routine methods, such as filtration or evaporation of the solvent.

Suitable amine salts of the formula 1 compounds include the ammonium and the primary, secondary, and tertiary $C_1$-$C_4$-alkylammonium and hydroxy-$C_2$-$C_4$-alkyl-ammonium salts. Illustrative amine salts include those formed by reaction of a formula 1 compound with ammonium hydroxide, methylamine, sec-butylamine, isopropylamine, diethylamine, di-isopropylamine, ethanolamine, triethylamine, 3-amino-1-propanol and the like.

The salts formed with organic amines can be prepared in a similar manner. For example, the gaseous or liquid amine can be added to a solution of the compound in a suitable solvent such as acetone; the solvent and excess amine can be removed by evaporation.

Representative and suitable acid-addition salts of the formula 1 compounds include those salts formed by standard

reaction with both organic and inorganic acids such as, for example, hydrochloric, sulfuric, phosphoric, acetic, succinic, citric, lactic, maleic, fumaric, palmitic, cholic, pamoic, mucic, D-glutamic, d-camphoric, glutaric, glycolic, phthalic, tartaric, lauric, stearic, salicyclic, methanesulfonic, benzenesulfonic, sorbic, picric, benzoic, cinnamic and like acids.

Pharmaceutically acceptable salts are especially useful. "Pharmaceutically acceptable" salts are those in which the toxicity of the compound as a whole toward warm-blooded animals is not increased relative to the non-salt form. Pharmaceutically acceptable alkali-metal, alkaline-earth-metal and amine salts and acid-addition salts are particularly useful compounds of this invention.

Preparation of the Formula 1 Compounds

A. The Starting A54145 Components (Formula 2 Group)

The first group of formula 1 compounds to be prepared are the A54145 components. These components are produced by an A54145-producing strain of Streptomyces fradiae under submerged aerobic conditions in a suitable culture medium. Cultures of three A54145-producing Streptomyces fradiae strains have been deposited and made part of the stock culture collection of the Midwest Area Northern Regional Research Center, Agricultural Research Service, U.S. Department of Agriculture, 1815 North University Street, Peoria, Illinois 61604, from which they are available to the public under the accession numbers NRRL 18158, NRRL 18159 and NRRL 18160.

The Streptomyces fradiae strain NRRL 18158 is a spontaneous mutant which was biologically purified from a culture isolated from a soil sample from Mexico. For convenience in describing this S. fradiae strain, it is called the A54145.1 culture. S. fradiae strains NRRL 18159 and NRRL 18160 are induced mutant strains obtained from the A54145.1 culture. For convenience, the NRRL 18159 and NRRL 18160 strains are called the A54145.2 and A54145.3 cultures, respectively.

Taxonomic studies of the A54145.1, A54145.2 and A54145.3 cultures were carried out by Frederick P. Mertz of the Lilly Research Laboratories. Based on these studies, the A54145.1 organism is classified as a strain of Streptomyces fradiae (Waksman and Curtis 1916) Waksman and Henrici 1948. This classification is based on direct laboratory comparisons and an examination of published descriptions of this species [E. B. Shirling and D. Gottlieb, "Cooperative Description of Type Strains of Streptomyces", Int. J. Syst. Bacteriol. 18(2):69-189 (1968); and S. A. Waksman, "The Actinomycetes Vol. II", The Williams and Wilkins Co., Baltimore, 1961].

Methods Used

The methods recommended by the International Streptomyces Project (ISP) for the characterization of Streptomyces species [E. B. Shirling and D. Gottlieb, "Methods for Characterization of Streptomyces Species", Int. J. Syst. Bacteriol. 16(3), 313-340 (1966)] have been followed along with certain supplementary tests (D. J. Blazevic and G. M. Ederer, "Principles of Biochemical Tests in Diagnostic Microbiology", John Wiley and Sons, Inc., New York, 1975, p. 136).

Carbon utilization was determined on ISP No. 9 basal medium to which filter-sterilized carbon sources were added to equal a final concentration of 1.0 percent. Plates were incubated at 30°C and read after 14 days.

Melanoid pigment production (chromogenicity) was determined with ISP No. 1 (tryptone-yeast extract broth), ISP No. 6 (peptone-yeast extract-iron agar), ISP No. 7 (tyrosine agar) and modified ISP No. 7 which has tyrosine removed.

Starch hydrolysis was determined by testing for the presence of starch with iodine on ISP No. 4 (inorganic salts-starch agar) plates (see Blazevic and Ederer, supra).

Morphology was studied using an optical light microscope. A scanning electron microscope (SEM) was used to study the spore-surface ornamentation.

NaC1 tolerance was measured by adding NaCl to ISP No. 2 agar to equal the concentration desired.

ICSS-NBS Centroid Color Charts, standard sample No. 2106 (National Bureau of Standards, 1958, U. S. Department of Commerce, Washington, D.C.) and the Color Harmony Manual (4th ed., Color Standards Department, Container Corporation of America, Chicago, Illinois, 1958) were used to assign color names.

The isomer of diaminopimelic acid (DAP) and the carbohydrates in hydrolysates of whole cells were established by the chromatographic methods of Becker et al. [B. Becker, M. P. Lechevalier, R. E. Gordon, and H. A. Lechevalier, "Rapid Differentiation between Nocardia and Streptomyces by Paper Chromatography of Whole-cell Hydrolysates", Appl. Microbiol. 12, 421-423 (1964)] and of Lechevalier [M. P. Lechevalier, "Identification of Aerobic Actinomycetes of Clinical Importance", J. Lab. Clin. Med. 71, 934-944 (1968)].

Resistance to lysozyme was measured by methods recommended by Gordon et al. [R. E. Gordon, D. A. Barnett, J. E. Handerhan and C. H. Pang, "Nocardia coeliaca, Nocardia autotrophica and the Nocardia Strain", Int. J. Syst. Bacteriol. 24(1), 54-63 (1974)].

Resistance to antibiotics was measured by padding antibiotic sensitivity discs onto the surface of seeded ISP No. 2 agar plates.

Phosphatase and urease were determined by methods described by Blazevic and Ederer, supra.

Cultural Characteristics

Cultures A54145.1, A54145.2 and A54145.3 did not produce abundant growth or abundant aerial mycelia in any of the 10 agar media tested. Moderate growth and limited aerial hyphae were produced on several complex and defined agar media. The aerial spore mass color was best represented on oatmeal agar (ISP No. 3) and inorganic salts-starch agar (ISP No. 4) media and was in the red (R) color series. The nearest matching color tab in the Tresner and Backus System [H. D. Tresner and E. J. Backus, "System of Color Wheels for Streptomycete Taxonomy," Appl. Microbiol. 11:335-338 (1956)] was 4 ec, grayish yellowish pink.

The reverse side of the cultures did not have a distinctive pigment. They were yellow-brown. A minor distinction between the strains was the tendency of A54145.3 to produce a darker shade of brown than A54145.1 or A54145.2 on ISP No. 3 and tap water agar.

No soluble pigments were produced, except for a light brown pigment seen in yeast-malt extract agar (ISP No. 2). Table III presents these cultural characteristics.

Table No. III: Cultural Characteristics of A54145.1, A54145.2, A54145.3 and S. fradiae[a]

| | | A54145.1 | A54145.2 | A54145.3 | S. fradiae |
|---|---|---|---|---|---|
| ISP No. 2 | G: | Good | Good | Fair | Abundant |
| | R: | 74.s.yBr | 74.s.yBr | 74.s.yBr | 71.m.OY |
| | Am: | Fair: 3ge l.gy.yBr | Good: 3ge l.gy.yBr | Fair: 3ge l.gy.yBr | Abundant: 2ca p.Y |
| | Sp: | Light-brown | Light-brown | Light-brown | None |
| ISP No. 3 | G: | Fair | Fair | Poor | Good |
| | R: | 57.1.Br | 57.1.Br | 58.m.Br | 76.1.yBr |
| | Am: | Fair: 4ec gy.yPk. | Good: 4ec gy.yPk. | Poor: 4ec gy.yPk. | Good: 4ec gy.yPk. |
| | Sp: | None | None | None | None |
| ISP No. 4 | G: | Fair | Fair | Fair | Good |
| | R: | 78.d.yBr | 75.deep yBr | 75.deep yBr | 74.s.yBr |
| | Am: | Fair: 4ec gy.yPk. | Fair: 4ec gy.yPk. | Poor: 4ec gy. yPk. | Abundant: 3ca p.OY |
| | Sp: | None | None | None | None |
| ISP No. 5 | G: | Fair | Fair | Fair | Good |
| | R: | 87.m.Y | 87.m.Y | 87.m.Y | 70.1.OY |
| | Am: | Fair 2dc yGy | Poor: 2dc yGy | Poor: 2dc yGy | Good: 2ca pY |
| | Sp: | None | None | None | None |
| ISP No. 7 | G: | Fair | Fair | Fair | Good |
| | R: | 88.d.Y | 88.d.Y | 88.d.Y | 72.d.OY |
| | Am: | Poor | Poor | Poor | Good: 3ca p.OY |
| | Sp: | None | None | None | None |

Table III continued

| Medium | | A54145.1 | A54145.2 | A54145.3 | S. fradiae |
|---|---|---|---|---|---|
| Czapeks | G: | Poor | Fair | Poor | Fair |
| | R: | 90.gy.Y | 93.y Gray | 77. m.yBr | 70.1.OY |
| | Am: | Trace | Trace | Trace | Fair: 3ca p.OY |
| | Sp: | None | None | None | None |
| Glucose Asparagine | G: | Poor | Trace | Poor | Fair |
| | R: | 88.d.Y | 88.d.Y | 88.d.Y | 88.d.Y |
| | Am: | None | Trace | None | Poor: b White |
| | Sp: | None | None | None | None |
| Glycerol Glycine | G: | Not grown | Not grown | Not grown | Poor |
| | R: | - | - | - | 87.m.Y |
| | Am: | - | - | - | None |
| | Sp: | - | - | - | None |
| Tomato Paste Oatmeal | G: | Fair | Fair | Fair | Fair |
| | R: | 84.s.Y | 74.s.yBr | 74.s.yBr | 94.1.01.Br |
| | Am: | Fair: 4ec gy.yPk | Fair: 4ec gy.yPk. | Fair: 4ec gy.yPk | Fair: 4ec gy.yPk. |
| | Sp: | None | None | None | None |
| Tap Water Agar | G: | Fair | Fair | Fair | Good |
| | R: | 57.1.Br | 57.1.Br | 58.m.Br | 76.1.yBr |
| | Am: | Fair: 4ec gy.yPk | Fair: 4ec gy.yPk. | Fair: 4ec gy.yPk | Good: 4ec gy.yPk. |
| | Sp: | None | None | None | None |

[a] G = growth; R = reverse; Am = aerial mycelia; Sp = soluble pigment

Morphological Characteristics

The mycelia of A54145.1, A54145.2 and A54145.3 were non-fragmenting and monopodially branched. Sporophores were short chains of 10 or more spores arranged in a hooked and looped configuration. No differences were observed between the three strains. They were placed in the Retinaculum-apertum (RA) configuration of Pridham [T. G. Pridham, C. W. Hesseltine, and R. C. Benedict, "A Guide for the Classification of Streptomycetes According to Selected Groups," Appl. Microbiol. 6:52-79 (1957)]. Rectus-flexibilis (RF) morphology was also observed.

The hooks and loops were primitive and incomplete. Morphology on some media, such as glycerol-asparagine agar (ISP No. 5) and Czapek's solution, was exclusively (RF) configuration. Morphology on other media such as ISP No. 3, ISP No. 4, and tap water agar clearly showed the (RA) configuration.

Scanning electron micrography was done on the parent strain, A54145.1. Spore shape was oblong. The average spore size was 1.5 x 1.0 µM. The spore-surface ornamentation was smooth (Sm).

Physiological Characteristics

Table IV gives the carbohydrate-utilization pattern of these strains.

**Table IV: Utilization of Carbon Compounds by A54145.1, A54145.2, A54145.3 and *S. fradiaea***

| | A54145.1 | A54145.2 | A54145.3 | S. fradiae |
|---|---|---|---|---|
| control | - | - | - | - |
| adonitol | - | - | - | - |
| L-arabinose | + | + | + | + |
| cellobiose | + | + | + | + |
| cellulose | - | - | - | + |
| dextran | - | - | - | - |
| D-fructose | + | - | - | + |
| D-galactose | + | + | + | + |
| D-glucose | + | + | + | + |
| i-inositol | - | - | - | - |
| inulin | - | - | - | - |
| D-lactose | + | - | + | + |
| mannitol | - | - | - | - |
| D-mannose | + | + | + | + |
| D-melezitose | - | - | - | - |
| D-melibiose | - | - | - | - |
| raffinose | - | - | - | - |
| L-rhamnose | - | - | - | - |
| ribose | + | + | + | + |
| salicin | - | - | - | + |
| sucrose | - | - | - | + |
| trehalose | + | + | + | + |
| xylitol | - | - | - | - |
| D-xylose | + | + | + | + |

[a]+ = utilized; - = not utilized

A54145.1, A54145.2, and A54145.3 had identical antibiotic-resistance patterns. The strains were resistant to lincomycin at 2 µg and penicillin G at 10 units. The strains were sensitive to bacitracin at 10 units, cephalothin at 30 µg, gentamicin at 10 µg, neomycin at 30 µg, oleandomycin at 15 µg, rifampin at 5 µg, streptomycin at 10 µg, tetracycline at 30 µg, tobramycin at 10 µg and vancomycin at 30 µg.

Table V shows additional physiological characteristics.

Table V: Comparison of A54145.1, A54145.2, A54145.3 and *S. fradiae*[a]

| Characteristic | A54145.1 | A54145.2 | A54145.3 | *S. fradiae* |
|---|---|---|---|---|
| Aerial spore-mass color | red | red | red | red |
| Carbon source utilized | | | | |
| D-fructose | + | - | - | + |
| D-lactose | + | - | + | + |
| salicin | - | - | - | + |
| sucrose | - | - | - | + |
| Catalase production | + | + | + | + |
| Degradation of: | | | | |
| adenine | + | NG[b] | + | + |
| calcium malate | - | - | - | - |
| casein | + | + | + | + |
| chitin | - | - | - | - |
| DNA | + | + | + | + |
| elastin | + | + | + | + |
| esculin | + | + | - | + |
| hippurate | - | - | - | - |
| hypoxanthine | + | + | + | - |
| keratin | - | - | - | - |
| starch | + | + | + | + |
| tyrosine | - | - | - | - |
| xanthine | - | - | - | - |
| Gelatin liquefaction | + | + | - | + |
| $H_2S$ production | + | + | + | - |
| Melanoid pigments | - | - | - | - |
| Morphology | RA | RA | RA | RA |
| NaCl tolerance (percent) | 6 | 4 | 6 | 7 |
| Nitrate reduction | + | + | - | + |
| Phosphatase production | + | + | + | + |

**Table V continued**

| Character | A54145.1 | A54145.2 | A54145.3 | *S. fradiae* |
|---|---|---|---|---|
| Resistance to lysozyme | - | - | - | - |
| Reverse side color | Y-Br | Y-Br | Y-Br | Y-Br |
| Skim-milk hydrolysis | - | - | - | - |
| Soluble pigments | - | - | - | - |
| Spore shape | oblong | ND[c] | ND | oblong |
| Spore surface | smooth | ND | ND | smooth |
| Survival at 50°C, 8 hrs. | + | + | + | + |
| Temperature range °C | 45 | 45 | 37 | 45 |
| Urease production | + | + | + | + |

[a] + = culture has trait; - = culture does not have trait

[b] NG = not grown out

[c] ND = not done

Table VI summarizes the characteristics in which the A54145 strains differ.

Table VI:

| Comparison of Differences in the A54145 Strains | | | |
|---|---|---|---|
| Characteristic | A54145.1 | A54145.2 | A54145.3 |
| Aerial hyphae production | fair | fair | poor |
| Esculin degradation | + | + | - |
| Fructose utilization | + | - | - |
| Gelatin liquefaction | + | + | - |
| Lactose utilization | + | - | + |

Continuation of the Table on the next page

Table VI: (continued)

| Comparison of Differences in the A54145 Strains | | | |
|---|---|---|---|
| Characteristic | A54145.1 | A54145.2 | A54145.3 |
| Nitrate reduction | + | + | - |
| % NaCl tolerance | 6 | 4 | 6 |
| Reverse side color | lt. brn | lt. brn | dk. brn |
| Temperature range - °C | 45 | 45 | 37 |

Cell-wall Analysis

Hydrolyzed whole cells of the original isolate A54145 contained LL-diaminopimelic acid with no meso isomer present. This is indicative of a Type I cell wall [M. P. Lechevalier, "Identification of Aerobic Actinomycetes of Clinical Importance," J. Lab Clin. Med. 71:934-944 (1968)].

A qualitative analysis of whole-cell methanolysates indicated that A54145.1 does not contain mycolic acids. The other strains were not analyzed; these characteristics are known not to change with mutation [M. P. Lechevalier, A. C. Horan and H. Lechevalier, "Lipid Composition in the Classification of Nocardiae and Mycobacteria," J. Bacteriol. 105 (1):313-318 (1971)].

Species Determination

The chemotaxonomic information plus the general cultural and morphological characteristics were consistent with the assignment of strain A54145.1 to the genus Streptomyces.

Six species selected from the published literature were similar to culture A54145.1:

Streptomyces cremeus*
Streptomyces roseolus*
Streptomyces termitus**
Streptomyces fradiae*
Streptomyces roseus*
Streptomyces roseosporus***

*E. B. Shirling and D. Gottlieb, "Comparative Description of Type Strains of Streptomyces," Int. J. Syst. Bacteriol. 18(2):69-189 (1968)

**ibid. 19(4):375-512 (1969)

***ibid 18(4):279-392 (1968)

These cultures belong in the red color series, have Retinaculum-apertum and Rectus-flexibilis sporophore morphology, smooth spore-surface ornamentation, lack melanoid and distinctive pigments, and have a carbon-utilization pattern and other cultural characteristics similar to those of culture A54145.1.

The six selected species were grown simultaneously with A54145.1, A54145.2 and A54145.3 to obtain direct laboratory comparisons. Two cultures, S. fradiae and S. roseosporus, were observed to be most similar to A54145.1. Of these two, S. roseosporus did not have a very close cultural match to A54145.1. On many of the media S. roseosporus was in the white color series. S. roseosporus generally grew better and produced more abundant aerial mycelia than A54145.1, except on Czapek's solution agar where S. roseosporus had no growth. S. roseosporus was in the Rectus-flexibilis morphology series, while A54145.1, although having the (RF) type of morphology, was predominantly of the Retinaculum-apertum type. Thus, although S. roseosporus had many physiological characteristics like those of A54145.1; the differences in cultural and morphological characteristics precluded assignment of A54145.1 as a strain of S. roseosporus.

S. fradiae was very similar to A54145.1 culturally, morphologically and physiologically. Both cultures had an excellent aerial hyphae color match, similar reverse color and absence of soluble pigments. S. fradiae and A54145.1 had the

same sporophore morphology, short spore chains, spore shape, and smooth spore-surface ornamentation.

Physiological characteristics were in good agreement, except for the antibiotic-resistance pattern, degradation of hypoxanthine, production of $H_2S$, and tolerance to NaCl. A summary of the differences and similarities between strain A54145.1 and S. fradiae is given in Table VII.

Table VII:

| Comparison of Strain A54145.1 and *S.* fradiae | |
|---|---|
| Similarities | Differences |
| absence of melanoid pigments<br>aerial spore mass color<br>carbon utilization<br>catalase production<br>cultural characteristics<br>degradation profile<br>gelation liquefaction<br>morphology<br>nitrate reduction<br>phosphatase production<br>skim milk reaction<br>survival at 50°C, 8 hr.<br>temperature range<br>urease production | antibiotic resistance<br>$H_2S$ production<br>hypoxanthine degradation<br>NaCl tolerance |

These comparisons indicate that culture A54145.1 is very similar to S. fradiae. Therefore, culture A54145.1 is classified as a strain of Streptomyces fradiae (Waksman and Curtis 1916) Waksman and Henrici 1948. S. fradiae is recognized in the Approved Lists of Bacterial Names [V. B. D. Skerman, V. McGowan, and P. H. A. Sneath (ed.), "Approved Lists of Bacterial Names," Int. J. Syst. Bacteriol. 30:225-420 (1980)] and consequently is a validly published species. Cultures A54145.2 and A54145.3 are classified as S. fradiae mutants derived from A54145.1.

As is the case with other organisms, the characteristics of the A54145-producing cultures Streptomyces fradiae NRRL 18158, NRRL 18159 and NRRL 18160 are subject to variation. Thus, progeny of these strains, e.g., spontaneous and induced mutants may be obtained by methods known in the art. Spontaneous mutants can be obtained by natural selection, or mutants can be induced by treatment with various known physical and chemical mutagens such as ultraviolet light, X rays, gamma rays and chemicals such as N-methyl-N'-nitro-N-nitrosoguanidine. Mutants of the Streptomyces fradiae strains NRRL 18158, NRRL 18159 and NRRL 18160 which retain the characteristic of producing recoverable amounts of the A54145 antibiotics are part of this invention.

The culture medium used to grow the A54145-producing Streptomyces fradiae strains can be any one of a number of media. For economy in production, optimal yield, and ease of product isolation, however, certain culture media are preferred. For example, preferred carbon sources are glucose, maltose, galactose, methyl oleate and peanut oil, although corn starch, potato dextrin, corn oil, cottonseed oil, soybean oil and the like can also be used.

Preferred nitrogen sources are soybean grits, soybean flour or an enzymatic hydrolysate of soybeans. Enzyme-hydrolyzed casein, peanut meal, fish meal, cottonseed meal, and the like are also useful nitrogen sources.

Among the nutrient inorganic salts which may advantageously be incorporated in the culture media are the customary soluble salts capable of yielding zinc, sodium, magnesium, calcium, ammonium, chloride, carbonate, sulfate, nitrate and like ions.

Essential trace elements necessary for the growth and development of the organism should also be included in the culture medium. Such trace elements commonly occur as impurities in other substituents of the medium in amounts sufficient to meet the growth requirements of the organism.

Small amounts (i.e. 0.2 ml/L) of an antifoam agent such as polypropylene glycol may be added to large scale fermentation media if needed.

For production of substantial quantities of the A54145 antibiotics, submerged aerobic fermentation in tanks is preferred. Small quantities may be obtained by shake-flask culture. Because of the time lag in antibiotic production commonly associated with inoculation of large tanks with the spore form of the organism, it is preferable to use a vegetative inoculum. The vegetative inoculum is prepared by inoculating a small volume of culture medium with the spore form or mycelial fragments of the organism to obtain a fresh, actively growing culture of the organism.- The vegetative inoculum is then transferred to a larger vessel. The vegetative inoculum medium can be the same as that used for larger fermentations, but other media are also suitable.

The A54145 antibiotics are produced by the Streptomyces fradiae strains when grown at temperatures between about 20° and about 35°C. A good temperature for A54145 production appears to be from about 25°C to about 29°C.

As is customary in submerged aerobic culture processes, sterile air is blown into the vessel from the bottom while the medium is stirred with conventional turbine impellors. In a fully baffled 165-liter fermentor containing approximately 115 liters of broth, an aeration rate of 0.25 v/v/m with an agitation rate of 150-200 rpm is sufficient to maintain the level of dissolved oxygen at or above 30% of air saturation.

Production of antibiotic A54145 can be followed during the fermentation by testing samples of the broth for antibiotic activity against organisms known to be sensitive to the antibiotic. One assay organism useful in testing for antibiotic A54145 is Bacillus subtilis. The bioassay is conveniently performed by the agar-well plate test.

One improved process for preparing an A54145 component of formula 2 comprises feeding a $C_4$-$C_{18}$-alkanoic or alkenoic acid or alcohol, or an ester or salt thereof, to an A54145-producing culture during its fermentation after initiating the production stage of the fermentation, continuing until a recoverable amount of A54145 component is produced. Optionally, the A54145 component may be recovered.

Another improved process of this invention is a process for preparing formula 5 compounds wherein R is $C_6$-$C_{10}$-alkanoyl and $R^2$ is hydrogen, which comprises adding a $C_6$-$C_{10}$-alkanoic or alkenoic acid or alcohol, or an ester or salt thereof, to an A54145-producing culture, after initiating the production stage of the fermentation, continuing until a recoverable amount of the formula 5 compound is produced.

These processes two processes provide significantly increased product yields.

In these processes the alkyl portion of the alkanoic or alkenoic acid or alcohol (the substrate) used can be a straight or branched chain. The straight-chain acids or alcohols, or their esters or salts, are recommended because of availability and lower cost. An especially preferred substrate is n-decanoic acid and its esters and salts.

When using an alkanoic acid ester, the $C_1$-$C_4$-alkyl esters are preferred. In such an ester, the $C_1$-$C_4$-alkyl group may also be straight or branched.

Representative suitable salts of alkanoic or alkenoic acids which may be used in this process include those formed from alkali metals and alkaline-earth metals such as sodium, potassium, lithium, cesium, rubidium, barium, calcium and magnesium. Suitable amine salts include the ammonium and the primary, secondary and tertiary $C_1$-$C_4$-alkyl-ammonium and hydroxy-C2-C4-alkyl-ammonium salts.

For these processes, it is preferable to add the substrate to the fermentation in the form of a sterile solution. For example, n-decanoic acid is a solid at room temperature, whereas its ethyl ester is a liquid. Thus, the ethyl ester is preferred because the acid must be dissolved in a compatible liquid, such as oleic acid or methyl oleate, for efficient feeding. Oleic acid is a particularly useful solvent for this purpose, although other solvents such as ethanol, ethyl acetate and $C_1$-$C_4$ esters of unsaturated fatty acids can be used. Those substrates which are suitably fluid at fermentation temperatures may be added directly and are, therefore, preferred.

The rate of addition of the substrate to the fermentation must be low enough to avoid producing a toxic effect on the fermentation, but high enough to increase the yield of the desired compound. Rates of addition of about 0.5 to about 4 mL of substrate per liter of fermentation broth per day can be used. A rate of from about 1.5 to about 3 mL of substrate per liter of fermentation broth per day is preferred.

The substrate is added to the growing A54145-producing culture during the production stage of the fermentation, beginning at from about 20 to about 26 hours and continuing until the fermentation is terminated. The substrate can be added by various methods. It is preferable, however, to add it by a method which best approaches a steady flow.

Another improved process for preparing A54145 compounds of formula 1 wherein R is $C_6$-$C_{10}$-alkanoyl and $R^1$ and $R^2$ are hydrogen comprises feeding glucose to an A54145-producing culture after initiating the production stage of the fermentation and continuing until a recoverable amount of the A54145 compound is produced. Preferably, addition is started from about 18 to about 24 hours after starting the production stage. The improvement obtained by this process is illustrated in Table VIII, which compares the results obtained by standard methods with results obtained using this process.

Table VII. Effect of Continuous Glucose Feed on A54145 Biosynthesis

| Glucose Level(%) | Glucose Addition Method | A54145 Yield (mcg/mL) |
|---|---|---|
| 4 | Included at time of medium make-up | 520 |
| 4 | Continuous feed from day 1 to day 8[a] | 1370 |

[a]Beginning 23 hours after initiating the production stage

As the results in Table VIII indicate, glucose feeding increases final A54145 yield by at least 150%.

In the continuous glucose feed process, the rate of addition of the glucose must be low enough to avoid toxic affects on the fermentation, but high enough to cause a significant increase in the yield of A54145 compound. A rate of about 6 to about 9 grams of glucose/liter of fermentation/day is recommended, but a rate of about 7.5 g/L/day is preferred for this process.

A third method for increasing product yields of A54145 components comprises feeding an enzymatic soy digest an A54145-producing culture after initiating the production stage of the fermentation, continuing throughout its fermentation. Preferably, addition is started from about 90 to about 190 hours after initiating the production stage and is carried out at a rate of from about 2 to about 4 grams of soy digest/liter of fermentation broth/day.

The biosynthetic processes can be carried out over a temperature range of from about 20° to about 34°C. Temperature affects the amount of total antibiotic produced and the type of nucleus and side chain produced. Thus, the temperature of the fermentation should be adjusted appropriately in order to obtain optimum yields of the desired product. Table IX summarizes temperature effects on A54145 production which were observed in fermentation studies in which only the temperature was varied.

Table IX: EFFECT OF TEMPERATURE ON A54145 NUCLEUS AND ACYL-CHAIN BIOSYNTHESIS IN A STIRRED 165-L BIOREACTOR

| Temperature (°C) | Total Antibiotic (mcg/mL) | Nuclei (%) | | | | Acyl Chains (%)[a] | | |
|---|---|---|---|---|---|---|---|---|
| | | A | B | C | F | $iC_{10}$ | $nC_{10}$ | $aC_{11}$ |
| 21 | 1015 | 60 | 31 | - | 9 | 79 | 15 | 6 |
| 25 | 1582 | 62 | 30 | - | 8 | 74 | 18 | 8 |
| 29 | 1623 | 39 | 51 | 3 | 6 | 64 | 21 | 14 |
| 31 | 1341 | 32 | 59 | 2 | 6 | 59 | 23 | 17 |
| 33 | 923 | 19 | 73 | 2 | 6 | 60 | 23 | 16 |

[a] $iC_{10}$ = 8-methylnonanoyl
$nC_{10}$ = n-decanoyl
$aC_{11}$ = 8-methyldecanoyl

B. The Cyclic Peptide Intermediates (Formula 4b and 4c Compounds)

The A54145 components or blocked components are deacylated by exposing the compound in an aqueous medium to an enzyme produced by a microorganism of the family Actinoplanaceae until substantial deacylation is accomplished. A preferred method comprises using an enzyme produced by the microorganism Actinoplanes utahensis NRRL 12052 to cleave the fatty acid side chain.

Deacylation is ordinarily accomplished by adding the appropriate A54145 component or blocked A54145 component to a growing culture of the A. utahensis strain and permitting the culture to incubate until deacylation is accomplished. The A54145 nucleus or blocked A54145 nucleus (formula 4b or 4c compounds) is then separated from the fermentation broth by methods in the art.

C. The Acylation Step

The formula 5 compounds are prepared by acylating a compound of formula 4b (which is blocked at the ε-amino group of lysine) with the desired alkanoyl or alkenoyl side chain, using methods conventional in the art for forming an amide bond. The acylation is accomplished, in general, by reacting the selected compound with an activated derivative of the alkanoic acid or alkenoic acid corresponding to the desired acyl side chain group (R). The term "activated derivative" means a derivative which renders the carboxyl function of the acylating agent reactive to coupling with the primary amino group to form the amide bond which links the acyl side chain to the nucelus. Suitable activated derivatives, their methods of preparation, and their methods of use as acylating agents for a primary amine will be recognized by those skilled in the art.

Preferred activated derivatives are: (a) an acid halide (e.g. an acid chloride), (b) an acid anhydride (e.g., an alkoxyformic acid anhydride) or (c) an activated ester (e.g. a 2,4,5,-trichlorophenyl ester). Other methods for activating the carboxyl function include reacting the carboxylic acid with a carbonyldiimide (e.g. N,N'-dicyclohexylcarbodiimide or N,N'-diisopropylcarbodiimide) to give a reactive intermediate which, because of instability, is not isolated, the reaction with the primary amine being carried out in situ.

Those skilled in the art will recognize that the formula 5 compounds are prepared using selective acylation procedures and with the assistance of amino-protecting groups. For example, when a formula 4c compound (R and $R^1$ are hydrogen) is the starting material, acylation can occur at both the α-amino group of tryptophan and the ε-amino group of lysine to give $N_{Trp}$, $N_{Lys}$-diacyl derivatives. To obtain derivatives monoacylated at the α-amino group of tryptophan, therefore, it is preferable to acylate a compound of formula 4b wherein the ε-amino group of lysine (the $R^1$ position) is blocked by an amino-protecting group. Such starting materials are preferably obtained by blocking the A54145 component at this position before it is deacylated.

Scheme I outlines the general procedure for preparing the formula 5 compounds. In this Scheme, the following symbols are used:

| | |
|---|---|
| [*] = | remainder of the A54145 component |
| $N_T$ = | α-amino group of tryptophan |
| $N_L$ = | ε-amino group of lysine |
| R = | substituents as defined |
| $R_N$ = | acyl group of natural factor |
| B = | amino-protecting group |
| Acyl = | an acylation step |
| Deacyl = | a deacylation step |
| Block = | acylation with an amino-protecting group |
| Deblock = | removal of an amino-protecting group |

Scheme I

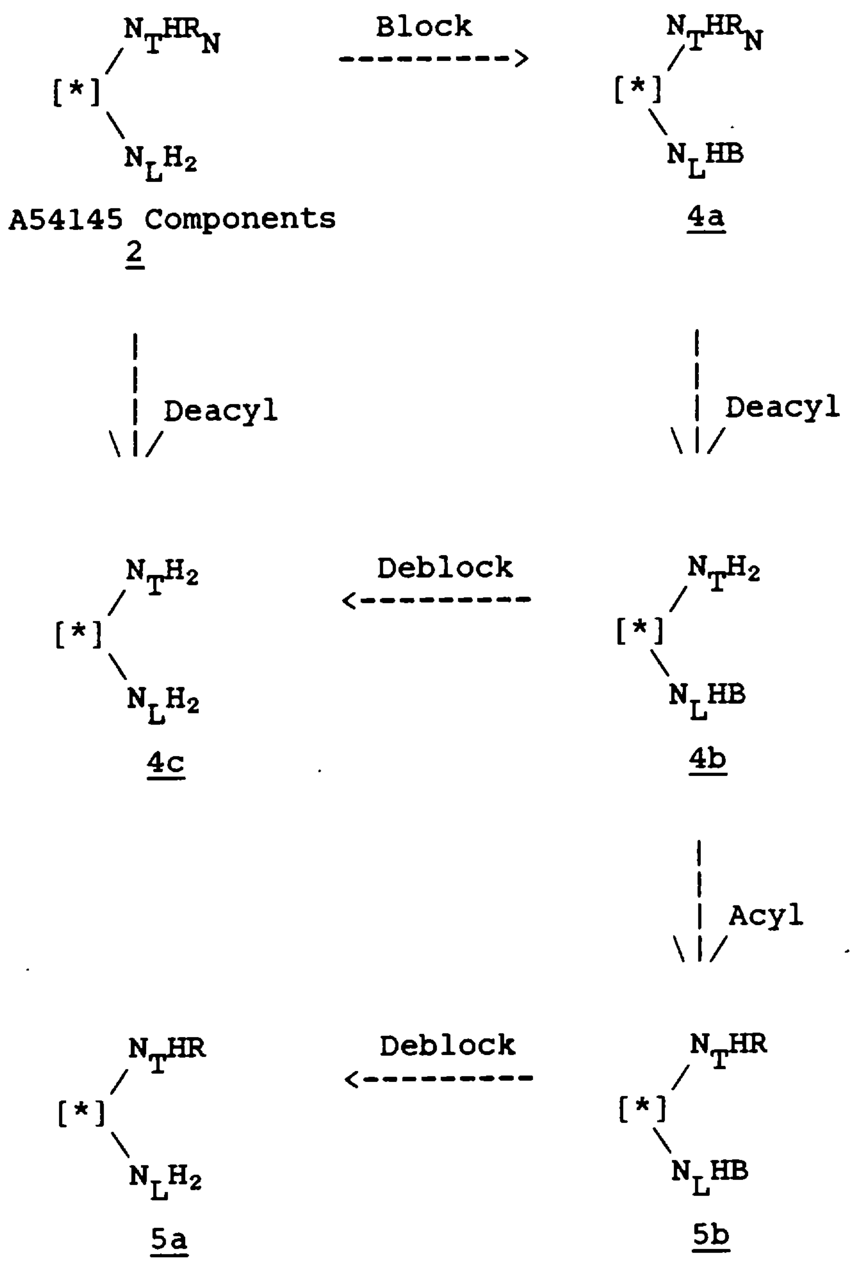


A54145 Components 2

4a

4c

4b

5a

5b

D. The Schiff's Bases

The formula 5 compounds in which a) R and $R^2$ represent an alkylidenyl group (5b compounds; the Schiff's bases) or b) R is an alkyl group (the reduced Schiff's bases) can be prepared by known methods for preparing Schiff's bases and reducing such bases, respectively. Thus, the Schiff's bases are prepared by reacting (condensing) the primary amino group of tryptophan of the corresponding A54145 nucleus with an appropriate aldehyde or ketone in a suitable solvent. Reduction of the imine bond of the Schiff's base, to obtain the corresponding formula 5 compound in which R is alkyl can be accomplished by known selective reduction procedures. A preferred reducing agent for this reaction is sodium cyanoborohydride.

The Schiff's bases are useful as intermediates to the reduced Schiff's bases. When the Schiff's base is used as an intermediate, it is not necessary to isolate the intermediate prior to reducing it to form the reduced Schiff's base.

A preferred method for preparing a formula 5 compound in which R is alkanoyl or alkenoyl is by the active ester method. A formula 4 compound wherein R = H and $R^1$ = t-BOC, i.e. an A54145 $N_{Lys}$-(t-BOC)-nucleus or "t-BOC nucleus", is an especially preferred starting material in the preparation of formula 5 compounds. The 2,4,5-trichlorophenyl ester of the desired alkanoic or alkenoic acid is a preferred acylating agent. In this method, an excess amount of the active ester is reacted with the t-BOC nucleus at room temperature in a non-reactive organic solvent such as DMF, THF, diethyl ether or dichloromethane. The reaction time is not critical, although a time of about 6 to about 20 hours is preferred. At the conclusion of the reaction, the solvent is removed, and the residue is purified. A particularly useful purification method is reverse-phase HPLC, using LP-1/$C_{18}$ as the stationary phase and a mixture of $H_2O/CH_3OH/CH_3CN$/pyridine/HOAc as the solvent system. The t-BOC group is removed by treatment with trifluoroacetic acid/anisole/triethylsilane or, preferably, trifluoroacetic acid/1,2-ethanedithiol for from about three to about five minutes at room temperature. After the solvent is removed, the residue can be purified by reverse-phase HPLC.

An alternative acylation method is a modified Schotten-Baumann procedure in which the unblocked nucleus is treated with the acid chloride of the desired alkanoic acid or alkenoic acid in a pyridine/water mixture. In this method, an excess of the acid chloride in a non-reactive organic solvent (such as acetone) is added slowly to a solution of the nucleus in 90% pyridine/10% water (by volume). The unreacted acid chloride is separated from the reaction product by extraction into an immiscible organic solvent (e.g., diethyl ether). Final purification can be accomplished by reverse-phase HPLC, as previously described.

The alkanoic and alkenoic acids used as starting materials for the acylation reaction, and the activated derivatives thereof (in particular, the acid chlorides and the 2,4,5-trichlorophenyl esters), are known compounds or can be prepared from known compounds by known methods. The 2,4,5-trichlorophenyl esters are conveniently made by treating the acid chloride of the alkanoic or alkenoic acid with 2,4,5-trichlorophenol in the presence of pyridine or by treating the free alkanoic or alkenoic acid with 2,4,5-trichlorophenol in the presence of N,N'-dicyclohexylcarbodiimide as a coupling agent. The 2,4,5-trichlorophenyl ester derivative can be purified by column chromatography over silica gel.

Thus, according to a further aspect of this invention, there is provided a process for preparing a compound of formula 1 which comprises:

A) cultivating Streptomyces fradiae NRRL 18158, NRRL 18159 or NRRL 18160, or an A54145-producing mutant thereof, in a culture medium containing assimilable sources of carbon, nitrogen, and inorganic salts under submerged aerobic fermentation conditions, optionally followed by separation of the antibiotic from the fermentation medium and/or salification of the antibiotic if not in salt form so as to produce a compound of formula 1 wherein:

$R^2$ and $R^1$ are hydrogen; and
R is methylnonanoyl, 8-methyldecanoyl or n-decanoyl;

provided that: when X is Val and Y is Glu, R must be 8-methylnonanoyl; and when X is Val and Y is 3-MG, R must be 8-methyldecanoyl;

B) optionally protecting the antibiotic with an amino-protecting group so as to prepare a compound of formula 1 wherein:

$R^2$ and $R^1$ are an amino-protecting group; and
R is 8-methylnonanoyl, 8-methyldecanoyl or n-decanoyl;

provided that: when X is Val and Y is Glu, R must be 8-methylnonanoyl; and when X is Val and Y is 3-MG, R must be 8-methyldecanoyl;

C) enzymatically deacylating the product of Step (A) or (B) so as to prepare a compound of formula 1 wherein:

R is hydrogen; and
$R^1$ and $R^2$ are hydrogen or an amino-protecting group; and/or salifying the compound if not in salt form; and optionally

D)

i) reacylating the product prepared in Step B with the desired alkanoyl or alkenoyl side chain so as to prepare a compound of formula 1 wherein:

R is optionally substituted $C_8$-$C_{18}$-alkanoyi or $C_8$-$C_{18}$-alkenoyl; and

$R^1$ and $R^2$ are hydrogen or an amino-protecting group;

provided that $R^1$ cannot be hydrogen:

1) when X = Ile, Y = Glu or 3-MG and R is 8-methylnonanoyl, 8-methyldecanoyl or n-decanoyl;
2) when X = Val, Y = Glu and R = n-decanoyl; and
3) when X = Val, Y = 3-MG and R = 8-methyldecanoyl; or

ii) condensing the primary amino group of tryptophan of the compound prepared in Step B with an appropriate aldehyde or ketone so as to prepare the compound of formula 1 wherein R and $R^2$, taken together, represent a $C_8$-$C_{18}$-alkylidene group; and
iii) optionally reducing the Step (C)(ii) compounds so as to prepare a compound of formula 1 wherein R is $C_8$-$C_{18}$-alkyl; and

F) optionally deblocking the Step C compound so as to prepare the formula 1 compound wherein $R^1$ and $R^2$ are hydrogen.

Certain of the A54145 antibiotics (the formula 2 and 5a compounds) inhibit the growth of pathogenic bacteria. These compounds are called A54145 active compounds. Tables X and XI show the inhibitory concentrations (MIC's) at which the formula 2 compounds and illustrative formula 5a compounds, respectively, inhibit certain organisms. The MIC's in these tables were determined by conventional agar-dilution assays.

Table X: Antibacterial Activity of the A54145 Components (Formula 2)[a]

| Test Organism | A | B | C | D | E | F | $A_1$ | $B_1$ |
|---|---|---|---|---|---|---|---|---|
| *Staphylococcus aureus* X1.1 | 8 | 2 | 2 | 4 | 1 | 16 | 4 | 2 |
| " " V41 | 8 | 2 | 2 | 4 | 2 | 16 | 4 | 2 |
| " " X400 | 8 | 2 | 2 | 4 | 2 | 16 | 8 | 2 |
| " " S13E | 8 | 2 | 2 | 4 | 2 | 16 | 8 | 2 |
| *Staphylococcus epidermidis* EPI1 | 4 | 2 | 2 | 2 | 1 | 16 | 4 | 1 |
| " " 222 | 4 | 2 | 1 | 2 | 1 | 8 | 4 | 1 |
| *Streptococcus pyogenes* C203 | 2 | 0.5 | 0.5 | 0.5 | 0.25 | 4 | 1 | 0.5 |
| " *pneumoniae* Park1 | 8 | 2 | 2 | 4 | 1 | 8 | 4 | 2 |
| " sp. group D X66 | 16 | 4 | 4 | 4 | 2 | 16 | 16 | 8 |
| " " 2041 | 64 | 8 | 4 | 32 | 4 | >32 | 32 | 32 |

[a]MIC in mcg/mL

**Table XI: Antibacterial Activity of Formula 5a Compounds**

| Test Organism | MIC's of Compound[b] | | | | |
|---|---|---|---|---|---|
| | 2 | 4 | 6 | 10 | 26 |
| Staphylococcus aureus X1.1 | 2 | 1 | 32 | 16 | 0.25 |
| " " V41 | 4 | 1 | 32 | 16 | 0.25 |
| " " X400 | 4 | 1 | 32 | 16 | 0.5 |
| " " S13E | 4 | 2 | 32 | 16 | 0.25 |
| Staphylococcus epidermidis EPI1 | 2 | 1 | 16 | 16 | 0.5 |
| " " 222 | 2 | 1 | 16 | 16 | 0.5 |
| Streptococcus pyogenes C203 | 0.5 | 0.125 | 4 | 4 | 0.06 |
| " pneumoniae Park 1 | 2 | 2 | 64 | 32 | 0.125 |
| " sp. group D X66 | 16 | 4 | 128 | 128 | 2 |
| " " 2041 | 32 | 8 | >128 | 128 | 4 |

[a] MIC's in mcg/mL

[b] Compound numbers from Table I

The in vitro activity of the A54145 active compounds is stimulated by the presence of calcium ions. For example, the in vitro activity of A54145A against Staphylococcus aureus 209P and Streptococcus faecalis ATCC 29212 was stimulated about 10 to 100 times by the presence of $Ca^{++}$ (50 mg/mL).

The A54145 active compounds have shown in vivo antimicrobial activity against experimentally-induced infections in laboratory animals. When two doses of test compound were administered to mice experimentally infected with Streptococcus pyogenes or Staphylococcus aureus, the activity observed was measured as an $ED_{50}$ value [effective dose in mg/kg to protect 50% of the test animals: see Warren Wick, et al., J. Bacteriol. 81, 233-235 (1961)]. The $ED_{50}$ values observed for illustrative compounds are given in Tables XII and XIII.

Table XII: $ED_{50}$ Values for A54145 Components in Mice[a]

| Infecting Organism | $ED_{50}$ Value[b] A54145A | A54145B | $A54145A_1$ |
|---|---|---|---|
| Streptococcus pyogenes | 2.4[c] | 0.94 | 5.0 |
| Staphylococcus aureus | 3.3 | ---[d] | --- |

[a]Administered subcutaneously

[b]mg/kg x 2; doses given 1 and 4 hours hours post-infection

[c]Average of two tests with individual values of 1.6 and 3.1

[d]Not done

Table XIII: $ED_{50}$ Values for Formula 5a Compounds[a] vs. Streptococcus pyogenes

| Compound No.[b] | $ED_{50}$[c] |
|---|---|
| 2 | 1.65 |
| 4 | 1.15 |
| 6 | 10.6 |
| 10 | 10.8 |

[a]Administered subcutaneously
[b]Compound numbers from Table I
[c]mg/kg x 2; doses given 1 and 4 hours post-infection

Table XIV summarizes the relative toxicity of illustrative formula 5a compounds.

Table XIV: Relative Toxicity of Formula 5a Compounds[a]

| Compound No.[b] | Median Lethal Dose (mg/kg) |
|---|---|
| 2 | >520 |
| 4 | 321 |
| 6 | >510 |
| 10 | >505 |

[a]When administered IP in mice
[b]Compound numbers from Table I

Thus, according to another aspect of this invention, there is provided a compound of formula 2 or formula 5 for use in the therapy of bacterial infections.

Yet another aspect of the invention provides a pharmaceutical composition comprising as an active ingredient a compound of formula 2 or 5 associated with one or more pharmaceutically acceptable carriers therefor.

The A54145 active compounds also improve growth performance in animals. The compounds are especially effective growth promoters in fowl, such as chickens and turkeys, but should also be effective in other animals such as swine.

The A54145 active compounds are typically effective in improving growth performance in animals when administered with feeds at a rate of from about 0.05 to about 100 grams of compared per ton of feed (0.055 to 110 ppm). A preferred rate is from about 0.05 to about 50 g/ton, and an especially preferred rate is from about 1 to about 20 g/ton.

The A54145 active compounds can be administered to animals orally or parenterally. The most practical way to administer the compound is by formulation into the feed supply. A variety of feeds, including the common dry feeds, liquid feeds, and pelleted feeds, may be used. Although the preferred method of administration is by mixing it with the animals' feed, it can also be administered in other ways, for example, tablets, drenches, boluses, or capsules. Each individual dosage unit should contain a quantity of compound directly related to the proper daily dose for the animal to be treated.

The methods of formulating drugs into animal feeds are well known. A preferred method is to make a concentrated drug premix which in turn is used to prepare medicated feeds. Thus, according to a further aspect of this invention, there is provided an animal feed premix comprising as an active ingredient a compound of formula 2 or formula 5 associated with one or more agronomically acceptable carriers therefor. Typical premixes may contain from about 1 to about 200 grams of drug per pound of premix. Premixes may be either liquid or solid preparations.

The final formulation of feeds for animals or poultry will depend upon the amount of drug to be administered. The common methods of formulating, mixing, and pelleting feeds may be used to prepare feeds containing a A54145 active compound.

The compounds may be formulated for parenteral administration by methods recognized in the veterinary pharmaceutical art. Effective injectable compositions containing the compounds may be in either suspension or solution form. In the solution form, the compound is dissolved in a physiologically acceptable carrier. Such carriers comprise a suitable solvent, preservatives such as benzyl alcohol, if needed, and buffers. Useful solvents include, for example, water, alcohols, glycols, or inert oils such as vegetable oils or highly refined mineral oils.

Injectable suspension compositions are prepared using a nonsolvent for the compound with adjuvants, as a carrier. The nonsolvent can be, for example, a glycol such as polyethylene glycol.

Suitable physiologically acceptable adjuvants are necessary to keep the compound suspended in suspension compositions. The adjuvants may be chosen from among thickeners such as carboxymethylcellulose, polyvinylpyrrolidone, gelatin, and the alginates. Many surfactants are also useful for suspending the compounds. Lecithin, alkylphenol polyethylene oxide adducts, naphthalenesulfonates, alkylbenzenesulfonates, and the polyoxyethylene sorbitan esters are useful suspending agents in liquid nonsolvents.

Many substances which affect the hydrophilicity, density, and surface tension of the liquid nonsolvent can assist in making injectable suspensions in individual cases. For example, silicone antifoams, glycols, sorbitol, and sugars can be useful suspending agents.

When an A54145 active compound is used as an antibacterial agent, it may be administered either orally or parenterally. As will be appreciated by those skilled in the art, the compound is commonly administered together with a pharmaceutically acceptable carrier or diluent. The dosage administered will depend upon a variety of considerations, such as, for example, the nature and severity of the particular infection to be treated. Those skilled in the art will recognize that appropriate dosage ranges and/or dosage units for administration may be determined by considering the MIC and $ED_{50}$ values and toxicity data herein provided together with factors such as pharmacokinetics, the patient or host and the infecting microorganism.

The following non-limiting examples are provided to illustrate this invention. In these examples the following numbers will be used to represent specific solvent systems:

| No. | System | Ratio |
|---|---|---|
| 1 | Pyridine/HOAc/$H_2O$ | 1:1:98 |
| 2 | Pyridine/HOAc/$H_2O$/$CH_3CN$ | 1:1:88:10 |
| 2a | Pyridine/HOAc/$H_2O$/$CH_3CN$ | 1:1:87:11 |
| 2b | Pyridine/HOAc/$H_2O$/$CH_3CN$ | 1:1:86:12 |
| 2c | Pyridine/HOAc/$H_2O$/$CH_3CN$ | 1:1:83:15 |
| 2d | Pyridine/HOAc/$H_2O$/$CH_3CN$ | 1:1:82:16 |
| 2e | Pyridine/HOAc/$H_2O$/$CH_3CN$ | 1:1:78:20 |

Continuation of the Table on the next page

(continued)

| No. | System | Ratio |
|---|---|---|
| 2f | Pyridine/HOAc/$H_2O$/$CH_3CN$ | 1:1:73:25 |
| 2g | Pyridine/HOAc/$H_2O$/$CH_3CN$ | 1:1:70.5:27.5 |
| 2h | Pyridine/HOAc/$H_2O$/$CH_3CN$ | 1:1:68:30 |
| 2i | Pyridine/HOAc/$H_2O$/$CH_3CN$ | 1:1:67:31 |
| 2j | Pyridine/HOAc/$H_2O$/$CH_3CN$ | 1:1:66:32 |
| 2k | Pyridine/HOAc/$H_2O$/$CH_3CN$ | 1:1:65:33 |
| 2m | Pyridine/HOAc/$H_2O$/$CH_3CN$ | 1:1:63:35 |
| 3a | Pyridine/HOAc/$H_2O$/$CH_3CN$/MeOH | 1:1:70:18:10 |
| 3b | Pyridine/HOAc/$H_2O$/$CH_3CN$/MeOH | 1:1:68:20:10 |
| 3c | Pyridine/HOAc/$H_2O$/$CH_3CN$/MeOH | 1:1:63:25:10 |
| 3d | Pyridine/HOAc/$H_2O$/$CH_3CN$/MeOH | 1:1:61:27:10 |
| 3e | Pyridine/HOAc/$H_2O$/$CH_3CN$/MeOH | 1:1:58:30:10 |
| 3f | Pyridine/HOAc/$H_2O$/$CH_3CN$/MeOH | 1:1:56:32:10 |
| 3g | Pyridine/HOAc/$H_2O$/$CH_3CN$/MeOH | 1:1:53:35:10 |
| 3h | Pyridine/HOAc/$H_2O$/$CH_3CN$/MeOH | 1:1:68:25:5 |
| 3i | Pyridine/HOAc/$H_2O$/$CH_3CN$/MeOH | 1:1:73:15:10 |
| 3j | Pyridine/HOAc/$H_2O$/$CH_3CN$/MeOH | 1:1:60.5:25:12.5 |
| 3k | Pyridine/HOAc/$H_2O$/$CH_3CN$/MeOH | 1:1:71:20:7 |
| 4 | $CH_3CN$/$H_2O$ | 1:1 |
| 4a | $CH_3CN$/$H_2O$ | 15:85 |
| 5 | $CH_3OH$/$H_2O$ | 1:1 |

Separation of the individual antibiotic A54145 components can be followed by TLC or HPLC. One convenient analytical HPLC system is:

Analytical HPLC System for A54145 Components

Column: 4.6- x 250-mm Zorbax C8 (Dupont)
Mobile Phase: acetonitrile/water containing 0.2% tri ethylamine and adjusted to pH 3 with phosphoric acid (35:65)
Detection: UV at 223 nm
Flow Rate: 2 mL/min

A54145 components A-F have the following approximate retention times in this system:

| A54145 Factor | Retention Time (min) |
|---|---|
| A | 12.1 |
| $A_1$ | 13.1 |
| B | 14.9 |
| $B_1$ | 13.7 |
| C | 17.0 |
| D | 19.6 |
| E | 22.4 |
| F | 9.4 |

Formation and purification of the A54145 nuclei can be monitored by analytical HPLC, using the following system:

Analytical HPLC System for A54145 Nuclei

Column: 4.6- x 250 mm Zorbax ODS (Dupont, 5μ)
Detection: UV at 223 nm
Flow Rate: Usually 2 mL/min
Solvent System: $CH_3CN$/0.04M aq. $NH_4OAc$ (9:91)

## Example 1

### Producing Antibiotic A54145 with Streptomyces fradiae A54145.1

### A. Shake-flask Fermentation of A54145.1

The culture Streptomyces fradiae NRRL 18158, either as a lyophilized pellet or as a suspension maintained in liquid nitrogen, is used to inoculate 50 mL of a vegetative medium having the following composition:

| Vegetative Medium I | |
|---|---|
| Ingredient | Amount (g/L) |
| Glucose | 15.0 |
| Potato dextrin | 20.0 |
| Soybean grits | 15.0 |
| Corn steep liquor | 10.0 |
| Yeast extract | 1.0 |
| $CaCO_3$ | 5.0 |
| Tap water | q.s. 1 liter |
| (Adjust the pH of the medium from ~6.1 to ~6.5 with NaOH before sterilizing; post-sterilization pH ~7) | |

The inoculated first-stage medium is incubated in a 250-mL Erlenmeyer flask at 30°C for about 48 hours on a shaker orbiting in a two-inch (5.08 cm) circle at 250 rpm.

This incubated first-stage medium (1 mL) is used to inoculate 50 mL of a production medium having the following composition:

| Production Medium I | |
|---|---|
| Ingredient | Amount (g/L) |
| Glucose | 45 |
| Soybean grits | 35 |
| Blackstrap molasses | 3 |
| $CaCO_3$ | 2.5 |
| Tap water | q.s. 1 liter |
| (Presterilization pH ~6.9; post-sterilization pH ~6.8) | |

The inoculated production medium is incubated in a 250-mL wide-mouth Erlenmeyer flask at 25°C for 6 to 7 days on a shaker orbiting in a two-inch circle at 250 rpm.

### B. Tank Fermentation of A54145.1

In order to provide a larger volume of inoculum, 10 mL of incubated vegetative medium, prepared as described in Section A, is used to inoculate 400 mL of a second-stage growth medium having the same composition as that of the first-stage vegetative medium. This second-stage vegetative medium is incubated in a two-liter wide-mouth Erlenmeyer flask for about 24 hours at 30°C on a shaker orbiting in a two-inch circle at 250 rpm.

Incubated second-stage vegetative medium (800 mL) thus prepared is used to inoculate 115 liters of sterile production medium, prepared as described in Section A, except that 0.2 g/L of a silicone antifoam such as Sag-471 (Union Carbide) is added. The inoculated production medium is allowed to ferment in a 165-liter stirred fermentation tank for 6 to 7 days at a temperature of 25°C. Low airflow (0.25 v/v/m) and low rpm (200-300) in the stirred vessel maintain a dissolved oxygen level above 30% of air saturation. The pH is not allowed to rise above 7.5.

Example 2

Producing Antibiotic A54145 with Streptomyces fradiae A54145.2

A. Shake-flask Fermentation of A54145.2

Shake-flask fermentation is carried out as in Example 1, Section A, with the following exceptions:

1) the culture is Streptomyces fradiae NRRL 18159;
2) the vegetative medium has the following composition:

| Vegetative Medium II | |
|---|---|
| Ingredient | Amount (g/L) |
| Glucose | 10 |
| Potato starch | 30 |
| Soybean flour | 20 |
| Defatted cottonseed flour | 20 |
| $CaCO_3$ | 2 |
| Tap water | 1 liter |

3) the vegetative medium is incubated at 25°C; and
4) the production medium has the following composition:

| Production Medium II | |
|---|---|
| Ingredient | Amount (g/L) |
| Glucose | 25.0 |
| Soybean grits | 18.75 |
| Blackstrap molasses | 3.75 |
| Casein | 1.25 |
| $CaCO_3$ | 3.125 |
| Sodium acetate | 8.0 |
| Tap water | q.s. to 1 L |
| (Pre-sterilization pH ~6.9; post-sterilization pH ~6.8) | |

B. Tank Fermentation of A54145.2

Incubated vegetative medium prepared as described in Section A is used, and the procedures of Example 1, Section B, are followed with these following exceptions:

1) the amount of incubated vegetative medium used to inoculate the second-stage growth medium is 8 mL;
2) the amount of second-stage medium used to inoculate the production medium is 2 L;
3) the air flow is 0.125 v/v/m; and
4) the pH is allowed to rise above 7.5.

Example 3

Producing Antibiotic A54145 with Streptomyces fradiae A54145.3

The procedures of Example 2, Section B, are followed except that 1) the culture used is Streptomyces fradiae NRRL 18160, 2) dissolved oxygen is controlled at 40% of air saturation, 3) pH is controlled at 7.0 and 4) the production medium has the following composition:

| Production Medium III | |
|---|---|
| Ingredient | Amount (g/L) |
| Soybean flour | 20.0 |
| Glucose | 5.0 |
| Blackstrap molasses | 2.5 |
| $Fe(SO_4)\cdot(NH_4)_2SO_4\cdot 6H_2O$ | 0.6 |
| Silicon defoamer | 0.2 |
| Polypropylene glycol (M.W. 2000) | 0.1 |
| Tap water | q.s. to 1 liter |

Example 4

Isolating Antibiotic A54145

Procedure A: Whole fermentation broth from two 100-L tanks (217 L), prepared as described in Example 2, was filtered through a filter press with 3% filter aid (Hyflo Super-Cel, Manville Products, Lompoc CA). The filtrate (185 L) was adjusted to pH 6.4, using 5N HCl. Diaion HP-20 resin (20 L) was added to the filtrate. The initial effluent (85 L) and a water wash (60 L) were discarded. The resin was then eluted as follows:

| Eluate | Solvent No. | Amount |
|---|---|---|
| 1 | 4a | 40 L |
| 2 | 4 | 30 L |
| 3 | 4 | 30 L |

Eluate 1 was discarded.

Eluates 2 and 3 were combined and chromatographed on 2 L of IRA-68($OAc^-$) (2.5" x 32"). The initial effluent (60 L), a wash with Solvent No. 4 (10 L) and an eluate with 0.1N HOAc:$CH_3CN$ (1:1, 10 L) were discarded. The column was then eluted with 14 L of 1.0N HOAc:$CH_3CN$ (1:1). This fraction was concentrated under vacuum and lyophilized to give 101.1 g of antibiotic A54145.

Procedure B: Whole fermentation broth from a large tank (4600 L), prepared as described in Example 2, was adjusted to pH 6.5 with HCl and filtered through a filter press with the aid of 4% Celite 545 to give 4600 L of filtrate having a pH of 6.3.

The filtrate was absorbed batch-wise onto Diaion HP-20 resin (200 L), adjusted to pH 6.0 and maintained at this pH while stirring for 2 hours. The mixture was filtered, and the filtrate was discarded.

The saturated HP-20 resin was transferred to a small tank with a welded membrane. The resin was washed first with water (800 L), agitating for 35 minutes, and then with Solvent No. 4a (400 L), agitating for 3 minutes. These washes were discarded. The resin was then eluted twice with Solvent No. 4 (600 L), agitating for 35 minutes.

The eluates were combined (1200 L) and chromatographed on an IRA-68 resin column (100 L), equilibrated in Solvent No. 4 and washed with this solvent (500 L). The column was then eluted with $CH_3CN$:0.2N HOAc (1:1), discarding the first fractions (300 L) and combining, concentrating and lyophilizing subsequent fractions (750 L) to give 3.65 kg of antibiotic A54145.

Example 5

Separating A54145B, A54145C, A54145D and A54145E

Antibiotic A54145 (60 g), obtained as described in Example 4, was subjected to preparative HPLC using a Chromatospac 100, 4-L Quantum LP-1/C18 silica-gel column (3" x 39"). The antibiotic was dissolved in Solvent No. 1 and added to the column. Elution was monitored by UV at 280 nm.

Fractions were combined based on analytical HPLC as described supra, but detecting at 289 and 223 nm and collecting 500-mL fractions at a flow rate of 100 mL/min. The column was eluted as follows:

| Solvent | Fractions |
|---|---|
| 1 | 1-8 |
| 3b | 9-29 |
| 3c | 30-73 |
| 3d | 74-161 |
| 4 | 8-L strip |

On the basis of the analytical HPLC results, fractions 114-161 were combined to give a total of 8.5 g of antibiotic A54145 enriched with components B, C, D and E. This material was rechromatographed on a Chromatospac column, repeating the previous conditions, but detecting at 223 nm and using the following solvents:

| Solvent | Fractions |
|---|---|
| 1 | 1-8 |
| 3c | 9-41 |
| 3f | 42-60 |
| 3h | 61-83 |
| 4 | 8-L strip |

From this column, fractions 76-78 gave 1.75 g of A54145B-enriched material, fractions 79-83 gave 1.02 g of A54145C-enriched material, and the strip fraction gave 0.8 g of A54145D-enriched material.

Example 6

Separating A54145 Enriched with A54145A, A54145C and A54145F

A54145 (60 g), obtained as described in Example 4, Procedure B, was chromatographed as in Example 5, but using the following solvents:

| Solvent | Fractions |
|---|---|
| 1 | 1-8 |
| 3c | 9-102 |
| 4 | 103-122 |

Fractions were combined on the basis of analytical HPLC to give 2.54 g of A54145F-enriched material, 5.1 g of A54145A-enriched material and 10.56 g of A54145C-enriched material.

Example 7

Isolating A54145A

A54145A-enriched material (1 g), obtained as described in Example 6, was purified, using the following preparative HPLC system: two 1" x 12" stainless steel columns packed with Zorbax ODS (12μ) in series.

Detection: UV at 280 nm
Flow Rate: 9 mL/minute

The material was dissolved in Solvent No. 1 for injection onto the column. The column was eluted as follows:

| Solvent | Fractions[a] |
|---|---|
| 1 | 1-18 |
| 3a | 19-145 |
| 4 | 146-165 |

[a]Fraction volume = 18 mL

Fractions containing A54145A (fractions 86-96) were combined, concentrated under vacuum and lyophilized to give 212 mg of purified A54145A.

Characteristics of A54145A

| | |
|---|---|
| Mol. Wt.: | 1643 |
| Mol. Formula: | $C_{72}H_{109}N_{17}O_{27}$ |
| High Resolution FABMS(M+H): | Found: 1644.7778,<br>Calcd. for $C_{72}H_{110}N_{17}O_{27}$: 1644.7757 |
| UV (EtOH) $\lambda_{max}$: | 219 nm (ε 35,000), 280 (ε 5,250), shoulder 288 (ε 4,600) |
| IR (KBr): | essentially the same as that of A54145B, infra |
| Optical Rotation: | $[\alpha]_{589}^{25°C}$ No Rotation ($CH_3OH$)<br>$[\alpha]_{365}^{25°C}$ -14.0° (c 0.1, $CH_3OH$) |
| Amino-acid Analysis: | Asp 973(2), Thr 441(1), Glu 1056(2), Gly 528(1), Ala 549(1), Ile 469(1), Lys (1), Trp 465(1) |
| $^1H$ NMR spectrum (in $D_2O$): | Figure 1. |

Example 8

Isolating A54145B

The A54145B-enriched A54145 material obtained in Example 5 (500 mg) was chromatographed using the procedure of Example 7. The column was eluted as follows:

| Solvent No. | Fractions[a] |
|---|---|
| 1 | 1-16 |
| 3g | 17-95 |
| 5 | 96-115 |

[a]Fraction volume = 18 mL

Fractions containing A54145B (fractions 64-70) were combined, concentrated under vacuum and lyophilized to give 330 mg of purified A54145B.

Characteristics of A54145B

| | |
|---|---|
| Mol. Wt.: | 1657 |

| | |
|---|---|
| Mol. Formula: | $C_{73}H_{111}N_{17}O_{27}$ |
| High Resolution FABMS(M+H): | Found: 1658.7954,<br>Calcd. for $C_{73}H_{112}N_{17}O_{27}$: 1658.7914 |
| UV (EtOH) $\lambda_{max}$: | 220 nm (ε 41,854), 281 (ε 5,613), 289 (ε 5,084) |
| IR (KBr): | ranging from 3335 to 3313; 2930, 1660, 1531, 1407, 1255 $cm^{-1}$ (see Figure 9) |
| Optical Rotation: | $[\alpha]_{589}^{25°C}$ = -8.55° (c 0.47, $H_2O$)<br>$[\alpha]_{365}^{25°C}$ = -36.32° (c 0.47, $H_2O$) |
| Amino-acid Analysis: | Asp 1039(2), Thr 466(1), Glu 564(1), Gly 528(1), Ala 525(1), Ile 491(1), Lys 514 (1), Trp 491(1), 3-MG 512(1). |
| $^1H$ NMR spectrum (in $D_2O$): | Figure 2. |

Example 9

Isolating A54145C

A54145C-enriched material (11.76 g), obtained as described in Examples 5 and 6, was purified using the following preparative HPLC system:

Column: 2" x 60-cm stainless steel
Packing: Quantum LP-1/C18 silica gel (20 mμ)
Detection: UV at 280 nm
Flow Rate: 18 mL/min

The material was dissolved in pyridine/HOAc/$H_2O$ (1:1:98, 37 mL) for application to the column. The column was eluted as follows:

| Solvent No. | Fractions[a] |
|---|---|
| 1 | 1-10 |
| 3e | 11-160 |
| 3h | 161-550 |
| 4 | 551-582 |

[a]Fraction volume = 18 mL

Fractions containing A54145C were combined (fractions 320-331, 817.8 mg). This material (800 mg) was further purified by HPLC using a 1" x 20" stainless-steel column packed with Quantum LP-1/C18 (20 mμ) silica gel column, detecting as in Example 7, and applying the material in pyridine/HOAc/$H_2O$ (1:1:98, 15 mL). The column was eluted at a flow rate of 8 mL/min as follows:

| Solvent No. | Fractions[a] |
|---|---|
| 1 | 1-18 |
| 3f | 19-69 |
| 3g | 70-114 |
| 5 | 115-137 |

[a]Fraction volume = 16 mL

Fractions containing A54145C (fractions 84-86 and 92-98) were combined, concentrated and lyophilized to give 350 mg of C-enriched material.

This process was repeated with some variation in the solvents used, i.e., varying the amount of $CH_3CN$ in the solvent

and sometimes eliminating methanol in the solvent mixture, to give an additional 27.6 mg of purified A54145C.

Characteristics of A54145C

| | |
|---|---|
| Mol. wt.: | 1657 |
| Mol. Formula: | $C_{73}H_{111}N_{17}O_{27}$ |
| High Resolution FABMS(M+H): | Found: 1658.7905,<br>Calcd. for $C_{73}H_{112}N_{17}O_{27}$: 1658.7914 |
| UV (EtOH) $\lambda_{max}$: | 219 nm ($\varepsilon$ 29,500), 281 ($\varepsilon$ 4,200), 288 ($\varepsilon$ 3,600) |
| IR (KBr): | essentially the same as that of A54145B, supra; |
| Amino-acid Analysis: | Asp 934(2), Thr 414(1), Glu 594(1), Gly 501(1), Ala 459(1), Val 359(1), Lys 451(1), 3-MG 487(1), Trp 308(1) |
| $^{1}H$ NMR spectrum (in $D_2O$): | Figure 3. |

Example 10

Isolating A54145D

A54145D-enriched material (750 mg), obtained as described in Example 5 was purified using the preparative HPLC system described in Example 7, except that only one column was used. The material was applied to the column in 25 mL of solvent 1, and the column was eluted at a flow rate of 7.5 mL/min as follows:

| Solvent No. | Fractions[a] |
|---|---|
| 1 | 1-6 |
| 3g | 7-89 |
| 2k | 90-101 |
| 4 | 102-115 |

[a]Fraction volume = 15 mL

Fractions containing A54145D (19-22) were combined, concentrated and lyophilized to give 219 mg of material further enriched with A54145D.

This material was purified by a second HPLC column, using the same conditions except that 5% methanol was added to solvent 4 and solvent 2k was eliminated.

The fractions from this column containing A54145D (fractions 72-74) were combined, concentrated and lyophilized to give 70 mg of purified A54145D.

Characteristics of A54145D

| | |
|---|---|
| Mol. Wt.: | 1657 |
| Mol. Formula: | $C_{73}H_{111}N_{17}O_{27}$ |
| High Resolution FABMS(M+H): | Found: 1658.7913,<br>Calcd. for $C_{73}H_{112}N_{17}O_{27}$: 1658.7914 |
| UV (EtOH) $\lambda_{max}$: | 219 nm ($\varepsilon$ 37,500), 280 ($\varepsilon$ 5,040), 289 ($\varepsilon$ 4,500) |
| IR (KBr): | essentially the same as that of A54145B, supra; |
| Amino-acid Analysis: | Asp 1011(2), Thr 427(1), Glu 967(2), Gly 515(1), Ala 487(1), Ile 434(1), Lys 543(1), Trp 577(1) |
| $^{1}H$ NMR spectrum (in $D_2O$): | Figure 4. |

Example 11

Isolating A54145E

A54145E-enriched material (1.0 g), obtained as described in Example 5, was purified using a preparative HPLC system as in Example 9, but using a 1" x 20". The material was applied in 15 mL of solvent 1, and the column was eluted at a flow rate of 9 mL/min as follows:

| Solvent No. | Fractions[a] |
|---|---|
| 1 | 1-19 |
| 2h | 20-118 |
| 2j | 119-215 |
| 4 | 216-225 |

[a] Fraction volume = 18 mL

Fractions containing A54145E (fractions 147-160) were combined, concentrated and lyophilized to give 49.7 mg of material further enriched with A54145E.

This material was purified using two 9.4- x 250-mm Zorbax ODS (5μ) columns in series, detecting by UV at 280 nm. The material was applied to the column in 3 mL of solvent, and the column was eluted at a flow rate of 3.25 mL/min as follows:

| Solvent No. | Fractions[a] |
|---|---|
| 1 | 1-12 |
| 2i | 13-180 |
| 4 | 181-193 |

[a]Fraction volume = 6.5 mL

Fractions containing A54145E (fractions 143-160) were combined, concentrated and lyophilized to give 16.07 mg of purified A54145E.

Characteristics of A54145E

| | |
|---|---|
| Mol. Wt.: | 1671 |
| Mol. Formula: | $C_{74}H_{113}N_{17}O_{27}$ |
| High Resolution FABMS(M+H): | Found: 1672.8065,<br>Calcd. for $C_{74}H_{114}N_{17}O_{27}$: 1672.8069 |
| UV (EtOH) $\lambda_{max}$: | 221 nm (ε 29,714), 278 (ε 4577), 289 (4044) |
| IR (KBr): | essentially the same as that of A54145B, supra |
| Amino-acid Analysis: | Asp 826(2), Thr 367(1), Glu 494(1), Gly 437(1), Ala 422(1), Ile 378(1), Lys 410(1), Trp 387(1), 3-MG 437(1) |
| $^1H$ NMR spectrum (in $D_2O$): | Figure 5. |

Example 12

Isolating A54145F

A54145F-enriched material (800 mg), obtained as described in Example 6, was purified using an HPLC system as in Example 9, but with a 1" x 20" column. The material was applied to the column in 10 mL of solvent, and the column was eluted at a flow rate of 8 mL/min as follows:

| Solvent No. | Fractions[a] |
|---|---|
| 1 | 1-10 |
| 2f | 11-60 |
| 2g | 61-99 |
| 2k | 100-134 |
| 4 | 135-150 |

[a]Fraction volume = 16 mL

Fractions containing A54145F (fractions 120-128) were combined, concentrated and lyophilized to give 366.2 mg of purified A54145F.

Characteristics of A54145F

| | |
|---|---|
| Mol. Wt.: | 1629 |
| Mol. Formula: | $C_{71}H_{107}N_{17}O_{27}$ |
| High Resolution FABMS(M+H): | Found: 1630.7634,<br>Calcd. for $C_{71}H_{108}N_{17}O_{27}$: 1630.7601 |
| UV (EtOH) $\lambda_{max}$: | 219 nm (ε 36,750), 280 (ε, 5,100), 288 (ε 4,450) |
| IR (KBr): | essentially the same as that of A54145B, supra |
| Optical Rotation: | $[\alpha]_{589}^{25°C}$ = -3.0° (c 1.0, $H_2O$)<br>$[\alpha]_{365}^{25°C}$ = -6.0° (c 1.0, $H_2O$) |
| Amino-acid Analysis: | Asp 959(2), Thr 428(1), Glu 965(2), Gly 494(1), Ala 487(1), Val 363(1), Lys 492(1), Trp 452(1) |
| $^1H$ NMR spectrum (in $D_2O$): | Figure 6. |

Example 13

Isolating A54145A1

Procedure A:

A54145$A_1$-enriched material was obtained using the following procedure: Whole broth (103 L), prepared as described in Example 2, was treated as described in Example 4 Procedure A except that instead of the IRA-68($OAc^-$) column, the combined eluates were chromatographed over a 40- x 780-mm BioRex 5 ($Cl^-$) column, using gradient elution with a 0.1N - 1.0N NaCl solvent system and collecting 100-mL fractions.

Fractions containing A54145 were combined and desalted over a 40- x 400-mm HP-20 column, again collecting 100-mL fractions. Fractions containing A54145 were combined and lyophilized to give 12.08 g of antibiotic A54145.

A portion of this antibiotic A54145 (2 g) is subjected to preparative HPLC using a Waters PrepPak 500 (C18) column, using a linear gradient of water to $H_2O/CH_3CN$ (1:1) containing 1% $NH_4H_2PO_4$. Fractions containing A54145$A_1$ are collected and desalted over an HP-20 column, eluting with Solvent 4.

This step is repeated twice, and the $A_1$-enriched material is combined (937 mg).

The $A_1$-enriched material is chromatographed over two 1" x 12" Zorbax ODS columns in series as described in Example 7. Fractions containing A54145$A_1$ are eluted with Solvent 2j, combined, concentrated and lyophilized to give crude A54145$A_1$ (109 mg).

This material is further purified by repeating this step to give more purified A54145$A_1$ (69.29 mg).

The material is even further purified by repeating this procedure three times, using Solvents 3j, 3h and 3k, respectively. The product obtained is desalted over HP-20 to give purified A54145$A_1$ (12.21 mg).

Procedure B:

Whole fermentation broth (160 L) is prepared as described in Example 25. With this procedure, the fermentation volume increases with time; therefore, beginning at 138 hours, 10-L aliquots are removed at intervals and frozen. By harvest (287 hours), a total of 50 L is removed and frozen. The frozen broth is added back to the fermentation at harvest. The whole broth is filtered with a filter aid or separated using a centrifuge. A portion of the filtrate (55 L) is worked up using the procedure of Example 14. Fractions containing A54145$A_1$ are eluted with solvent 4, concentrated and freeze-dried. Following this procedure gave 111.3 g of A54145$A_1$-enriched material.

This material is chromagraphed over a 1" x 16" Zorbax C8 (12μ) column. The column is eluted with solvent 2h. Following this procedure gave 374 mg of further A54145$A_1$-enriched material, which contained approximately 46% A54145$A_1$, 19% A54145$B_1$, 14% A54145A, 13% A54145B and 8% of an unidentified material (HPLC analysis).

Preparative HPLC using appropriate solvents is carried out on the further purified material to obtain A54145$A_1$ in pure form.

Characteristics of A54145A1

| | |
|---|---|
| Mol. Wt.: | 1643 |
| Mol. Formula: | $C_{72}H_{109}N_{17}O_{27}$ |
| High Resolution FABMS(M+H): | Found: 1644.7691, |
| | Calcd. for $C_{72}H_{110}N_{17}O_{27}$: 1644.7757 |
| UV (EtOH) $\lambda_{max}$: | 220 nm (ε 41,623), 281 (ε, 5,750), 289 (ε 4,950) |
| Optical Rotation: | $[\alpha]_{589}^{25°C}$ -10.4° (c 0.69, $CH_3OH$) |
| Amino-acid Analysis: | Asp 1209(2), Thr 554(1), Glu 1209(2), Gly 636(1), Ala 617(1), Ile 576(1), Lys 604 (1), Trp 514(1) |
| $^1H$ NMR spectrum (in $D_2O$): | Figure 7. |

Example 14

Esolating A54145$B_1$

Whole fermentation broth (100 L), prepared as described in Example 3, was worked up as described in Example 4, Procedure A, except that chromatography on IRA-68 was omitted. The material was eluted with solvent 4a, concentrated and freeze-dried to give 248.2 g of crude antibiotic A54145.

A portion of this material (60 g) was chromatographed on a 2" x 60-cm LP-1/C18 silica gel column.

Detection: UV at 254 and 280 mm.
Flow Rate: 25 mL/minute/fraction.

The column was eluted as follows:

| Solvent No. | Fractions |
|---|---|
| 1 | 1-138 |
| 2f | 139-411 |
| 2h | 412-560 |
| 2m | 561-976 |
| 4 | 977-1000 |

Fractions containing A54145B and A54145$B_1$ were pooled as follows:

| Pool | Fraction | Weight(g) |
|---|---|---|
| 1 | 951-1000 | 1.10 |
| 2 | 635-667 | 4.62 |
| 3 | 685-719 | 3.95 |

The A54145B and A54145$B_1$-enriched fractions (Pools 2-3) were further purified over two 1" x 12" Amicon C18 columns.

Detection: UV at 280 mm.
Flow Rate: 20 mL/1.6 minute/fraction.

The columns were eluted with pyridine/ HOAc/$H_2O$/$CH_3CN$ (0.1/0.1/67.3/32.5). Fractions containing A54145B were combined to give 554 mg of A54145B, and fractions containing A54145$B_1$ were combined to give 207 mg of purified A54145$B_1$.

Other A54145B-enriched fractions (Pool 1) were also purified in this manner to give an additional 394.5 mg of A54145B.

Characteristics of A54145$B_1$

| | |
|---|---|
| Mol. Wt.: | 1657 |
| Mol. Formula: | $C_{73}H_{111}N_{17}O_{27}$ |
| High Resolution FABMS(M+H): | Found: 1658.7911 |
| | Calcd. for $C_{73}H_{112}N_{17}O_{27}$: 1658.7914 |
| UV (EtOH) $\lambda_{max}$: | 221 nm ($\varepsilon$ 39,100), 282 ($\varepsilon$, 5,500), 290 ($\varepsilon$ 4,740) |
| IR (KBr): | essentially the same as that of A54145B, supra |
| Amino-acid Analysis: | Asp 935(2), Thr 422(1), Glu 556(1), Gly 480(1), Ala 434(1), Ile 438(1), Lys 467(1), Trp 440(1), 3-MG 426(1) |
| $^1H$ NMR spectrum (in $D_2O$): | Figure 8. |

Example 15

Preparing A54145A Nucleus

A. Fermenting Actinoplanes utahensis

A stock culture of Actinoplanes utahensis NRRL 12052 is prepared and maintained under liquid nitrogen as described in U.S. Patent 4,524,135 (cols. 22-23) or on slants, using the following medium:

## MEDIUM A

| Ingredient | Amount |
|---|---|
| Oatmeal | 20.0 g |
| Sucrose | 20.0 g |
| Yeast (debittered dried brewer's) | 2.5 g |
| Corn distillers dried solubles* | 5.0 g |
| $K_2HPO_4$ | 1.0 g |
| Czapek's mineral stock** | 5.0 mL |
| Deionized water | q.s. to 1 liter |

*Nadrisol, National Distillers Products Co., 99 Park Ave., New York, NY

**Czapek's mineral stock has the following composition:

| Ingredient | Amount |
|---|---|
| $FeSO_4 \cdot 7H_2O$ (dissolved in 2 mL conc HCl) | 2 g |
| KCl | 100 g |
| $MgSO_4 \cdot 7H_2O$ | 100 g |
| Deionized water | q.s. to 1 liter |

For agar slants, 20.0 g agar is added to Medium A. Slants are incubated at 30° for about 8 to 10 days.

Liquid nitrogen suspension (1 mL) is used to inoculate 200 mL of a vegetative medium (Medium A). The inoculated vegetative medium is incubated in a 1-L wide-mouth Erlenmeyer flask at 30°C for about 76 hours on a shaker rotating through an arc two inches in diameter at 250 RPM.

The incubated vegetative medium (500 mL) is used to inoculate 10 liters of sterile production medium having the following composition:

| MEDIUM B | |
|---|---|
| Ingredient | Amount (g/L) |
| Peanut meal | 10.0 |
| Soluble meat peptone | 5.0 |
| Sucrose | 20.0 |
| $KH_2PO_4$ | 0.5 |
| $K_2HPO_4$ | 1.2 |
| $MgSO_4 \cdot 7H_2O$ | 0.25 |
| Tap water | q.s. to 1 liter |

Post-sterilization pH is ~6.9.

The inoculated production medium is allowed to ferment in a 14-liter fermentation tank at a temperature of about 30°C for about 68 hours. The fermentation medium is stirred with conventional agitators at about 600 RPM and aerated with sterile air to maintain the dissolved oxygen level at or above 30% of air saturation at atmospheric pressure.

B. Deacylating A54145A

A fermentation of A. utahensis is carried out as described in Section A. After incubating the production medium for about 68 hours, A54145A (50 g) in water (300 mL) is added. The pH of the medium is adjusted to 7.2 with NaOH.

The fermentation is allowed to continue at 30°C, stirring at 600 RPM to maintain the dissolved oxygen level at 30% or above, until deacylation is complete. Deacylation is measured by disappearance of activity vs. Micrococcus luteus (about 22 hrs).

C. Isolating A54145A and A54145F Nuclei

Whole fermentation broth (10 liters), obtained as described in Section B, was vacuum filtered with Hyflo SuperCel (2 L). The mycelial cake was washed with water (3 L) and then discarded. The original filtrate (9.5 L) and the mycelial wash (3 L) were combined. This solution was adjusted to pH 3.5 with HCl, mixed with 1.4 liters of HP-20 resin (Diaion High Porous Polymer, HP-Series, Mitsubishi Chemical Industries Limited, Tokyo, Japan), stirred for 1 hr and placed in a 6.5- x 75-cm column. The first effluent (13 L) and a water wash (adjusted to pH 2 with HOAc) were discarded. The column was then eluted with $CH_3CN:H_2O$ (1:9) (5 L). This eluate was concentrated under vacuum and lyophilized to give 14.25 g of crude nucleus.

The crude nucleus was subjected to preparative HPLC using the following system:

Column: 2" x 60-cm reverse phase silica gel (Quantum LP-1/$C_{18}$)
Detection: UV at 280 nm

The crude nucleus (14 g) was dissolved in Solvent No. 1 and injected onto the column. The column was eluted at a flow rate of 12 mL/min. as follows:

| Solvent No. | Fractions[a] |
|---|---|
| 1 | 1-16 |
| 2 | 17-269 |
| 4 | 270-330 |

[a]Fraction volume: 24 mL

Fractions containing A54145F nucleus (fractions 84-106) were combined, concentrated and lyophilized to give 1.95 g of impure A54145F nucleus, and fractions containing A54145A nucleus (fractions 150-250) were combined, concentrated and lyophilized to give 4.8 g of impure A54145A nucleus.

D. Purifying A54145A Nucleus

Impure A54145A nucleus (1.9 g), obtained as described in Section C, was purified by HPLC as in Section C, but using two 1" x 12" silica gel (Zorbax ODS, 12μ) columns in series.

The nucleus was applied to the column in solvent 1. The column was eluted at a flow rate of 8 mL/min as follows:

| Solvent No. | Fractions[a] |
|---|---|
| 1 | 1-25 |
| 2 | 26-93 |
| 2a | 94-177 |
| 4 | 178-200 |

[a]Fraction volume: 16 mL

Fractions containing A54145A nucleus (fractions 111-117) were combined, concentrated and lyophilized to give 457 mg of A54145A nucleus.

A54145A nucleus has the following characteristics:

| | |
|---|---|
| Mol. wt. (FABMS): | 1489 |
| pKa ($H_2O$): | 3.2, 4.3, 4.9, 5.6, 9.5 |
| UV (EtOH): | 217 nm (ε ~50,COO), 279 (ε 4,100), 288 (ε 3,600) |
| IR (KBr): | 3324, 3317, 3313, 3069, 1668, 1534, 1407, 1253, 1214 and 1200 $cm^{-1}$ (Figure 13) |
| Optical Rotation: | $\alpha_{589}^{25°C}$ = -4.93° (c, $H_2O$) |
| | $\alpha_{365}^{25°C}$ = -23.65° (c, $H_2O$) |
| Analysis, Found: | C, 45.36; H, 5.66; N, 12.74 |
| Amino-Acid Analysis: | Asp 722(2), Thr 321(1), Glu 734(2), Gly 368(1), Ala 362(1), Ile 339(1), Lys 362(1), Trp 286 (1), Sar 377(1) |
| $^1H$ NMR spectrum (in $D_2O$): | Figure 10. |

Example 16

Preparing A54145B Nucleus

A. Deacylating A54145B

A culture of Actinoplanes utahensis NRRL 12052, prepared in medium A, incubated for eight days at 30°C and stored at 4°C, was used to inoculate 50 mL of a vegetative medium (medium A) in a 250-mL Erlenmeyer flask. The vegetative medium was incubated at 30°C on a rotary shaker at 250 RPM for 48 hours.

Incubated vegetative medium (5 mL) was used to inoculate 100 mL of sterile production medium (medium B) in a 500-mL Erlenmeyer flask. The production medium was incubated at 30°C on a rotary shaker at 250 RPM for 120 hours.

Semi-pure antibiotic A54145B (100 mg) in water (2 mL) was added to the production flask. The culture was then incubated at 30°C on rotary shakers at 250 RPM for another 20 hours to achieve complete deacylation.

B. Isolating A54145B Nucleus

Whole fermentation broth (100 mL at pH 6.8), prepared as described in Section A, was vacuum filtered. The mycelial cake was washed with water (20 mL), and the water wash was combined with the filtrate. This solution was adjusted to pH 4.5 with HCl and added to an HP-20 resin column (38 mL). The initial efluent (112 mL) and a water wash (adjusted to pH 3 with HCl, 111 mL) were discarded. The column was then eluted with $CH_3CN:H_2O$ (3:1, 100 mL). This eluent was concentrated under vacuum and lyophilized to give 169 mg of crude A54145B nucleus.

C. Purifying A54145B Nucleus

The crude nucleus (169 mg) was dissolved in solvent No. 1 and injected on a preparative HPLC column, using the following system:

Column: Two 9.4- x 250-mm Zorbax ODS (5μ) columns in series

Detection: UV at 280 nm
Flow Rate: 2 mL/min

The column was eluted as follows:

| Solvent No. | Fractions[a] |
|---|---|
| 1 | 1-20 |
| 2b | 21-153 |
| 2d | 154-168 |
| 3h | 169-186 |
| 4 | 187-200 |

[a] Fraction volume: 4 mL

Fractions containing A54145B nucleus (#122-150) were combined, concentrated and lyophilized to give 39 mg of A54145B nucleus.

A54145B nucleus has the following characteristics: Mol. wt. (FABMS): 1503
UV (EtOH): 218 nm (ε 31,193), 365 (ε 472), 279 (ε 5,308), 288 (ε 4,615)
IR (KBr): 3346, 3338, 3327, 3324, 3319, 3315, 3309, 1669, 1665 and 1540 $cm^{-1}$
Analysis, Found: C, 41.20; H, 5.58; N, 12.33
Amino-Acid Analysis: Asp 1009(2), Thr 442(1), Glu 575(1), Gly 571(1), Ala 513(1), Ile 480(1), Lys 503(1), Trp 489 (1), 3-MG 557(1)
$^1H$ NMR spectrum (in $D_2O$): Figure 11.

Example 17

Preparing A54145C Nucleus

A. Deacylating A54145C

A production culture of Actinoplanes utahensis NRRL 12052 was obtained as described in Example 5, but using two 50-mL Erlenmeyer flasks each containing 10 mL of medium. Impure A54145C (10 mg) in water (0.2 mL) was added to each flask.

B. Isolating A54145C Nucleus

The culture was harvested and worked up as described in Example 16, using a 3-mL water wash and combining it with the filtrate. The wash/filtrate solution was adjusted to pH 4.5 with HCl and chromatographed over HP-20 resin (7 mL). The initial effluent (14 mL) and a water wash (adjusted to pH 3 with HCl, 10 mL) were discarded. The column was then eluted with $CH_3CN:H_2O$ (3:1, 12 mL). This eluate was concentrated and lyophilized to give 35.6 mg of crude A54145C nucleus.

C. Purifying A54145C

The crude nucleus (35 mg) was dissolved in solvent No. 1 (3 mL) and injected onto a preparative HPLC column, using the system of Example 28.
The column was eluted as follows:

| Solvent No. | Fractions[a] |
|---|---|
| 1 | 1-22 |
| 2 | 23-66 |
| 2c | 67-85 |
| 2e | 86-105 |
| 2h | 106-124 |
| 4 | 125-136 |

[a] Fraction volume: 6 mL

Fractions containing A54145C nucleus (#75-81) were combined, concentrated and lyophilized to give 5.5 mg of A54145C nucleus.

A54145C nucleus has a molecular weight (FABMS) of 1489. The $^1H$ NMR spectrum of A54145C nucleus (in $D_2O$) is shown in Figure 12

Example 18

Preparing NLys-(t-BOC)-A54145

Crude A54145 (25 g) was dissolved in water (200 mL adjusted to pH 3.7). This solution was adjusted to pH 9.1 with 5N NaOH and stirred at ambient temperature. Di-tert-butyl dicarbonate (32 mL) and tert-butanol (50 mL) were added, and the solution was stirred for 4½ hours. The solution was then lyophilized. The residue thus obtained was dissolved in water (400 mL) and extracted 3 times with equal volumes of dichloromethane. The aqueous phase was then concentrated and lyophilized to give 39 g of $N_{Lys}$-(t-BOC)-A54145.

The reaction was monitored by analytical HPLC using the following system:

Column: 4.6- x 250-mm Zorbax ODS ($C_{18}$, 5μ)
Detection: UV at 289 and 223 nm
Flow Rate: 2 mL/min
Solvent: $CH_3CN$:MeOH:0.04 M aq.$NH_4OAc$ (25:12.5:62.5)

This reaction was repeated except that the pH was maintained at 8.5-9.0 with NaOH and only 7 mL of di-tert-butyl dicarbonate was added. Under these conditions, the reaction took place in only 80 minutes.

Example 19

Preparing NLys-(t-BOC)-A54145 Nucleus

The procedure of Example 15, Section A, was repeated except that the vegetative medium had the following composition:

MEDIUM C

| Ingredient | Amount (g/L) |
|---|---|
| Sucrose | 20 |
| Soybean flour | 10 |
| $K_2HPO_4$ | 1.2 |
| $KH_2PO_4$ | 0.5 |
| $MgSO_4 \cdot 7_2O$ | 0.25 |
| Defoamer* | 0.3 |
| Tap water | q.s. to 1 L |
| Adjust pH to 6.8 if necessary | |

*Sag 471 (Union Carbide)

The first-stage medium had a volume of 800 mL (400 mL/2-L flask) and was incubated at 30°C on a rotary shaker for 72 hr.

The second stage medium (Medium C) had a volume of 950 L. The fermentation was carried out in a 350-gal. fermentor at 30°C, stirring at 155 RPM with an air flow of 28 cfm for 72 hours.

The second-stage medium was used to inoculate 810 L of production medium (Medium B) in a 350-gal. fermentor. This fermentation was carried out at 30°C, stirring at 130 RPM with an air flow of 10 cmf for 43 hours.

A portion of this production medium (15 L) was transferred to a 68-L fermentor and $N_{Lys}$-(t-BOC)-A54145 (prepared from 50 g of A54145 as described in Example 16 and dissolved in water) was added. The fermentation was continued at 30°C until deacylation was complete as determined by HPLC to give $N_{Lys}$-(t-BOC)-A54145 nucleus.

Example 20

Purifying NLys-(t-Boc)-A54145 Nucleus

Whole fermentation broth (18 L), obtained as described in Example 19, was vacuum filtered with 2-3 L of filter aid (Hyflo Super-Cel). The mycelial cake was rinsed with water (2 L) and filtered. The water wash was combined with the original filtrate (18 L). The mycelial cake was extracted with methanol (4 L) and again filtered. The methanol extract was concentrated to remove the methanol, and the aqueous solution remaining was lyophilized. The lyophilized material was reconstituted in water (500 mL) and combined with the filtrate and water wash to give a total volume of 20.5 L.

This solution was adjusted to pH 4.7 with HCl mixed with HP-20 resin (2.4 L), stirred for 2 hours and then placed in a 2" x 60" column. The initial effluent (22 L) and the initial water wash (adjusted to pH 3.5 with HOAc, 14.5 L) were discarded. The column was then eluted with $CH_3CN/H_2O$ in the following ratios and amounts: 5:95 (4 L); 1:9 (4 L); 15:85 (2 L); 3:7 (8 L). The 3:7 eluate was concentrated and lyophilized to give 23.0 g of purified $N_{Lys}$-(t-BOC)-A54145 nucleus.

Example 21

Separation of NLys-(t-BOC)-A54145A Nucleus and NLys--(t-BOC)- A54145F Nucleus

$N_{Lys}$-(t-BOC)-A54145 nucleus (23 g), prepared as described in Example 20, was subjected to preparative HPLC, using a Chromatospac 100 silica-gel column (4 L, Quantum LP-1/C18). The material was dissolved in Solvent 1 (200 mL) and added to the column. Elution was monitored by UV at 280 nm. The column was eluted at a flow rate of 60 mL/min as follows:

| Solvent No. | Fractions[a] |
|---|---|
| 1 | 1-5 |
| 3i | 6-70 |
| 4 | 71-73 |

[a]Fraction volume: 480 mL

Fractions were combined based on analytical HPLC, using the following system:

| | |
|---|---|
| Column : | 4.6- x 250-mm Zorbax ODS (5μ) |
| Detection: | UV at 223 nm |
| Solvent : | $CH_3CN$/MeOH/0.04 M aq. $NH_4OAc$ (12.5:5:82.5) |
| Flow rate: | 2 mL/min |

From this column, fractions 28-31 gave 1.8 g of $N_{Lys}$-(t-BOC)-A54145F nucleus, and fractions 39-42 gave 2.6 g of $N_{Lys}$-(t-BOC)-A54145A nucleus.

$N_{Lys}$-(t-BOC)-A54145F nucleus is deblocked using standard procedures to give A54145F nucleus.

A54145F nucleus has the following characteristics:

| | |
|---|---|
| Mol. Wt. (FABMS): | 1475 |
| UV (EtOH): | 218 nm (ε 30,672), 278 (ε 4,547), 289 (ε 3,883) |
| IR (KBr): | 3367, 3355, 3345, 3340, 3387, 1667 and 1542 $cm^{-1}$ |
| Analysis, Found: | C, 37.87; H, 4.88; N, 11.29 |
| Amino-Acid Analysis: | Asp 962 (2), Thr 415 (1), Glu 974 (2), Gly 510 (1), Ala 486 (1), Val 459 (1), Lys 488 (1), Trp 393 (1). |

Example 22

Preparation of Synthetic A54145B

A. Acylation

$N_{Lys}$-(t-BOC)-A54145B nucleus (930 mg, 0.58 mmoles) was dissolved in dimethylformamide (DMF) (anhyd.), and O-(n-decanoyl)-hydroxybenzotriazole active ester (335 mg, 1.16 mmoles) was added. The reaction mixture was stirred at room temperature under nitrogen for 3 hours when silica gel TLC, using a $CH_3CN/H_2O/HOAc$ (70:25:5) solvent system, indicated no starting material remained. The volatile solvent was removed under vacuum to give a near-white tacky residue. The residue was triturated with diethyl ether/$CHCl_3$ (2:1) (3 x 200 mL) and worked up to give 1.1 g of $N_{Lys}$-(t-BOC)-$N_{Trp}$-(n-decanoyl)-A54145B nucleus as a cream-white powder.

B. Deblocking

This product was added to a prechilled solution of TFA containing 10% anisole, and the solution was stirred for ~35 min. under nitrogen. Volatile solvents were removed under vacuum to give the product as an off-white residue. This residue was triturated with diethyl ether/$CHCl_3$ (3:1) (3 x 50 mL). The solids (TFA-salt) were collected by filtration and dissolved in $H_2O$ (50 mL). The pH of this solution was adjusted to ~6.3 with several drops of pyridine and filtered. The filtrate was lyophilized to give 940 mg of $N_{Trp}$-(n-decanoyl)-A54145B nucleus.

Example 23

Effect of Lipid Precursors, Media and Feeding Enzymatic Soy Digest on A54145 Production

A54145 fermentations were carried out as in Example 3, but using the following three production media, with and without lipid feeding:

| Medium A | |
|---|---|
| Ingredient | Amount (g/L) |
| Glucose | 25.0 |
| Soybean grits | 15.0 |
| Blackstrap molasses | 3.0 |
| Acid-hydrolyzed casein | 1.0 |
| $CaCO_3$ | 2.5 |
| Tap water | q.s. 1 liter |

(Pre-sterilization pH ~7.0; post-sterilization pH ~7.1)

| Medium B | |
|---|---|
| Ingredient | Amount (g/L) |
| Soybean flour | 20.0 |
| Glucose | 5.0 |
| Blackstrap molasses | 2.5 |
| $Fe(SO_4)\cdot(NH_4)_2SO_4\cdot6H_2O$ | 0.6 |
| Silicon defoamer | 0.2 |
| Polypropylene glycol (M.W. 2000) | 0.1 |
| Potato dextrin | 30.0 |
| Tap water | q.s. to 1 liter |

| Medium C | |
|---|---|
| Ingredient | Amount (g/L) |
| Soybean flour | 20.0 |
| Glucose | 5.0 |
| Blackstrap molasses | 2.5 |
| $Fe(SO_4)\cdot(NH_4)_2SO_4\cdot6H_2O$ | 0.6 |
| Silicon defoamer | 0.2 |
| Polypropylene glycol (M.W. 2000) | 0.1 |
| Tap water | q.s. to 1 liter |

Medium D

Medium C with an enzymatic-soy-digest (Hy Soy, Sheffield Products, Norwich New York) feeding.
Table XV summarizes the results of these studies.

**Table XV: EFFECT OF LIPID PRECURSORS AND MEDIA ON YIELDS AND FACTOR SIDE CHAINS OF A54145 IN A 165-L BIOREACTOR**

| Medium | Lipid Precursor[a] | Total Antibiotic (mcg/mL) | Factor Side Chains (%)[b] | | |
|---|---|---|---|---|---|
| | | | $iC_{10}$ | $nC_{10}$ | $aC_{11}$ |
| A | ---- | 97 | 68 | 20 | 12 |
| A | $nC_{10}$ | 179 | 20 | 79 | 1 |
| B | ---- | 570 | 70 | 17 | 13 |
| B | $nC_{10}$ | 1046 | 7 | 91 | 2 |
| C | ---- | 1100 | 76 | 14 | 10 |
| C | $nC_{10}/C_{18:1}$ | 2316 | 19 | 74 | 7 |
| D | $nC_{10}/C_{18:1}$ | 3570 | 21 | 71 | 8 |

a $nC_{10}$ = ethyl caprate
$nC_{10}/C_{18:1}$ = n-decanoic acid in methyl oleate (1:1)

b $iC_{10}$ = 8-methylnonanoyl
$nC_{10}$ = n-decanoyl
$aC_{11}$ = 8-methyldecanoyl

Example 24

Effect of Amino-Acid Enrichment on A54145 Production

A54145 fermentations were carried out as in Example 2, Section A, but using the culture used in Example 3 and the following production medium:

| Ingredient | Amount (g/L) |
|---|---|
| Glucose | 30.00 |
| Soybean flour | 25.0 |
| Blackstrap molasses | 5.0 |
| $CaCO_3$ | 4.0 |
| $Fe(SO_4)\cdot(NH_4)_2SO_4\cdot 6H_2O$ | 0.6 |
| Tap water | q.s. to 1 liter |

Different amino acids were added to study their effects on the A54145 nuclei and acyl side chains produced. Table XVI summarizes the results of these studies.

**Table XVI: EFFECT OF AMINO ACID ENRICHMENT ON BIOSYNTHESIS OF A54145 NUCLEI AND ACYL CHAINS IN SHAKEN FLASKS**

| Amino Acid | Level (M) | Total Antibiotic (%) | Nuclei[a] | | | | Acyl Chains[a,b] | | |
|---|---|---|---|---|---|---|---|---|---|
| | | | A | B | C | F | $iC_{10}$ | $nC_{10}$ | $aC_{11}$ |
| ----- | | 100[c] | 50 | 39 | 1 | 10 | 65 | 18 | 17 |
| L-Val | .03 | 32 | 32 | 20 | 2 | 54 | 98 | 0 | 2 |
| L-Leu | .02 | 56 | 42 | 40 | 2 | 16 | 76 | 7 | 17 |
| L-Ile | .04 | 73 | 48 | 47 | 2 | 0 | 16 | 18 | 66 |
| L-Glu | .02 | 85 | 49 | 39 | 1 | 11 | 63 | 20 | 16 |
| L-Asp | .005 | 134 | 56 | 34 | 1 | 10 | 64 | 19 | 17 |
| L-Tyr | .02 | 59 | 12 | 76 | 1 | 11 | 67 | 18 | 15 |

[a] Percent of total produced

[b] $iC_{10}$ = 8-methylnonanoyl
$nC_{10}$ = n-decanoyl
$aC_{11}$ = 8-methyldecanoyl

[c] $C_{550}$ mcg/mL

Table XVII summarizes the results of a similar study of the effect L-tyrosine has on A54145 production. Thus study was made in a 115-liter fermentation run.

Table XVII: EFFECT OF L-TYROSINE ENRICHMENT ON BIOSYNTHESIS OF A54145 NUCLEI IN A 165-L BIOREACTOR

| Amino Acid | Level (M) | Total Antibiotic (%) | Nuclei[a] | | | | Acyl Chains[a,b] | | |
|---|---|---|---|---|---|---|---|---|---|
| | | | A | B | C | F | $iC_{10}$ | $nC_{10}$ | $aC_{11}$ |
| ----- | ---- | 100[c] | 19 | 78 | 1 | 2 | 19 | 74 | 7 |
| L-Tyr | 0.01 | 102 | 10 | 87 | 1 | 1 | 28 | 67 | 5 |

[a] Percent of total produced

[b] $iC_{10}$ = 8-methylnonanoyl
$nC_{10}$ = n-decanoyl
$aC_{11}$ = 8-methyldecanoyl

[c] 3200 mcg/mL

Comparing the results in Tables XVI and XVII shows that the scale of the fermentation affects the amount of a) total antibiotic produced, b) nuclei produced and c) acyl side chains produced. Adding L-tyrosine decreased total antibiotic production in the shaken-flask fermentation, but did not adversely affect production in the tank fermentation. The unsupplemented shaken-flask fermentation produced more A nucleus and more $iC_{10}$ side chain product, whereas the unsupplemented tank fermentation produced more B nucleus and more $nC_{10}$ side chain. L-tyrosine increased the percentage of B-nucleus produced in both shaken flasks and tanks, but the effect was more pronounced in flasks.

Adding L-valine or L-leucine increased the percentage of F nucleus produced and the percentage of $iC_{10}$ side chain product. This effect was more pronounced with L-valine.

Adding L-isoleucine increased the percentage of both B nucleus and $aC_{11}$ side chain produced.

Example 25

An A54145 fermentation was carried out as described in Example 3 except that the following production medium was used:

| Ingredient | Amount (g/L) |
|---|---|
| Soybean flour | 30.0 |
| Blackstrap molasses | 5.0 |
| Glucose | 3.0 |
| $Fe(SO_4)\cdot(NH_4)_2SO_4\cdot 6H_2O$ | 0.6 |
| Deionized water | q.s. 1 liter |

Antifoam agents were added, and the pH was adjusted from ~6.2 to ~7.2 with 5N NaOH.

Beginning about 23 hours after the fermentation was initiated, glucose was fed to the fermentation at a rate of approximately 6.5 g/L/day. Beginning at about 25 hours after the fermentation was initiated, a sterile solution consisting of decanoic acid and oleic acid (1:1, v/v) was fed to the fermentation at a rate of approximately 6.0 mL/L/day.

At about 117 hours after the fermentation was initiated, a feeding of enzymatic soy digest was initiated and continued at a rate of about 3.0 g/L/day.

The yield of A54145 from the fermentation after about 280 hours was 3969 mcg/mL. This yield is substantially greater than the yield of about 500 mcg/mL ordinarily obtained using similar conditions, but without the glucose, enzymatic soy digest and decanoic acid feeds used in this fermentation.

Example 26

Another series of fermentations was carried out using the procedures of Example 23 with Medium C, but adding different $C_4$-$C_{18}$-alkanoic acids and esters to enhance A54145 production. The results of these studies are shown in Table XVIII.

Table XVIII: EFFECT OF LIPID PRECURSORS ON BIOSYNTHESIS OF A54145 SIDE CHAINS IN A 165-L BIOREACTOR

| | RQ[a] | | Total A54145 | Known Side Chains Percent of Total | | | New Analogs[b,c] | | | | | |
|---|---|---|---|---|---|---|---|---|---|---|---|---|
| Precursor | Calcd.[d] | Found[e] | (%) | $iC_{10}$ | $nC_{10}$ | $aC_{11}$ | $C_6A$ | $C_6B$ | $C_8A$ | $C_8B$ | $C_9A$ | $C_9B$ |
| None | 1.0 | 1.0 | 100[f] | 76 | 14 | 10 | | | | | | |
| Acetate | 1.0 | 1.0 | 104 | 73 | 15 | 11 | | | | | | |
| Propionate | 0.88 | 0.96 | 28 | 69 | 22 | 8 | | | | | | |
| Butyrate | 0.8 | 0.93 | 49 | 28 | 58 | 15 | | | | | | |
| Hexanoate | 0.75 | 0.83 | 56 | 2 | 2 | - | 67 | 29 | | | | |
| Caprylate | 0.73 | 0.8 | 84 | 17 | 9 | 5 | | | 33 | 36 | | |
| Nonanoate | 0.72 | 0.85 | 95 | - | - | - | | | | | 75 | 25 |
| Caprate | 0.71 | 0.86 | 184 | 17 | 91 | 2 | | | | | | |
| Undecanoate | 0.76 | 0.9 | 136 | 11 | 3 | 26 | | | | | 17 | 16 |
| Undecylenate | 0.71 | 0.87 | 153 | 27 | 56 | 2 | | | | | | |
| Laurate | 0.71 | 0.9 | 154 | 43 | 54 | 3 | | | | | | |
| Tridecanoate[g] | 0.7 | 0.76 | 64 | 40 | 19 | 5 | | | | | 12 | 28 |
| Myristate | 0.7 | 0.81 | 207 | 10 | 85 | 5 | | | | | | |
| Oleate | 0.7 | 0.9 | 142 | 49 | 48 | 3 | | | | | | |
| Decyl Alcohol | 0.67 | 0.86 | 157 | 22 | 75 | 3 | | | | | | |

[a] Respiration Quotient

[b] Abbreviations as follows: "$C_6A$" = A nucleus with a $C_6$ side chain

[c] Undecanoate and tridecanoate precursors each produced two additional unknown factors [amounts: 13 and 14% (undecanoate) and 7 and 8% (tridecanoate)]

[d] For metabolism as sole carbon source

[e] Represents glucose metabolism or co-metabolism with glucose

[f] 1100 mcg/mL

[g] In 50% methyl oleate

Examples 27-28

Preparation of $N_{Lys}$-(t-BOC)-$N_{Trp}$-(n-Undecanoyl)-A54145A Nucleus (Compound 1) and $N_{Trp}$-(n-Undecanoyl)-A54145A Nucleus (Compound 2)

A. n-Undecanoyl Trichlorophenyl Ester

Undecanoic acid (7.5 g) was added to a solution of 2, 4, 5-trichlorophenol (8.6 g) in tetrahydrofuran (THF) (175 mL, anhyd.). This mixture was chilled in an ice bath and stirred under nitrogen for 10 minutes. The reaction mixture was removed from the ice bath, and N,N-dicyclohexylcarbodiimide (DCC) (9.0 g) was added.

The reaction mixture was stirred overnight under nitrogen at room temperature and then was concentrated to a volume of about 100 mL under vacuum. The concentrate was filtered to remove precipitated material, and the filtrate was evaporated to dryness.

The residue obtained was dissolved in diethyl ether and hexane. This solution was concentrated under vacuum and filtered to remove the red-pink precipitate which formed. The filtrate was evaporated under vacuum. The white precipitate which formed was collected by filtration and dried to give 5.27 g of n-undecanoyl "active ester".

B. Acylation of $N_{Lys}$-(t-BOC)-A54145A Nucleus

$N_{Lys}$-(t-BOC)-A54145A nucleus (500 mg) was added to anhydrous DMF (15 mL) in a 50-mL round bottom flask. After the solution was purged with nitrogen, the undecanoyl "active" ester (300 mg) and hydroxybenzotriazole (HBT) (8 mg) were added. The reaction mixture was stirred under nitrogen at room temperature for 21 hours and then concentrated under vacuum to a low volume (about 1/10th). Diethyl ether (30 mL) was added, and the precipitate which formed was separated by sonicating the suspension and filtering. The precipitate was washed twice with diethyl ether (30 mL each) and dried under vacuum to give 425 mg of $N_{Lys}$-(t-BOC)-$N_{Trp}$-(n-undecanoyl)-A54145A nucleus (Compound 1).

C. Deblocking

The t-BOC derivative obtained in Section B was dissolved in TFA (5 mL) containing anisole (0.5 mL) and stirred at room temperature for one hour under nitrogen. The solution was concentrated under vacuum to a low volume. A precipitate was formed by adding $CH_2Cl_2$/diethyl ether (2:1, 30 mL). The precipitate was separated by filtration and washed twice with $CH_2Cl_2$/diethyl ether (2:1, 30 mL each) and dried. The residue was dissolved in water (10 mL). The pH of the solution (1.72) was adjusted to 6.2 with pyridine, and the solution was lyophilized to give 645.5 mg of product.

D. Purification by Preparative HPLC

The product obtained in Section C was purified by preparative HPLC, using the following conditions:

Column: 1- x 12-in. Zorbax ODS (Dupont, 12µ)
Detection: UV at 280 nm
Flow Rate: 9 mL/min

The material, dissolved in solvent 1 (5 mL), was injected onto the column. The column was eluted at a flow rate of 9 mL/min, collecting 18-mL fractions, as follows:

| Solvent | Fractions |
|---|---|
| 1 | 1-10 |
| 3g | 11-63 |
| 4 | 64-80 |

Fractions were monitored by analytical HPLC, using the following conditions:

Column: 4.6- x 250-mm Zorbax ODS (Dupont, 5µ)
Detection: UV at 223 nm
Flow Rate: 2 mL/min
Solvent: $CH_3CN$:MeOH:0.04 M aq. $NH_4OAc$ (25:12.5:62.5)

Fractions containing the product were combined, concentrated and lyophilized to give 241 mg of $N_{Trp}$-(n-undecanoyl)-A54145A nucleus (compound 2).

FABMS (M+1): 1658
UV (EtOH): 280 nm (ε 4,752) and 220 nm (ε 34,413).

Examples 29-30

Preparation of $N_{Lys}$-(t-BOC)-$N_{Trp}$-(n-Dodecanoyl)-A54145A Nucleus (Compound 3) and $N_{Trp}$-(n-Dodecanoyl)-A54145A Nucleus (Compound 4)

A. Preparation of n-Dodecanoyl Trichlorophenyl Ester

n-Dodecanoyl trichlorophenyl ester was prepared by heating n-dodecanoyl chloride (2.5 g) and 2,4,5-trichlorophenol (1.75 g) in diethyl ether (40 mL). The reaction mixture was stirred under nitrogen at room temperature until materials were dissolved. Pyridine (2 mL) was added, and the mixture was stirred under nitrogen at room temperature for 21 hours.

The reaction mixture was filtered to remove the precipitate which formed. The precipitate was washed with diethyl ether, and the diethyl ether wash was combined with the filtrate and concentrated under vacuum to give crude product.

This product was purified over a 2.5- x 30-cm silica-gel column (Woelm), eluting the n-dodecanoyl "active" ester with toluene. The fractions containing the ester were combined and concentrated to dryness to give 3.0 g of the "active" ester.

B. Acylation of $N_{Lys}$-(t-BOC)-A54145A Nucleus

The active ester prepared in Section A was used to acylate $N_{Lys}$-(t-BOC)-A54145A nucleus, using a procedure like that described in Examples 27-28, Section B, to give 438 mg of $N_{Lys}$-(t-BOC)-$N_{Trp}$-(n-dodecanoyl)-A54145A nucleus (Compound 3).

C. Deblocking

The material obtained in Section B was deblocked using the procedure of Examples 27-28, Section C, to give 630 mg of product.

The product was purified by preparative HPLC using the procedure of Examples 27-28, Section D, with the following eluting solvents:

| Solvent | Fractions |
|---|---|
| 1 | 1-13 |
| 3g | 14-86 |
| 4 | 87-105 |

The column was monitored by analytical HPLC as in Examples 27-28 except that the solvent system used was $CH_3CN$:0.04 M aq. $NH_4OAc$ (35:65). Fractions containing the product (#42-49) were combined, concentrated and lyophilized to give 225 mg of $N_{Trp}$-(n-dodecanoyl)-A54145A nucleus(Compound 4).

FABMS (M+1): 1671
UV (EtOH): 280 nm (ε 5,056) and 219 nm (ε 36,055).

Examples 31-32

Preparation of $N_{Lys}$-(t-BOC)-$N_{Trp}$-(n-Nonanoyl)-A54145A Nucleus (Compound 5) and $N_{Trp}$-(n-Nonanoyl)-A54154A Nucleus (Compound 6)

A. n-Nonanoyl Trichlorophenyl Ester

The procedure of Examples 27-28, Section A, was followed, except using 2.5 g (2.7 mL) of nonanoic acid, 2.9 g of 2,4,5-trichlorophenol, 55 mL of anhydrous THF and 3.0 g of DCC to give approximately 3 g of n-nonanoyl "active" ester.

B. Acylation of $N_{Lys}$-(t-BOC)-A54145A Nucleus

The active ester from Section A was used to acylate $N_{Lys}$-(t-BOC)-A54145A nucleus, using a procedure analogous to that of Examples 27-28, Section B, to give 437.1 mg of $N_{Lys}$-(t-BOC)-$N_{Trp}$-(n-nonanoyl)-A54145A nucleus.

C. Deblocking

The product obtained in Section B was deblocked, using a procedure like that of Examples 27-28, Section C, to give 605 mg of product.

The product was purified by preparative HPLC as described in Examples 27-28, but using the following solvents:

| Solvent | Fractions |
|---|---|
| 1 | 1-37 |
| 3c | 38-132 |
| 4 | 133-146 |

The column was monitored by analytical HPLC as an Example 27, except that the solvent system was $CH_3CN$:MeOH:0.04 M aq. $NH_4OAc$ (22.5:7.5:70). Fractions containing the product (#91-110) were combined, concentrated and lyophilized to give 178 mg of $N_{Trp}$-(n-nonanoyl)-A54145A nucleus.

FABMS (M+1): 1630
UV (EtOH): 279 nm (ε 5,459) and 220 nm (ε 39,124).

Examples 33-34

Preparation of $N_{Lys}$-(t-BOC)-$N_{Trp}$-(n-Decanoyl)-A-54145F Nucleus (Compound 9) and $N_{Trp}$-(n-Decanoyl)-A54145F Nucleus (Compound 10)

The n-decanoyl trichlorophenyl ester was prepared as described in Examples 27-28, Section A. This material was used to acylate $N_{Lys}$-(t-BOC)-A54145F nucleus, using a procedure analogous to that of Examples 27-28, Section B, to give about 400 mg of $N_{Lys}$-(t-BOC)-$N_{Trp}$-(n-decanoyl)-A54145F nucleus (Compound 9).

This material was deblocked using a procedure like that of Examples 1-2, Section C, to give 527.05 mg of product.

The product was purified using preparative HPLC as described in Examples 27-28, Section D, except that fractions of 16 mL were collected and the flow rate was 8 mL/min. The following solvents were used:

| Solvent | Fractions |
|---|---|
| 1 | 1-12 |
| 3d | 13-89 |
| 4 | 90-100 |

The column was monitored by analytical HPLC as in Examples 27-28, Section D, except that the solvent ratio was 25:5:70. Fractions containing the product (#55-67) were combined to give 160.37 mg of $N_{Trp}$-(n-decanoyl)-A54145F nucleus (Compound 10).

FABMS (M+1):
UV (EtOH):

Examples 35-36

Preparation of $N_{Lys}$-(t-BOC)-$N_{Trp}$-(n-Tetradecanoyl)-A54145A Nucleus (Compound 25) and $N_{Trp}$-(n-Tetradecanoyl) -A54145

Nucleus (Compound 26)

The general procedure described in Example 22 was followed, but using $N_{Lys}$-(t-BOC)-A54145A nucleus (550 mg), O-(n-tetradecanoyl) active ester (400 mg) and DMF (50 mL) to give 630 mg of Compound 25:

FABMS (P+1+Na): 1822.
Deblocking Compound 25 as in Examples 27-28 Procedure C, gave 550 mg of Compound 26:
FABMS (P+1): 1700.

Example 37

A54145-Improved Broiler Starter Ration

The following recipe provides a balanced broiler starter ration adapted to feed chicks for improved weight gains.

| Ingredient | Percent | Lbs/Ton |
|---|---|---|
| Ground Yellow Corn | 55.99 | 1119.8 |
| Animal - Vegetable Fat | 3.13 | 62.6 |
| Soybean Meal (48%) | 32.37 | 647.4 |
| Fish Meal | 2.50 | 50.0 |
| Feather Meal - Hydr. | 2.50 | 50.0 |
| Dicalcium Phosphate | 1.66 | 33.2 |
| Ground Limestone | 0.77 | 15.4 |
| Salt | 0.30 | 6.0 |
| Vitamin Premix[1] | 0.50 | 10.0 |
| Trace Mineral Premix[2] | 0.10 | 2.0 |
| Methionine Hyd. Anal. | 0.13 | 2.6 |
| A54145 Premix[3] | 0.05 | 1.0 |
| Total | 100.00 | 2000.0 |

[1]Vitamin premix provides 3000 IU of vitamin A, 900 ICU of vitamin $D_3$, 40 mg of vitamin E, 0.7 mg of vitamin K, 1000 mg of choline, 70 mg of niacin, 4 mg of pantothenic acid, 4 mg of riboflavin, 100 mcg of vitamin $B_{12}$, 100 mcg of biotin and 125 mg of ethoxyquin per kg of complete feed.

[2]Trace mineral premix provides 75 mg of manganese, 50 mg of zinc, 25 mg of iron, 1 mg of iodine and 0.1 mg of selenium per kg of complete feed.

[3]Premix containing 1-20 g of formula 1a compound per pound premix provides a corresponding 1-20 g of A54145 compound/ton of finished feed.

This diet is usually fed to chicks from day 1 until a day between 17 and 28.

Example 38

A54145-Improved Broiler Finisher Ration

The following recipe provides a balanced broiler finisher ration adapted to feed chicks for improved weight gains:

| Ingredient | Percent | Lbs/Ton |
| --- | --- | --- |
| Ground Yellow Corn | 66.35 | 1327.0 |
| Animal - Vegetable Fat | 1.53 | 30.6 |
| Corn Glut. Meal (60%) | 4.00 | 80.0 |
| Soybean Meal (48%) | 19.19 | 383.8 |
| Fish Meal | 2.50 | 50.0 |
| Feather Meal - Hydr. | 2.50 | 50.0 |
| Dicalcium Phosphate | 1.71 | 34.2 |
| Ground Limestone | 0.83 | 16.6 |
| Salt | 0.30 | 6.0 |
| Vitamin Premix[1] | 0.50 | 10.0 |
| Trace Mineral Premix[2] | 0.10 | 2.0 |
| Methionine Hyd. Anal. | 0.15 | 3.0 |
| Lysine HCl | 0.29 | 5.8 |
| A54145 Premix[3] | 0.05 | 1.0 |
| Total | 100.00 | 2000.0 |

[1]Vitamin premix provides 3000 IU of vitamin A, 900 ICU of vitamin $D_3$, 40 mg of vitamin E, 0.7 mg of vitamin K, 1000 mg of choline, 70 mg of niacin, 4 mg of pantothenic acid, 4 mg of riboflavin, 100 mcg of vitamin $B_{12}$, 100 mcg of biotin and 125 mg of ethoxyquin per kg of complete feed.

[2]Trace mineral premix provides 75 mg of manganese, 50 mg of zinc, 25 mg of iron, 1 mg of iodine and 0.1 mg of selenium per kg of complete feed.

[3]Premix containing 1-20 g of A54145 active compound per pound of premix provides a corresponding 1-20 g of A54145 compound/ton of finished feed.

## Claims

**Claims for the following Contracting States : AT, BE, CH, DE, FR, GB, IT, LI, NL, SE**

1. A compound of formula 1

```
NRR²-Trp-Glu-(HO)Asn-Thr-Sar-Ala-Asp-(Lys-R¹)
                     |                  |
                     O                  |
                     |                  |
                     X-Y-Asn-Gly-(MeO)Asp
```

**1**

wherein:

R is hydrogen, $C_8$-$C_{18}$-alkyl, optionally substituted $C_8$-$C_{18}$-alkanoyl or $C_8$-$C_{18}$-alkenoyl;
(Lys-$R^1$) represents -NH$(CH_2)_4$CH($NHR^1$)CO-;
$R^1$ and $R^2$ are hydrogen or an amino-protecting group; or
R and $R^2$, taken together, represent a $C_8$-$C_{18}$-alkylidene group;
X is Ile or Val; and
Y is Glu or 3-MG;

or a pharmaceutically acceptable salt thereof.

**2.** The lipopeptide antibiotic which can be produced by submerged aerobic fermentation of Streptomyces fradiae NRRL 18158, NRRL 18159 or NRRL 18160 in a culture medium containing assimilable sources of carbon, nitrogen and inorganic salts.

**3.** A compound of formula 4

```
NRR²-Trp-Glu-(HO)Asn-Thr-Sar-Ala-Asp-(Lys-R¹)
                     |                  |
                     O                  |
                     |                  |
                     X-Y-Asn-Gly-(MeO)Asp
```

**4**

wherein:

R is selected from the group consisting of hydrogen, an amino-protecting group, 8-methylnonanoyl, 8-methyldecanoyl and n-decanoyl; (Lys-$R^1$) represents -NH$(CH_2)_4$CH($NHR^1$)CO-;
$R^1$ is hydrogen or an amino-protecting group;
$R^2$ is hydrogen;
X is Ile or Val; and
Y is Glu or 3-MG;

provided that:
and

1) when $R^1$ = H, X = Ile and Y = Glu or 3-MG, R cannot be 8-methylnonanoyl, 8-methyldecanoyl or n-decanoyl;
2) when $R^1$ = H, X = Val and Y = 3-MG, R cannot be 8-methyldecanoyl; and
3) when $R^1$ = H, X = Val and Y = Glu, R cannot be 8-methylnonanoyl.

or a pharmaceutically acceptable salt thereof.

**4.** A compound of formula 5

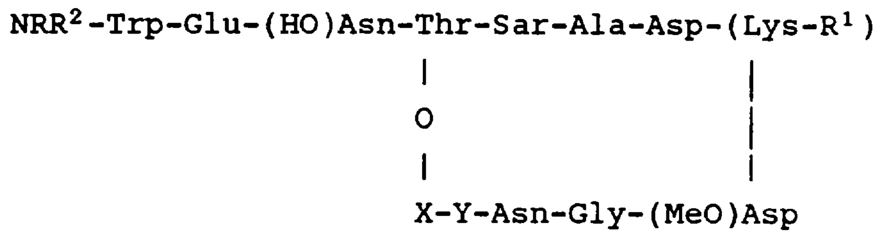

5

wherein:

R is $C_8$-$C_{18}$-alkyl, optionally substituted $C_8$-$C_{18}$-alkanoyl or $C_8$-$C_{18}$-alkenoyl;
(Lys-$R^1$) represents -NH$(CH_2)_4$CH(NH$R^1$)CO-;
$R^1$ is hydrogen or an amino-protecting group; and
$R^2$ is hydrogen; or
R and $R^2$, taken together, represent a $C_8$-$C_{18}$-alkylidene group;
X is Ile or Val; and
Y is Glu or 3-MG;

provided that: 1) when $R^1$ = H, X = Ile and Y = Glu or 3-MG, R cannot be 8-methylnonanoyl, 8-methyldecanoyl or n-decanoyl; 2) when $R^1$ = H, X = Val and Y = 3-MG, R cannot be 8-methyldecanoyl; and 3) when $R^1$ = H, X = Val and Y = Glu, R cannot be 8-methylnonanoyl;
or a pharmaceutically acceptable salt thereof.

**5.** Antibiotic A54145 of claim 2, or a pharmaceutically acceptable salt thereof.

**6.** Antibiotic A54145 component A of claim 1, wherein in formula 1 $R^2$ is H, X is Ile, Y is Glu and R is 8-methylnonanoyl, or a pharmaceutically acceptable salt thereof.

**7.** Antibiotic A54145 component $A_1$ of claim 1, wherein in formula 1 $R^2$ is H, X is Ile, Y is Glu and R is n-decanoyl, or a pharmaceutically acceptable salt thereof.

**8.** Antibiotic A54145 component B of claim 1, wherein in formula 1 $R^2$ is H, X is Ile, Y is 3-MG and R is n-decanoyl, or a pharmaceutically acceptable salt thereof.

**9.** Antibiotic A54145 component $B_1$ of claim 1, wherein in formula 1 $R^2$ is H, X is Ile, Y is 3-MG and R is 8-methylnonanoyl, or a pharmaceutically acceptable salt thereof.

**10.** Antibiotic A54145 component C of claim 1, wherein in formula 1 $R^2$ is H, x is Val, Y is 3-MG and R is 8-methyldecanoyl, or a pharmaceutically acceptable salt thereof.

**11.** Antibiotic A54145 component D of claim 1, wherein in formula 1 $R^2$ is H, X is Ile, Y is Glu and R is 8-methyldecanoyl, or a pharmaceutically acceptable salt thereof.

**12.** Antibiotic A54145 component E of claim 1, wherein in formula 1 $R^2$ is H, X is Ile, Y is 3-MG and R is 8-methyldecanoyl, or a pharmaceutically acceptable salt thereof.

**13.** Antibiotic A54145 component F of claim 1, wherein in formula 1 $R^2$ is H, X in Val, Y is Glu and R is 8-methylnonanoyl, or a pharmaceutically acceptable salt thereof.

**14.** A pharmaceutical composition comprising as an active ingredient a compound as claimed in any one of claims 1 to 2 or 4 to 13, associated with one or more pharmaceutically acceptable carriers therefor.

**15.** A compound as claimed in any one of claims 1 to 2 or 4 to 13 for use in the therapy of bacterial infections.

**16.** An animal feed premix comprising as an active ingredient a compound as claimed in any one of claims 1 to 2 or 4 to 13 associated with one or more agronomically acceptable carriers therefor.

**17.** A process for preparing a compound of formula 1 as claimed in any one of claims 1 and 3 to 13 which comprises:

A) cultivating Streptomyces fradiae NRRL 18158, NRRL 18159 or NRRL 18160, or an A54145-producing mutant thereof, in a culture medium containing assimilable sources of carbon, nitrogen, and inorganic salts under submerged aerobic fermentation conditions, optionally followed by separation of the antibiotic from the fermentation medium and/or salification of the antibiotic if not in salt form so as to produce a compound of formula 1 wherein:

$R^2$ and $R^1$ are hydrogen; and
R is methylnonanoyl, 8-methyldecanoyl or n-decanoyl;

provided that: when X is Val and Y is Glu, R must be 8-methylnonanoyl; and when X is Val and Y is 3-MG, R must be 8-methyldecanoyl;
B) optionally protecting the antibiotic with an amino-protecting group so as to prepare a compound of formula 1 wherein:

R and $R^1$ are an amino-protecting group; and
R is 8-methylnonanoyl, 8-methyldecanoyl or n-decanoyl;

provided that: when X is Val and Y is Glu, R must be 8-methylnonanoyl; and when X is Val and Y is 3-MG, R must be 8-methyldecanoyl;
C) enzymatically deacylating the product of Step (A) or (B) so as to prepare a compound of formula 1 wherein:

R is hydrogen; and
$R^1$ and $R^2$ are hydrogen or an amino-protecting group; and/or salifying the compound if not in salt form; and optionally

D)

i) reacylating the product prepared in Step C with the desired alkanoyl or alkenoyl side chain so as to prepare a compound of formula 1 wherein:

R is optionally substituted $C_8$-$C_{18}$-alkanoyl or $C_8$-$C_{18}$-alkenoyl; and
$R^1$ and $R^2$ are hydrogen or an amino-protecting group;

provided that $R^1$ cannot be hydrogen:

1) when X = Ile, Y = Glu or 3-MG and R is 8-methylnonanoyl, 8-methyldecanoyl or n-decanoyl;
2) when X = Val, Y = Glu and R = n-decanoyl; and
3) when X = Val, Y = 3-MG and R = 8-methyldecanoyl; or

ii) condensing the primary amino group of tryptophan of the compound prepared in Step B with an appropriate aldehyde or ketone so as to prepare the compound of formula 1 wherein R and $R^2$, taken together, represent a $C_8$-$C_{18}$-alkylidene group; and
iii) optionally reducing the Step (C)(ii) compounds so as to prepare a compound of formula 1 wherein R is $C_8$-$C_{18}$-alkyl;

and
F) optionally deblocking the Step C compound so as to prepare the formula 1 compound wherein $R^1$ and $R^2$ are hydrogen.

**18.** A process for preparing an A54145 component of the formula:

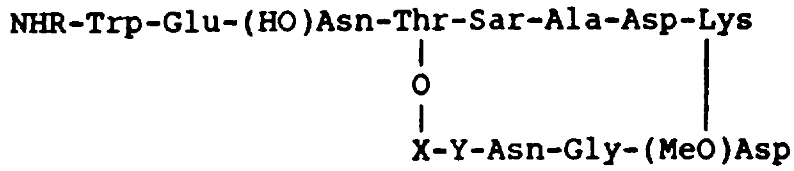

wherein R is selected from n-decanoyl, 8-methyldecanoyl and 8-methylnonanoyl;

X is Ile or Val; and
Y is Glu or 3-MG;
provided that: 1) when X = Val and Y = 3-MG, R must be 8-methyldecanoyl; and 2) when X = Val and Y = Glu, R must be 8-methylnonanoyl;

which comprises adding a $C_4$-$C_{18}$-alkanoic or alkenoic acid or alcohol, or an ester or salt thereof, to an A54145-producing culture after initiating the production stage of the fermentation, continuing until a recoverable amount of A54145 component is produced.

**19.** A process for preparing an A54145 cyclic peptide derivative of the formula:

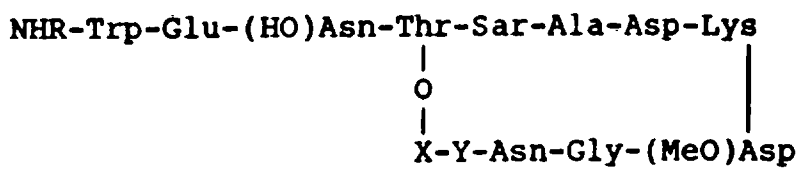

wherein:

R is $C_6$-$C_{10}$-alkanoyl;
X is Ile or Val; and
Y is Glu or 3-MG;

provided that:

1) when X = Ile and Y = Glu or 3-MG, R cannot be 8-methylnonanoyl, 8-methyldecanoyl or n-decanoyl;
2) when X = Val and Y = 3-MG, R cannot be 8-methyldecanoyl; and
3) when X = Val and Y = Glu, R cannot be 8-methylnonanoyl;

which comprises adding a $C_6$-$C_{10}$-alkanoic or alkenoic acid or alcohol, or an ester or salt thereof, to an A54145-producing culture after initiating the production stage of the fermentation, continuing until a recoverable amount of the A54145 derivative is produced.

**20.** A process for preparing an A54145 compound of the formula: .

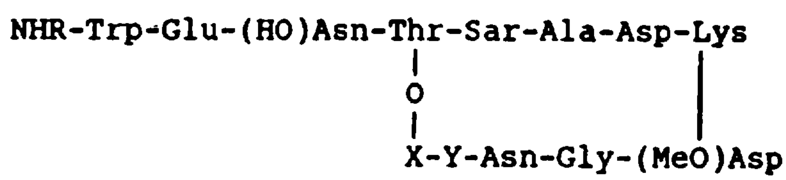

wherein R is $C_6$-$C_{10}$-alkanoyl;

X is Ile or Val; and
Y is Glu or 3-MG;

which comprises feeding glucose to an A54145-producing culture after initiating the production stage of the fermentation, continuing until a recoverable amount of A54145 compound is produced.

**21.** A process for preparing an A54145 compound of the formula:

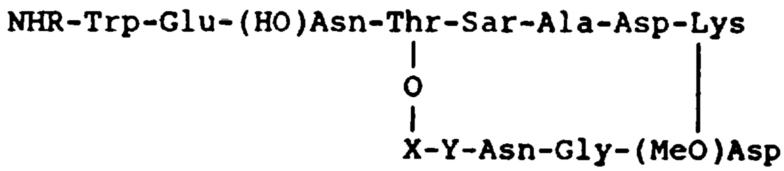

wherein R is $C_6$-$C_{10}$-alkanoyl;

X is Ile or Val; and
Y is Glu or 3-MG;

which comprises feeding an enzymatic soy digest to an A54145-producing culture after initiating the production stage of the fermentation, continuing until a recoverable amount of A54145 compound is produced.

**22.** A biologically purified culture of <u>Streptomyces</u> <u>fradiae</u> NRRL 18158, NRRL 18159 or NRRL 18160, or an A54145-producing mutant thereof.

**Claims for the following Contracting State : ES**

**1.** A process for preparing a compound of formula 1:

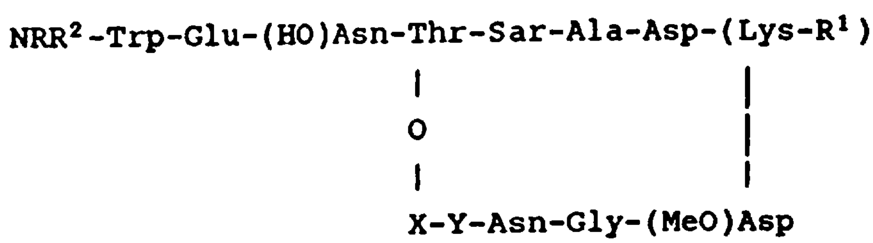

**1**

wherein:

R is hydrogen, $C_8$-$C_{18}$-alkyl, optionally substituted $C_8$-$C_{18}$-alkanoyl or $C_8$-$C_{18}$-alkenoyl;
(Lys-$R^1$) represents $-NH(CH_2)_4CH(NHR^1)CO-$;
$R^1$ and $R^2$ are hydrogen or an amino-protecting group; or
R and $R^2$, taken together, represent a $C_8$-$C_{18}$-alkylidene group;
X is Ile or Val; and
Y is Glu or 3-MG;

or a pharmaceutically acceptable salt thereof; which comprises:

<u>A)</u> cultivating <u>Streptomyces</u> <u>fradiae</u> NRRL 18158, NRRL 18159 or NRRL 18160, or an A54145-producing mutant thereof, in a culture medium containing assimilable sources of carbon, nitrogen, and inorganic salts under submerged aerobic fermentation conditions, optionally followed by separation of the antibiotic from the fermentation medium and/or salification of the antibiotic if not in salt form so as to produce a compound of formula <u>1</u> wherein:

$R^2$ and $R^1$ are hydrogen; and
R is methylnonanoyl, 8-methyldecanoyl or n-decanoyl;

provided that: when X is Val and Y is Glu, R must be 8-methylnonanoyl; and when X is Val and Y is 3-MG, R must be 8-methyldecanoyl;
<u>B)</u> optionally protecting the antibiotic with an amino-protecting group so as to prepare a compound of formula <u>1</u> wherein:

$R^2$ and $R^1$ are an amino-protecting group; and
R is 8-methylnonanoyl, 8-methyldecanoyl or n-decanoyl;

provided that: when X is Val and Y is Glu, R must be 8-methylnonanoyl; and when X is Val and Y is 3-MG, R must be 8-methyldecanoyl;
C) enzymatically deacylating the product of Step (A) or (B) so as to prepare a compound of formula 1 wherein:

R is hydrogen; and
$R^1$ and $R^2$ are hydrogen or an amino-protecting grpup; and/or salifying the compound if not in salt form; and optionally

D) i) reacylating the product prepared in Step C

with the desired alkanoyl or alkenoyl side chain so as to prepare a compound of formula 1 wherein:

R is optionally substituted $C_8$-$C_{18}$-alkanoyl or $C_8$-$C_{18}$-alkenoyl; and
$R^1$ and $R^2$ are hydrogen or an amino-protecting group;

provided that $R^1$ cannot be hydrogen:

1) when X = Ile, Y = Glu or 3-MG and R is 8-methylnonanoyl, 8-methyldecanoyl or n-decanoyl;
2) when X = Val, Y = Glu and R = n-decanoyl; and
3) when X = Val, Y = 3-MG and R = 8-methyldecanoyl;

or
ii) condensing the primary amino group of tryptophan of the compound prepared in Step B with an appropriate aldehyde or ketone so as to prepare the compound of formula 1 wherein R and $R^2$, taken together, represent a $C_8$-$C_{18}$-alkylidene group; and
iii) optionally reducing the Step (C)(ii) compounds so as to prepare a compound of formula 1 wherein R is $C_8$-$C_{18}$-alkyl;

and
F) optionally deblocking the Step C compound so as to prepare the formula 1 compound wherein $R^1$ and $R^2$ are hydrogen.

**2.** A process for preparing an A54145 component of the formula:

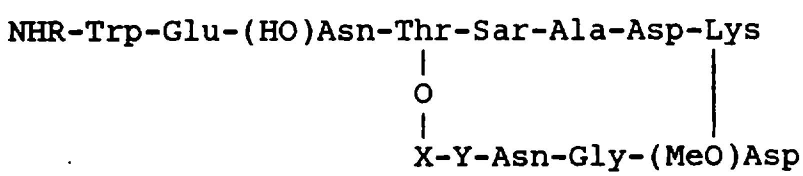

wherein R is selected from n-decanoyl, 8-methyldecanoyl and 8-methylnonanoyl;

X is Ile or Val; and
Y is Glu or 3-MG;

provided that: 1) when X = Val and Y = 3-MG, R must be 8-methyldecanoyl; and 2) when X = Val and Y = Glu, R must be 8-methylnonanoyl;
which comprises adding a $C_4$-$C_{18}$-alkanoic or alkenoic acid or alcohol, or an ester or salt thereof, to an A54145-producing culture after initiating the production stage of the fermentation, continuing until a recoverable amount of A54145 component is produced.

**3.** A process for preparing an A54145 cyclic peptide derivative of the formula:

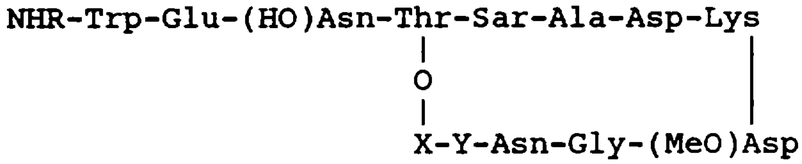

wherein:

R is $C_6$-$C_{10}$-alkanoyl;
X is Ile or Val; and
Y is Glu or 3-MG;

provided that:

1) when X = Ile and Y = Glu or 3-MG, R cannot be 8-methylnonanoyl, 8-methyldecanoyl or n-decanoyl;
2) when X = Val and Y = 3-MG, R cannot be 8-methyldecanoyl; and
3) when X = Val and Y = Glu, R cannot be 8-methylnonanoyl;

which comprises adding a $C_6$-$C_{10}$-alkanoic or alkenoic acid or alcohol, or an ester or salt thereof, to an A54145-producing culture after initiating the production stage of the fermentation, continuing until a recoverable amount of the A54145 derivative is produced.

**4.** A process for preparing an A54145 compound of the formula:

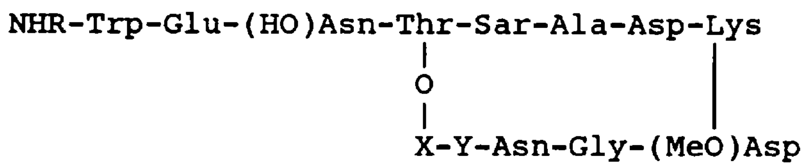

wherein R is $C_6$-$C_{10}$-alkanoyl;

X is Ile or Val; and
Y is Glu or 3-MG;

which comprises feeding glucose to an A54145-producing culture after initiating the production stage of the fermentation, continuing until a recoverable amount of A54145 compound is produced.

**5.** A process for preparing an A54145 compound of the formula:

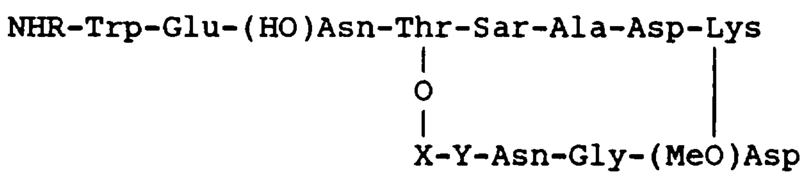

wherein R is $C_6$-$C_{10}$-alkanoyl;

X is Ile or Val; and
Y is Glu or 3-MG;

which comprises feeding an enzymatic soy digest to an A54145-producing culture after initiating the production stage of the fermentation, continuing until a recoverable amount of A54145 compound is produced.

**6.** A process for preparing a veterinary formulation comprising admixing a compound as claimed in any one of claims 1 to 2 or 4 to 14 with one or more agronomically acceptable carriers therefor.

**7.** A biologically purified culture of Streptomyces fradiae NRRL 18158, NRRL 18159 or NRRL 18160, or an

A54145-producing mutant thereof.

**Claims for the following Contracting State : GR**

1. A compound of formula 4

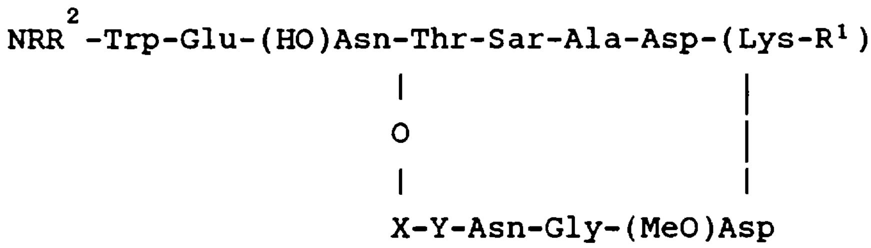

4

wherein:

R is selected from the group consisting of hydrogen, an amino-protecting group, 8-methylnonanoyl, 8-methyldecanoyl and n-decanoyl;
(Lys-$R^1$) represents $-NH(CH_2)_4CH(NHR^1)CO-$;
$R^1$ is hydrogen or an amino-protecting group;
$R^2$ is hydrogen;
X is Ile or Val; and
Y is Glu or 3-MG;

provided that:
and

1) when $R^1$ = H, X = Ile and Y = Glu or 3-MG, R cannot be 8-methylnonanoyl, 8-methyldecanoyl or n-decanoyl;
2) when $R^1$ = H, X = Val and Y = 3-MG, R cannot be 8-methyldecanoyl; and
3) when $R^1$ = H, X = Val and Y = Glu, R cannot be 8-methylnonanoyl.

or a pharmaceutically acceptable salt thereof.

2. A process for preparing a compound of formula 1:

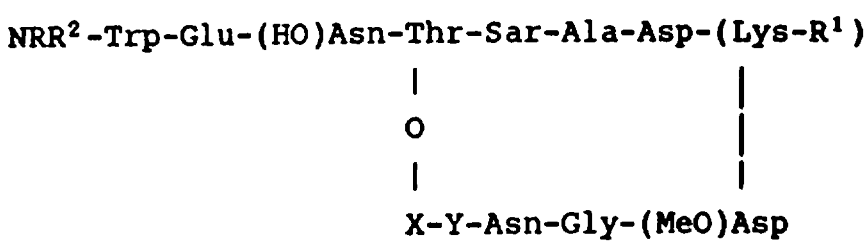

1

wherein:

R is hydrogen, $C_8$-$C_{18}$-alkyl, optionally substituted $C_8$-$C_{18}$-alkanoyl or $C_8$-$C_{18}$-alkenoyl;
(Lys-$R^1$) represents $-NH(CH_2)_4CH(NHR^1)CO-$;
$R^1$ and $R^2$ are hydrogen or an amino-protecting group; or
R and $R^2$, taken together, represent a $C_8$-$C_{18}$-alkylidene group;
X is Ile or Val; and

Y is Glu or 3-MG;

or a pharmaceutically acceptable salt thereof; which comprises:

A) cultivating Streptomyces fradiae NRRL 18158, NRRL 18159 or NRRL 18160, or an A54145-producing mutant thereof, in a culture medium containing assimilable sources of carbon, nitrogen, and inorganic salts under submerged aerobic fermentation conditions, optionally followed by separation of the antibiotic from the fermentation medium and/or salification of the antibiotic if not in salt form so as to produce a compound of formula 1 wherein:

$R^2$ and $R^1$ are hydrogen; and
R is methylnonanoyl, 8-methyldecanoyl or n-decanoyl;

provided that: when X is Val and Y is Glu, R must be 8-methylnonanoyl; and when X is Val and Y is 3-MG, R must be 8-methyldecanoyl;
B) optionally protecting the antibiotic with an amino-protecting group so as to prepare a compound of formula 1 wherein:

$R^2$ and $R^1$ are an amino-protecting group; and
R is 8-methylnonanoyl, 8-methyldecanoyl or n-decanoyl;

provided that: when X is Val and Y is Glu, R must be 8-methylnonanoyl; and when X is Val and Y is 3-MG, R must be 8-methyldecanoyl;
C) enzymatically deacylating the product of Step (A) or (B) so as to prepare a compound of formula 1 wherein:

R is hydrogen; and
$R^1$ and $R^2$ are hydrogen or an amino-protecting group; and/or salifying the compound if not in salt form; and optionally

D) i) reacylating the product prepared in Step C

with the desired alkanoyl or alkenoyl side chain so as to prepare a compound of formula 1 wherein:

R is optionally substituted $C_8$-$C_{18}$-alkanoyl or $C_8$-$C_{18}$-alkenoyl; and
$R^1$ and $R^2$ are hydrogen or an amino-protecting group;

provided that $R^1$ cannot be hydrogen:

1) when X = Ile, Y = Glu or 3-MG and R is 8-methylnonanoyl, 8-methyldecanoyl or n-decanoyl;
2) when X = Val, Y = Glu and R = n-decanoyl; and
3) when X = Val, Y = 3-MG and R = 8-methyldecanoyl;

or
ii) condensing the primary amino group of tryptophan of the compound prepared in Step B with an appropriate aldehyde or ketone so as to prepare the compound of formula 1 wherein R and $R^2$, taken together, represent a $C_8$-$C_{18}$-alkylidene group; and
iii) optionally reducing the Step (C)(ii) compounds so as to prepare a compound of formula 1 wherein R is $C_8$-$C_{18}$-alkyl;

and
F) optionally deblocking the Step C compound so as to prepare the formula 1 compound wherein $R^1$ and $R^2$ are hydrogen.

**3.** A process for preparing an A54145 component of the formula:

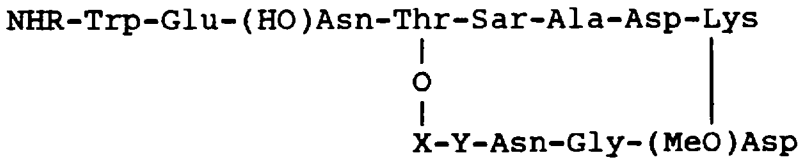

wherein R is selected from n-decanoyl, 8-methyldecanoyl and 8-methylnonanoyl;

X is Ile or Val; and
Y is Glu or 3-MG;

provided that: 1) when X = Val and Y = 3-MG, R must be 8-methyldecanoyl; and 2) when X = Val and Y = Glu, R must be 8-methylnonanoyl;
which comprises adding a $C_4$-$C_{18}$-alkanoic or alkenoic acid or alcohol, or an ester or salt thereof, to an A54145-producing culture after initiating the production stage of the fermentation, continuing until a recoverable amount of A54145 component is produced.

**4.** A process for preparing an A54145 cyclic peptide derivative of the formula:

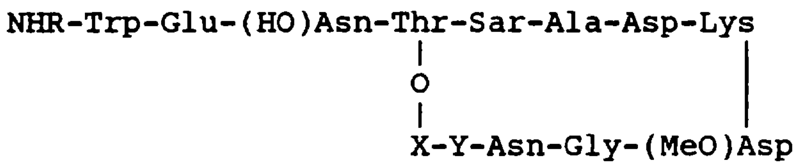

wherein:

R is $C_6$-$C_{10}$-alkanoyl;
X is Ile or Val; and
Y is Glu or 3-MG;

provided that:

1) when X = Ile and Y = Glu or 3-MG, R cannot be 8-methylnonanoyl, 8-methyldecanoyl or n-decanoyl;
2) when X = Val and Y = 3-MG, R cannot be 8-methyldecanoyl; and
3) when X = Val and Y = Glu, R cannot be 8-methylnonanoyl;

which comprises adding a $C_6$-$C_{10}$-alkanoic or alkenoic acid or alcohol, or an ester or salt thereof, to an A54145-producing culture after initiating the production stage of the fermentation, continuing until a recoverable amount of the A54145 derivative is produced.

**5.** A process for preparing an A54145 compound of the formula:

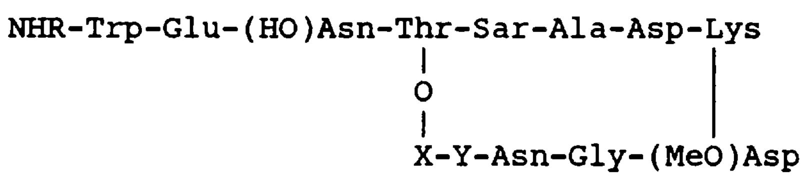

wherein R is $C_6$-$C_{10}$-alkanoyl;

X is Ile or Val; and
Y is Glu or 3-MG;

which comprises feeding glucose to an A54145-producing culture after initiating the production stage of-the fermentation, continuing until a recoverable amount of A54145 compound is produced.

6. A process for preparing an A54145 compound of the formula:

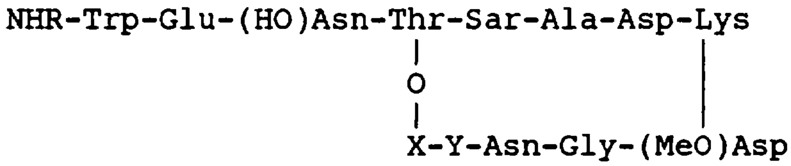

wherein R is $C_6$-$C_{10}$-alkanoyl;

X is Ile or Val; and
Y is Glu or 3-MG;

which comprises feeding an enzymatic soy digest to an A54145-producing culture after initiating the production stage of the fermentation, continuing until a recoverable amount of A54145 compound is produced.

7. A process for preparing a veterinary formulation comprising admixing a compound of formula 1 with one or more agronomically acceptable carriers therefor.

8. A biologically purified culture of Streptomyces fradiae NRRL 18158, NRRL 18159 or NRRL 18160, or an A54145-producing mutant thereof.

## Patentansprüche

### Patentansprüche für folgende Vertragsstaaten : AT, BE, CH, DE, FR, GB, IT, LI, NL, SE

1. Verbindung der Formel 1

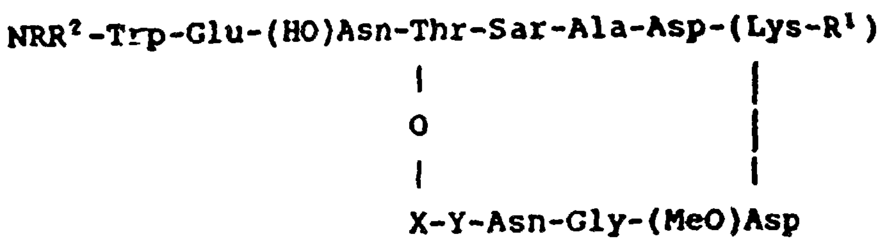

1

worin

R für Wasserstoff, $C_8$-$C_{18}$-Alkyl, wahlweise substituiertes $C_8$-$C_{18}$-Alkanoyl oder $C_8$-$C_{18}$-Alkenoyl steht,
(Lys-$R^1$) für -$NH(CH_2)_4CH(NHR^1)CO$- steht,
$R^1$ und $R^2$ für Wasserstoff oder eine Aminoschutzgruppe stehen, oder
R und $R^2$ zusammengenommen für eine $C_8$-$C_{18}$-Alkylidengruppe stehen,
X für Ile oder Val steht, und
Y für Glu oder 3-MG steht,
oder ein pharmazeutisch annehmbares Salz hiervon.

2. Lipopeptidantibiotikum, das durch submerse aerobe Fermentation von Streptomyces fradiae NRRL 18158, NRRL 18159 oder NRRL 18160 in einem Kulturmedium hergestellt werden kann, das assimilierbare Quellen für Kohlenstoff, Stickstoff und anorganische Salze enthält.

3. Verbindung der Formel 4

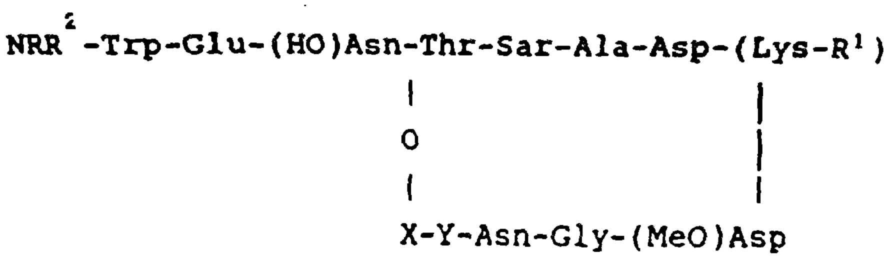

4

worin

R aus der Gruppe ausgewählt wird, die besteht aus Wasserstoff, einer Aminoschutzgruppe, 8-Methylnonanoyl, 8-Methyldecanoyl und n-Decanoyl,
(Lys-$R^1$) für -$NH(CH_2)_4CH(NHR^1)CO$- steht,
$R^1$ für Wasserstoff oder eine Aminoschutzgruppe steht,
$R^2$ für Wasserstoff steht,
X für Ile oder Val steht, und
Y für Glu oder 3-MG steht,

mit der Maßgabe, daß:

1) wenn $R^1$ = H, X = Ile und Y = Glu oder 3-MG ist, dann kann R nicht für 8-Methylnonanoyl, 8-Methyldecanoyl oder n-Decanoyl stehen,
2) wenn $R^1$ = H, X = Val und Y = 3-MG ist, dann kann R nicht für 8-Methyldecanoyl stehen, und
3) wenn $R^1$ = H, X = Val und Y = Glu ist, dann kann R nicht für 8-Methylnonanoyl stehen,

oder ein pharmazeutisch annehmbares Salz hiervon.

**4.** Verbindung der Formel 5

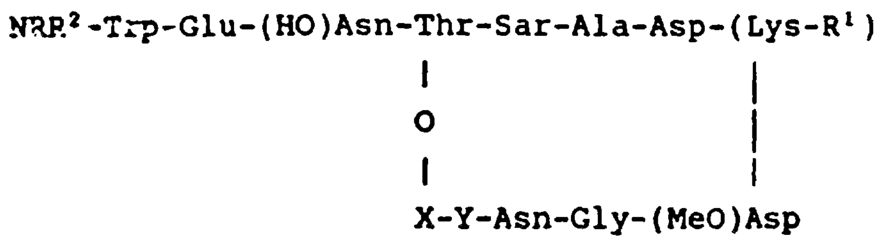

5

worin

R für $C_8$-$C_{18}$-Alkyl, wahlweise substituiertes $C_8$-$C_{18}$-Alkanoyl oder $C_8$-$C_{18}$-Alkenoyl steht,
(Lys-$R^1$) für -$NH(CH_2)_4CH(NHR^1)CO$- steht,
$R^1$ für Wasserstoff oder eine Aminoschutzgruppe steht, und
$R^2$ für Wasserstoff steht, oder
R und $R^2$ zusammengenommen für eine $C_8$-$C_{18}$-Alkylidengruppe stehen,
X für Ile oder Val steht, und
Y für Glu oder 3-MG steht,

mit der Maßgabe, daß:

1) wenn $R^1$ = H, X = Ile und Y = Glu oder 3-Mg ist, dann kann R nicht für 8-Methylnonanoyl, 8-Methyldecanoyl oder n-Decanoyl stehen,

2) wenn $R^1$ = H, X = Val und Y = 3-MG ist, dann kann R nicht für 8-Methyldecanoyl stehen, und
3) wenn $R^1$ = H, X = Val und Y = Glu ist, dann kann R nicht für 8-Methylnonanoyl stehen,

oder ein pharmazeutisch annehmbares Salz hiervon.

**5.** Antibiotikum A54145 nach Anspruch 2 oder ein pharmazeutisch annehmbares Salz hiervon.

**6.** Antibiotikum A54145 Komponente A nach Anspruch 1, worin in Formel 1 $R^2$ für H steht, X für Ile steht, Y für Glu steht und R für 8-Methylnonanoyl steht, oder ein pharmazeutisch annehmbares Salz hiervon.

**7.** Antibiotikum A54145 Komponente $A_1$ nach Anspruch 1, worin in Formel 1 $R^2$ für H steht, X für Ile steht, Y für Glu steht und R für n-Decanoyl steht, oder ein pharmazeutisch annehmbares Salz hiervon.

**8.** Antibiotikum A54145 Komponente B nach Anspruch 1, worin in Formel 1 $R^2$ für H steht, X für Ile steht, Y für 3-MG steht und R für n-Decanoyl steht, oder ein pharmazeutisch annehmbares Salz hiervon.

**9.** Antibiotikum A54145 Komponente $B_1$ nach Anspruch 1, worin in Formel 1 $R^2$ für H steht, X für Ile steht, Y für 3-MG steht und R für 8-Methylnonanoyl steht, oder ein pharmazeutisch annehmbares Salz hiervon.

**10.** Antibiotikum A54145 Komponente C nach Anspruch 1, worin in Formel 1 $R^2$ für H steht, X für Val steht, Y für 3-MG steht und R für 8-Methyldecanoyl steht, oder ein pharmazeutisch annehmbares Salz hiervon.

**11.** Antibiotikum A54145 Komponente D nach Anspruch 1, worin in Formel 1 $R^2$ für H steht, X für Ile steht, Y für Glu steht und R für 8-Methyldecanoyl steht, oder ein pharmazeutisch annehmbares Salz hiervon.

**12.** Antibiotikum A54145 Komponente E nach Anspruch 1, worin in Formel 1 $R^2$ für H steht, X für Ile steht, Y für 3-MG steht und R für 8-Methyldecanoyl steht, oder ein pharmazeutisch annehmbares Salz hiervon.

**13.** Antibiotikum A54145 Komponente F nach Anspruch 1, worin in Formel 1 $R^2$ für H steht, X für Val steht, Y für Glu steht und R für 8-Methylnonanoyl steht, oder ein pharmazeutisch annehmbares Salz hiervon.

**14.** Pharmazeutische Zusammensetzung, die als Wirkstoff eine Verbindung nach einem der Ansprüche 1 bis 2 oder 4 bis 13 zusammen mit einem oder mehreren pharmazeutisch annehmbaren Trägern hierfür enthält.

**15.** Verbindung nach einem der Ansprüche 1 bis 2 oder 4 bis 13 zur Verwendung bei der Therapie bakterieller Infektionen.

**16.** Tierfuttervormischung, die als aktiven Wirkstoff eine Verbindung nach einem der Ansprüche 1 bis 2 oder 4 bis 13 zusammen mit einem oder mehreren landwirtschaftlich annehmbaren Trägern hierfür enthält.

**17.** Verfahren zur Herstellung einer Verbindung der Formel 1 nach einem der Ansprüche 1 und 3 bis 13, das gekennzeichnet ist durch:

A) Kultivierung von Streptomyces fradiae NRRL 18158, NRRL 18159 oder NRRL 18160 oder einer A54145-bildenden Mutante hiervon in einem Kulturmedium, das assimilierbare Quellen für Kohlenstoff, Stickstoff und anorganische Salze enthält, unter submersen aeroben Fermentationsbedingungen, wahlweise gefolgt von einer Abtrennung des Antibiotikums vom Fermentationsmedium und/oder Salzbildung des Antibiotikums falls es nicht in Salzform vorliegt unter Bildung einer Verbindung der Formel 1 worin:

$R^2$ und $R^1$ für Wasserstoff stehen, und
R für Methylnonanoyl, 8-Methyldecanoyl oder n-Decanoyl steht mit der Maßgabe, daß:
wenn X für Val steht und Y für Glu steht, R für 8-Methylnonanoyl stehen muß und wenn X für Val steht und Y für 3-MG steht, R für 8-Methyldecanoyl stehen muß,

B) wahlweises Schützen des Antibiotikums mit einer Aminoschutzgruppe, um eine Verbindung der Formel 1 herzustellen, worin:

R und $R^1$ für eine Aminoschutzgruppe stehen, und

R für 8-Methylnonanoyl, 8-Methyldecanoyl oder n-Decanoyl steht, mit der Maßgabe, daß:
wenn X für Val steht und Y für Glu steht, R für 8-Methylnonanoyl stehen muß und wenn X für Val steht und Y für 3-MG steht, R für 8-Methyldecanoyl stehen muß,

C) enzymatische Deacylierung des Produkts von Schritt (A) oder (B), um eine Verbindung der Formel 1 herzustellen, worin:

R für Wasserstoff steht, und
$R^1$ und $R^2$ für Wasserstoff oder eine Aminoschutzgruppe stehen, und/oder Salzbildung der Verbindung falls sie nicht in Salzform vorliegt, und wahlweise

D)

i) Reacylierung des in Schritt C hergestellten Produkts mit der gewünschten Alkanoyl- oder Alkenoylseitenkette, um eine Verbindung der Formel 1 herzustellen, worin:

R für wahlweise substituiertes $C_8$-$C_{18}$-Alkanoyl oder $C_8$-$C_{18}$-Alkenoyl steht, und
$R^1$ und $R^2$ für Wasserstoff oder eine Aminoschutzgruppe stehen, mit der Maßgabe, daß $R^1$ nicht für Wasserstoff stehen kann:

1) wenn X = Ile, Y = Glu oder 3-MG ist und R für 8-Methylnonanoyl, 8-Methyldecanoyl oder n-Decanoyl steht,
2) wenn X = Val, Y = Glu und R = n-Decanoyl ist, und
3) wenn X = Val, Y = 3-MG und R = 8-Methyldecanoyl ist, oder

ii) Kondensierung der primären Aminogruppe von Tryptophan der in Schritt B hergestellten Verbindung mit einem geeigneten Aldehyd oder Keton, um die Verbindung der Formel 1 herzustellen, worin R und $R^2$ zusammengenommen für eine $C_8$-$C_{18}$-Alkylidengruppe stehen, und
iii) wahlweise Reduktion der Verbindungen von Schritt (C)(ii), um eine Verbindung der Formel 1 herzustellen, worin R für $C_8$-$C_{18}$-Alkyl steht, und

F) wahlweise Abspalten der Schutzgruppen von der Verbindung von Schritt C, um eine Verbindung der Formel 1 herzustellen, worin $R^1$ und $R^2$ für Wasserstoff stehen.

**18.** Verfahren zur Herstellung einer A54145 Komponente der Formel

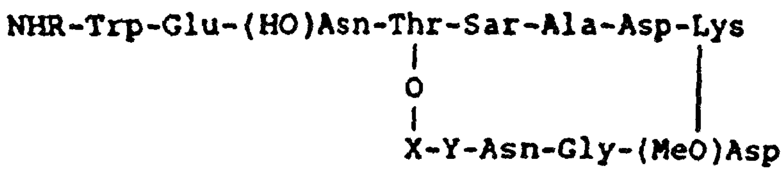

worin R ausgewählt ist aus n-Decanoyl, 8-Methyldecanoyl und 8-Methylnonanoyl,

X für Ile oder Val steht, und
Y für Glu oder 3-MG steht,

mit der Maßgabe, daß:

1) wenn X = Val und Y = 3-MG ist, R für 8-Methyldecanoyl stehen muß und
2) wenn X = Val und Y = Glu ist, R für 8-Methylnonanoyl stehen muß,

gekennzeichnet durch Zugabe einer $C_4$-$C_{18}$-Alkan- oder Alkensäure oder eines $C_4$-$C_{18}$-Alkan- oder Alkenalkohols oder eines Esters oder Salzes hiervon zu einer A54145-bildenden Kultur nach dem Auslösen der Produktionsstufe der Fermentation und Beibehaltung der Zugabe, bis eine gewinnbare Menge der A54145 Komponente gebildet ist.

**19.** Verfahren zur Herstellung eines cyclischen A54145 Peptidderivats der Formel:

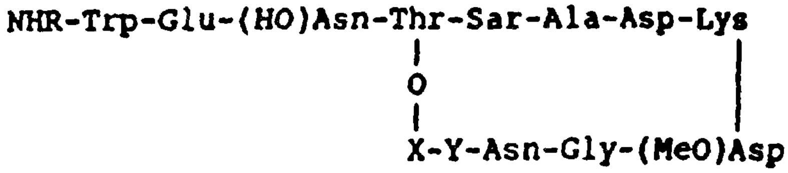

worin

R für $C_6$-$C_{10}$-Alkanoyl steht,
X für Ile oder Val steht, und
Y für Glu oder 3-MG steht,

mit der Maßgabe, daß:

1) wenn X = Ile und Y = Glu oder 3-MG ist, R nicht für 8-Methylnonanoyl, 8-Methyldecanoyl oder n-Decanoyl stehen kann,
2) wenn X = Val und Y = 3-MG ist, R nicht für 8-Methyldecanoyl stehen kann, und
3) wenn X = Val und Y = Glu ist, R nicht für 8-Methylnonanoyl stehen kann,

gekennzeichnet durch Zugabe einer $C_6$-$C_{10}$-Alkan- oder Alkensäure oder eines $C_6$-$C_{10}$-Alkan- oder Alkenalkohols oder eines Esters oder Salzes hiervon zu einer A54145-bildenden Kultur nach dem Auslösen der Produktionsstufe der Fermentation und Beibehaltung der Zugabe, bis eine gewinnbare Menge des A54145 Derivats gebildet ist.

**20.** Verfahren zur Herstellung einer A54145 Verbindung der Formel

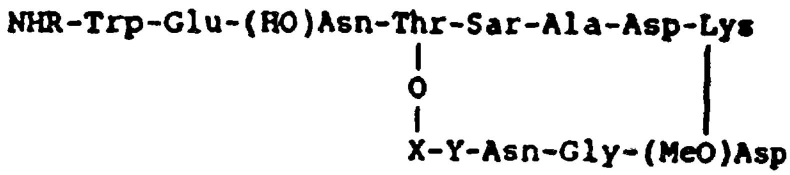

worin R für $C_6$-$C_{10}$-Alkanoyl steht,

X für Ile oder Val steht, und
Y für Glu oder 3-MG steht,

gekennzeichnet durch Zufütterung von Glucose zu einer A54145-bildenden Kultur nach dem Auslösen der Produktionsstufe der Fermentation und Beibehaltung der Zugabe, bis eine gewinnbare Menge der A54145 Verbindung gebildet ist.

**21.** Verfahren zur Herstellung einer A54145 Verbindung der Formel

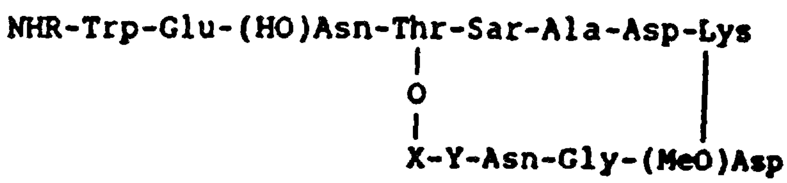

worin R für $C_6$-$C_{10}$-Alkanoyl steht,

X für Ile oder Val steht, und'
Y für Glu oder 3-MG steht,

gekennzeichnet durch Zufütterung eines enzymatischen Sojaverdaus zu einer A54145-bildenden Kultur nach dem Auslösen der Produktionsstufe der Fermentation und Beibehaltung der Zugabe, bis eine gewinnbare Menge der A54145 Verbindung gebildet ist.

**22.** Biologisch gereinigte Kultur von Streptomyces fradiae NRRL 18158, NRRL 18159 oder NRRL 18160 oder einer A54145-bildenden Mutante hiervon.

**Patentansprüche für folgenden Vertragsstaat : ES**

**1.** Verfahren zur Herstellung der Verbindung der Formel 1

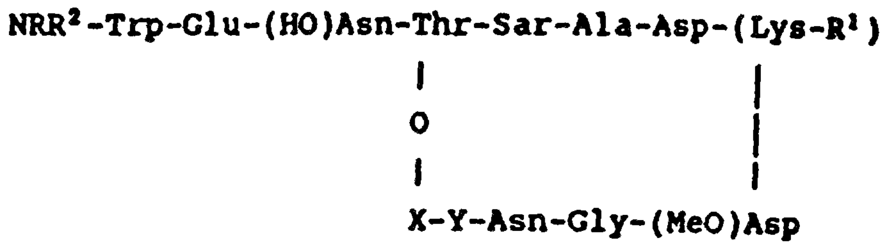

1

worin

R für Wasserstoff, $C_8$-$C_{18}$-Alkyl, wahlweise substituiertes $C_8$-$C_{18}$-Alkanoyl oder $C_8$-$C_{18}$-Alkenoyl steht,
(Lys-$R^1$) für $-NH(CH_2)_4CH(NHR^1)CO-$ steht,
$R^1$ und $R^2$ für Wasserstoff oder eine Aminoschutzgruppe stehen, oder
R und $R^2$ zusammengenommen für eine $C_8$-$C_{18}$-Alkylidengruppe stehen,
X für Ile oder Val steht, und
Y für Glu oder 3-MG steht,

oder eines pharmazeutisch annehmbaren Salzes hiervon, gekennzeichnet durch

A) Kultivierung von Streptomyces fradiae NRRL 18158, NRRL 18159 oder NRRL 18160 oder einer A54145-bildenden Mutante hiervon in einem Kulturmedium, das assimilierbare Quellen für Kohlenstoff, Stickstoff und anorganische Salze enthält, unter submersen aeroben Fermentationsbedingungen, wahlweise gefolgt von einer Abtrennung des Antibiotikums vom Fermentationsmedium und/oder Salzbildung des Antibiotikums falls es nicht in Salzform vorliegt unter Bildung einer Verbindung der Formel 1 worin:

$R^2$ und $R^1$ für Wasserstoff stehen, und
R für Methylnonanoyl, 8-Methyldecanoyl oder n-Decanoyl steht mit der Maßgabe, daß:
wenn X für Val steht und Y für Glu steht, R für 8-Methylnonanoyl stehen muß und wenn X für Val steht und Y für 3-MG steht, R für 8-Methyldecanoyl stehen muß,

B) wahlweises Schützen des Antibiotikums mit einer Aminoschutzgruppe, um eine Verbindung der Formel 1 herzustellen, worin:

$R^2$ und $R^1$ für eine Aminoschutzgruppe stehen, und
R für 8-Methylnonanoyl, 8-Methyldecanoyl oder n-Decanoyl steht, mit der Maßgabe, daß:
wenn X für Val steht und Y für Glu steht, R für 8-Methylnonanoyl stehen muß und wenn X für Val steht und Y für 3-MG steht, R für 8-Methyldecanoyl stehen muß,

C) enzymatische Deacylierung des Produkts von Schritt (A) oder (B), um eine Verbindung der Formel 1 herzustellen, worin:

R für Wasserstoff steht, und
$R^1$ und $R_2$ für Wasserstoff oder eine Aminoschutzgruppe stehen, und/oder Salzbildung der Verbindung falls sie nicht in Salzform vorliegt, und wahlweise

D)

i) Reacylierung des in Schritt C hergestellten Produkts mit der gewünschten Alkanoyl- oder Alkenoylseitenkette, um eine Verbindung der Formel 1 herzustellen, worin:

R für wahlweise substituiertes $C_8$-$C_{18}$-Alkanoyl oder $C_8$-$C_{18}$-Alkenoyl steht, und
$R^1$ und $R^2$ für Wasserstoff oder eine Aminoschutzgruppe stehen, mit der Maßgabe, daß $R^1$ nicht für Wasserstoff stehen kann:

1) wenn X = Ile, Y = Glu oder 3-MG ist und R für 8-Methylnonanoyl, 8-Methyldecanoyl oder n-Decanoyl steht,
2) wenn X = Val, Y = Glu und R = n-Decanoyl sit, und
3) wenn X = Val, Y = 3-MG und R = 8-Methyldecanoyl ist, oder

ii) Kondensierung der primären Aminogruppe von Tryptophan der in Schritt B hergestellten Verbindung mit einem geeigneten Aldehyd oder Keton, um die Verbindung der Formel 1 herzustellen, worin R und $R^2$ zusammengenommen für eine $C_8$-$C_{18}$-Alkylidengruppe stehen, und
iii) wahlweise Reduktion der Verbindungen von Schritt (C)(ii), um eine Verbindung der Formel 1 herzustellen, worin R für $C_8$-$C_{18}$-Alkyl steht, und

F) wahlweise Abspalten der Schutzgruppen von der Verbindung von Schritt C, um eine Verbindung der Formel 1 herzustellen, worin $R^1$ und $R^2$ für Wasserstoff stehen.

**2.** Verfahren zur Herstellung einer A54145 Komponente der Formel

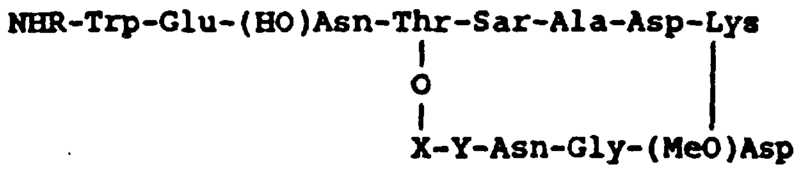

worin R ausgewählt ist aus n-Decanoyl, 8-Methyldecanoyl und 8-Methylnonanoyl,

X für Ile oder Val steht, und
Y für Glu oder 3-MG steht,

mit der Maßgabe, daß:

1) wenn X = Val und Y = 3-MG ist, R für 8-Methyldecanoyl stehen muß und
2) wenn X = Val und Y = Glu ist, R für 8-Methylnonanoyl stehen muß,

gekennzeichnet durch Zugabe einer $C_4$-$C_{18}$-Alkan- oder Alkensäure oder eines $C_4$-$C_{18}$-Alkan- oder Alkenalkohols oder eines Esters oder Salzes hiervon zu einer A54145-bildenden Kultur nach dem Auslösen der Produktionsstufe der Fermentation und Beibehaltung der Zugabe, bis eine gewinnbare Menge der A54145 Komponente gebildet ist.

**3.** Verfahren zur Herstellung eines cyclischen A54145 Peptidderivats der Formel

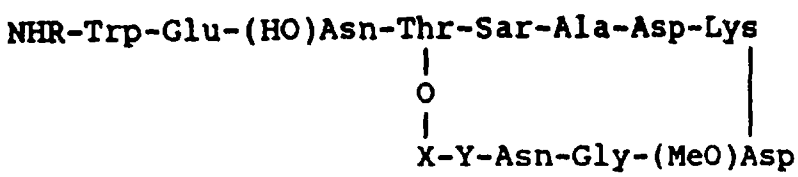

worin

R für $C_6$-$C_{10}$-Alkanoyl steht,
X für Ile oder Val steht, und
Y für Glu oder 3-MG steht,

mit der Maßgabe, daß:

1) wenn X = Ile und Y = Glu oder 3-MG ist, R nicht für 8-Methylnonanoyl, 8-Methyldecanoyl oder n-Decanoyl stehen kann,
2) wenn X = Val und Y = 3-MG ist, R nicht für 8-Methyldecanoyl stehen kann, und
3) wenn X = Val und Y = Glu ist, R nicht für 8-Methylnonanoyl stehen kann,

gekennzeichnet durch Zugabe einer $C_6$-$C_{10}$-Alkan- oder Alkensäure oder eines $C_6$-$C_{10}$-Alkan- oder Alkenalkohols oder eines Esters oder Salzes hiervon zu einer A54145-bildenden Kultur nach dem Auslösen der Produktionsstufe der Fermentation und Beibehaltung der Zugabe, bis eine gewinnbare Menge des A54145 Derivats gebildet ist.

**4.** Verfahren zur Herstellung einer A54145 Verbindung der Formel

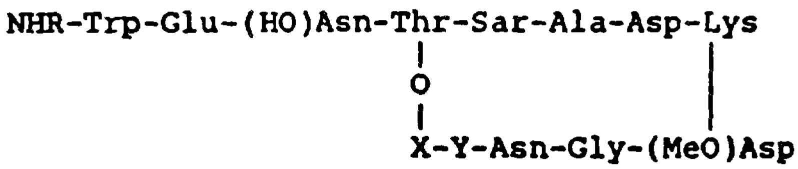

worin R für $C_6$-$C_{10}$-Alkanoyl steht,

X für Ile oder Val steht, und
Y für Glu oder 3-MG steht,

gekennzeichnet durch Zufütterung von Glucose zu einer A54145-bildenden Kultur nach dem Auslösen der Produktionsstufe der Fermentation und Beibehaltung der Zugabe, bis eine gewinnbare Menge der A54145 Verbindung gebildet ist.

**5.** Verfahren zur Herstellung einer A54145 Verbindung der Formel

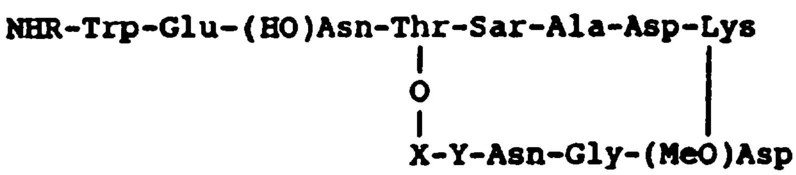

worin R für $C_6$-$C_{10}$-Alkanoyl steht,

X für Ile oder Val steht, und
Y für Glu oder 3-MG steht,

gekennzeichnet durch Zufütterung eines enzymatischen Sojaverdaus zu einer A54145-bildenden Kultur nach dem Auslösen der Produktionsstufe der Fermentation und Beibehaltung der Zugabe, bis eine gewinnbare Menge der A54145 Verbindung gebildet ist.

**6.** Verfahren zur Herstellung einer veterinärmedizinischen Formulierung, gekennzeichnet durch Mischen einer Verbindung der Formel 1 mit einem oder mehreren landwirtschaftlich annehmbaren Trägern hierfür.

**7.** Biologisch gereinigte Kultur von Streptomyces fradiae NRRL 18158, NRRL 18159 oder NRRL 18160 oder einer A54145-bildenden Mutante hiervon.

**Patentansprüche für folgenden Vertragsstaat : GR**

**1.** Verbindung der Formel 4

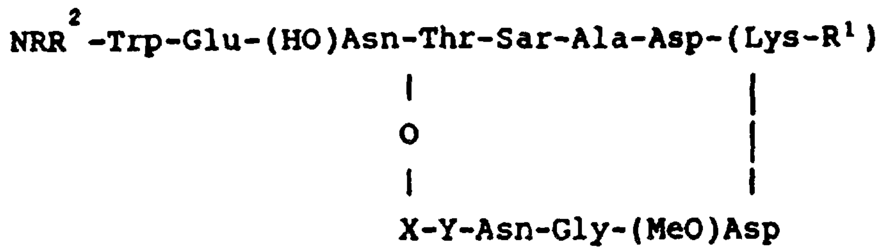

4

worin

R aus der Gruppe ausgewählt wird, die besteht aus Wasserstoff, einer Aminoschutzgruppe, 8-Methylnonanoyl, 8-Methyldecanoyl und n-Decanoyl,
(Lys-$R^1$) für -$NH(CH_2)_4CH(NHR^1)CO$- steht,
$R^1$ für Wasserstoff oder eine Aminoschutzgruppe steht,
$R^2$ für Wasserstoff steht,
X für Ile oder Val steht, und
Y für Glu oder 3-MG steht,

mit der Maßgabe, daß:

1) wenn $R^1$ = H, X = Ile und Y = Glu oder 3-MG ist, dann kann R nicht für 8-Methylnonanoyl, 8-Methyldecanoyl oder n-Decanoyl stehen,
2) wenn $R^1$ = H, X = Val und Y = 3-MG ist, dann kann R nicht für 8-Methyldecanoyl stehen, und
3) wenn $R^1$ m H, X = Val und Y = Glu ist, dann kann R nicht für 8-Methylnonanoyl stehen,

oder ein pharmazeutisch annehmbares Salz hiervon.

**2.** Verfahren zur Herstellung einer Verbindung der Formel 1

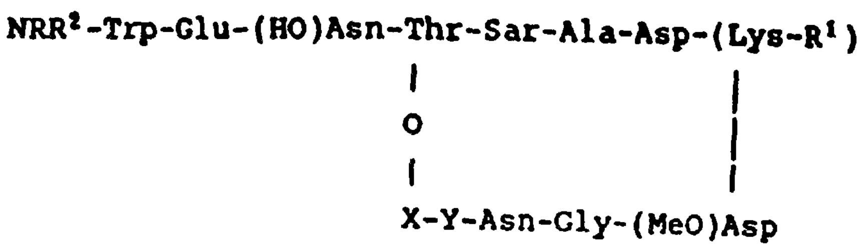

1

worin

R für Wasserstoff, $C_8$-$C_{18}$-Alkyl, wahlweise substituiertes $C_8$-$C_{18}$-Alkanoyl oder $C_8$-$C_{18}$-Alkenoyl steht,
(Lys-$R^1$) für -$NH(CH_2)_4CH(NHR^1)CO$- steht,
$R^1$ und $R^2$ für Wasserstoff oder eine Aminoschutzgruppe stehen, oder
R und $R^2$ zusammengenommen für eine $C_8$-$C_{18}$-Alkylidengruppe stehen,
X für Ile oder Val steht, und
Y für Glu oder 3-MG steht,

oder eines pharmazeutisch annehmbaren Salzes hiervon, das gekennzeichnet ist durch

A) Kultivierung von Streptomyces fradiae NRRL 18158, NRRL 18159 oder NRRL 18160 oder einer A54145-bildenden Mutante hiervon in einem Kulturmedium, das assimilierbare Quellen für Kohlenstoff, Stickstoff und anorganische Salze enthält, unter submersen aeroben Fermentationsbedingungen, wahlweise gefolgt von

einer Abtrennung des Antibiotikums vom Fermentationsmedium und/oder Salzbildung des Antibiotikums falls es nicht in Salzform vorliegt unter Bildung einer Verbindung der Formel 1 worin:

$R^2$ und $R^1$ für Wasserstoff stehen, und
R für 8-Methylnonanoyl, 8-Methyldecanoyl oder n-Decanoyl steht mit der Maßgabe, daß:
wenn X für Val steht und Y für Glu steht, R für 8-Methylnonanoyl stehen muß und wenn X für Val steht und Y für 3-MG steht, R für 8-Methyldecanoyl stehen muß,

B) wahlweise Schützen des Antibiotikums mit einer Aminoschutzgruppe, um eine Verbindung der Formel 1 herzustellen, worin:

$R^2$ und $R^1$ für eine Aminoschutzgruppe stehen, und
R für 8-Methylnonanoyl, 8-Methyldecanoyl oder n-Decanoyl steht, mit der Maßgabe, daß:
wenn X für Val steht und Y für Glu steht, R für 8-Methylnonanoyl stehen muß und wenn X für Val steht und Y für 3-MG steht, R für 8-Methyldecanoyl stehen muß,

C) enzymatische Deacylierung des Produkts von Schritt (A) oder (B), um eine Verbindung der Formel 1 herzustellen, worin:

R für Wasserstoff steht, und
$R^1$ und $R^2$ für Wasserstoff oder eine Aminoschutzgruppe stehen, und/oder Salzbildung der Verbindung falls sie nicht in Salzform vorliegt, und wahlweise

D)

i) Reacylierung des in Schritt C hergestellten Produkts mit der gewünschten Alkanoyl- oder Alkenoylseitenkette, um eine Verbindung der Formel 1 herzustellen, worin:

R für wahlweise substituiertes $C_8$-$C_{18}$-Alkanoyl oder $C_8$-$C_{18}$-Alkenoyl steht, und
$R^1$ und $R^2$ für Wasserstoff oder eine Aminoschutzgruppe stehen, mit der Maßgabe, daß $R^1$ nicht für Wasserstoff stehen kann:

1) wenn X = Ile, Y = Glu oder 3-MG ist und R für 8-Methylnonanoyl, 8-Methyldecanoyl oder n-Decanoyl steht,
2) wenn X = Val, Y = Glu und R = n-Decanoyl ist, und
3) wenn X = Val, Y = 3-MG und R = 8-Methyldecanoyl ist, oder

ii) Kondensierung der primären Aminogruppe von Tryptophan der in Schritt B hergestellten Verbindung mit einem geeigneten Aldehyd oder Keton, um die Verbindung der Formel 1 herzustellen, worin R und $R^2$ zusammengenommen für eine $C_8$-$C_{18}$-Alkylidengruppe stehen, und
iii) wahlweise Reduktion der Verbindungen von Schritt (C)(ii), um eine Verbindung der Formel 1 herzustellen, worin R für $C_8$-$C_{18}$-Alkyl steht, und

F) wahlweise Abspalten der Schutzgruppen von der Verbindung von Schritt C, um eine Verbindung der Formel 1 herzustellen, worin $R^1$ und $R^2$ für Wasserstoff stehen.

**3.** Verfahren zur Herstellung einer A54145 Komponente der Formel

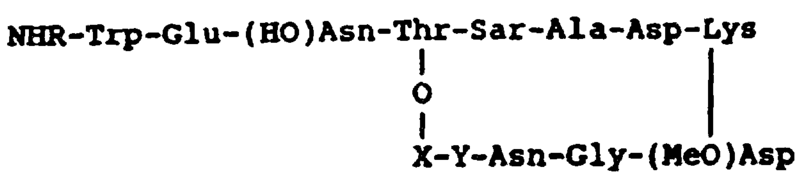

worin R ausgewählt ist aus n-Decanoyl, 8-Methyldecanoyl und 8-Methylnonanoyl,

X für Ile oder Val steht, und
Y für Glu oder 3-MG steht,

mit der Maßgabe, daß:

1) wenn X = Val und Y = 3-MG ist, R für 8-Methyldecanoyl stehen muß und
2) wenn X = Val und Y = Glu ist, R für 8-Methylnonanoyl stehen muß,

gekennzeichnet durch Zugabe einer $C_4$-$C_{18}$-Alkan- oder Alkensäure oder eines $C_4$-$C_{18}$-Alkan- oder Alkenalkohls oder eines Esters oder Salzes hiervon zu einer A54145-bildenden Kultur nach dem Auslösen der Produktionsstufe der Fermentation und Beibehaltung der Zugabe, bis eine gewinnbare Menge der A54145 Komponente gebildet ist.

**4.** Verfahren zur Herstellung eines cyclischen A54145 Peptidderivats der Formel

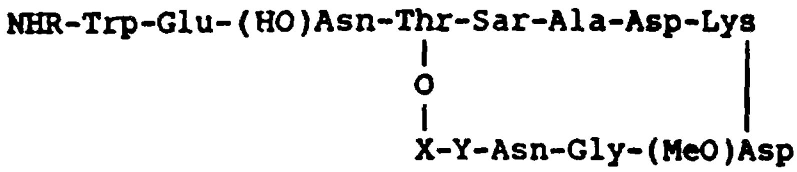

worin

R für $C_6$-$C_{10}$-Alkanoyl steht,
X für Ile oder Val steht, und
Y für Glu oder 3-MG steht,

mit der Maßgabe, daß:

1) wenn X = Ile und Y = Glu oder 3-MG ist, R nicht für 8-Methylnonanoyl, 8-Methyldecanoyl oder n-Decanoyl stehen kann,
2) wenn X = Val und Y = 3-MG ist, R nicht für 8-Methyldecanoyl stehen kann, und
3) wenn X = Val und Y = Glu ist, R nicht für 8-Methylnonanoyl stehen kann,

gekennzeichnet durch Zugabe einer $C_6$-$C_{10}$-Alkan- oder Alkensäure oder eines $C_6$-$C_{10}$-Alkan- oder Alkenalkohols oder eines Esters oder Salzes hiervon zu einer A54145-bildenden Kultur nach dem Auslösen der Produktionsstufe der Fermentation und Beibehaltung der Zugabe, bis eine gewinnbare Menge des A54145 Derivats gebildet ist.

**5.** Verfahren zur Herstellung einer A54145 Verbindung der Formel

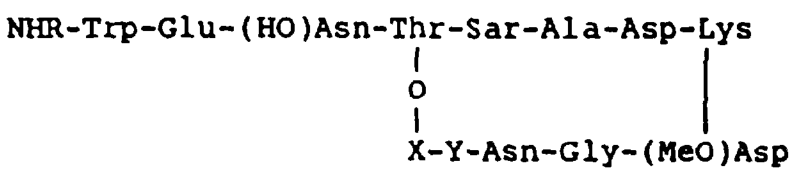

worin R für $C_6$-$C_{10}$-Alkanoyl steht,

X für Ile oder Val steht, und
Y für Glu oder 3-MG steht,

gekennzeichnet durch Zufütterung von Glucose zu einer A54145-bildenden Kultur nach dem Auslösen der Produktionsstufe der Fermentation und Beibehaltung der Zugabe, bis eine gewinnbare Menge der A54145 Verbindung gebildet ist.

**6.** Verfahren zur Herstellung einer A54145 Verbindung der Formel

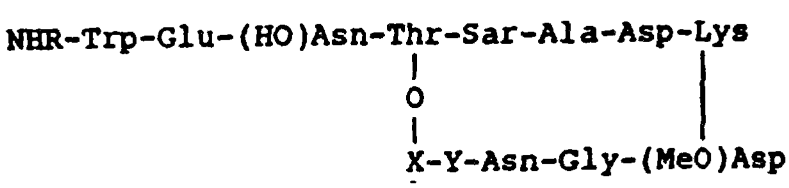

worin R für $C_6$-$C_{10}$-Alkanoyl steht,

X für Ile oder Val steht, und
Y für Glu oder 3-MG steht,

gekennzeichnet durch Zufütterung eines enzymatischen Sojaverdaus zu einer A54145-bildenden Kultur nach dem Auslösen der Produktionsstufe der Fermentation und Beibehaltung der Zugabe, bis eine gewinnbare Menge der A54145 Verbindung gebildet ist.

**7.** Verfahren zur Herstellung einer veterinärmedizinischen Formulierung, gekennzeichnet durch Mischen einer Verbindung der Formel 1 mit einem oder mehreren landwirtschaftlich annehmbaren Trägern hierfür.

**8.** Biologisch gereinigte Kultur von Streptomyces fradiae NRRL 18158, NRRL 18159 oder NRRL 18160 oder einer A54145-bildenden Mutante hiervon.

**Revendications**

**Revendications pour les Etats contractants suivants : AT, BE, CH, DE, FR, GB, IT, LI, NL, SE**

**1.** Composé répondant à la formule 1 :

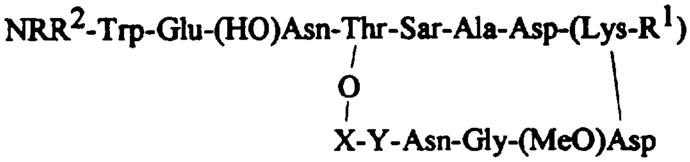

**1**

dans laquelle :

R représente un atome d'hydrogène, un groupe alkyle en $C_8$-$C_{18}$, un groupe alcanoyle en $C_8$-$C_{18}$ ou alcénoyle en $C_8$-$C_{18}$ facultativement substitué;
(Lys-$R^1$) représente un groupe $-NH(CH_2)_4CH(NHR^1)CO-$;
$R^1$ et $R^2$ représentent un atome d'hydrogène ou un groupe amino-protecteur; ou
R et $R^2$, pris ensemble, représentent un groupe alkylidène en $C_8$-$C_{18}$;
X représente Ile ou Val; et
Y représente Glu ou 3-MG;

ou un sel pharmaceutiquement acceptable de celui-ci.

**2.** Antibiotique lipopeptidique qui peut être produit par une fermentation aérobie immergée de Streptomyces fradiae NRRL 18158, NRRL 18159 ou NRRL 18160 dans un milieu de culture contenant des sources assimilables de carbone, d'azote et de sels inorganiques.

**3.** Composé répondant à la formule 4 :

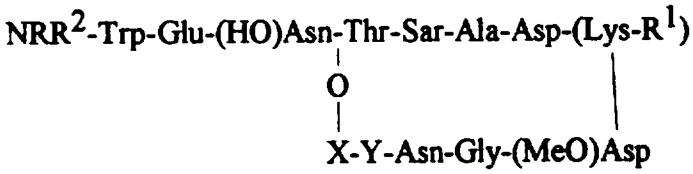

**4**

dans laquelle :

R est choisi parmi le groupe constitué d'un atome d'hydrogène, d'un groupe amino-protecteur, du groupe 8-méthylnonanoyle, du groupe 8-méthyldécanoyle et du groupe n-décanoyle;
(Lys-$R^1$) représente un groupe $-NH(CH_2)_4CH(NHR^1)CO-$;
$R^1$ représente un atome d'hydrogène ou un groupe amino-protecteur;
$R^2$ représente un atome d'hydrogène;
X représente Ile ou Val; et
Y représente Glu ou 3-MG;

pour autant que :

1) lorsque $R^1$ = H, X = Ile et Y = Glu ou 3-MG, R ne peut pas être un groupe 8-méthylnonanoyle, un groupe 8-méthyldécanoyle ou un groupe n-décanoyle;
2) lorsque $R^1$ = H, X = Val et Y = 3-MG, R ne peut pas être un groupe 8-méthyldécanoyle; et
3) lorsque $R^1$ = H, X = Val et Y = Glu, R ne peut pas être un groupe 8-méthylnonanoyle;

ou un sel pharmaceutiquement acceptable de celui-ci.

**4.** Composé répondant à la formule 5 :

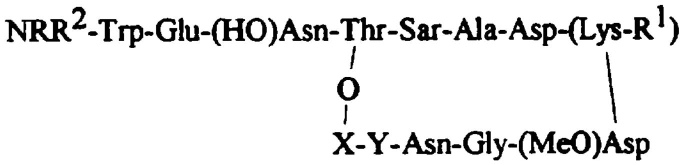

5

dans laquelle :

R représente un groupe alkyle en $C_8$-$C_{18}$, un groupe alcanoyle en $C_8$-$C_{18}$ ou alcénoyle en $C_1$-$C_{18}$ facultativement substitué;
(Lys-$R^1$) représente un groupe $-NH(CH_2)_4CH(NHR^1)CO-$;
$R^1$ représente un atome d'hydrogène ou un groupe amine-protecteur, et
$R^2$ représente un atome d'hydrogène; ou
R et $R^2$, pris ensemble, représentent un groupe en $C_8$-$C_{18}$;
X représente Ile ou Val; et
Y représente Glu ou 3-MG;

pour autant que : 1) lorsque $R^1$ = H, X = Ile et Y = Glu ou 3-MG, R ne peut pas être un groupe 8-méthylnonanoyle, un groupe 8-méthyldécanoyle ou un groupe n-décanoyle; 2) lorsque $R^1$ = H, X = Val et Y = 3-MG, R ne peut pas être un groupe 8-méthyldécanoyle; et 3) lorsque $R^1$ = H, X = Val et Y = Glu, R ne peut pas être un groupe 8-méthylnonanoyle;
ou un sel pharmaceutiquement acceptable de celui-ci.

**5.** Antibiotique A54145 selon la revendication 2, ou un sel pharmaceutiquement acceptable de celui-ci.

**6.** Composant A d'antibiotique A54145 selon la revendication 1, dans lequel dans la formule 1 $R^2$ représente H, X représente Ile, Y représente Glu et R représente un groupe 8-méthylnonanoyle, ou un sel pharmaceutiquement acceptable de celui-ci.

**7.** Composant $A_1$ d'antibiotique A54145 selon la revendication 1, dans lequel dans la formule 1 $R^2$ représente H, X représente Ile, Y représente Glu et R représente un groupe n-décanoyle, ou un sel pharmaceutiquement acceptable de celui-ci.

**8.** Composant B d'antibiotique A54145 selon la revendication 1, dans lequel dans la formule 1 $R^2$ représente H, X représente Ile, Y représente 3-MG et R représente un groupe n-décanoyle, ou un sel pharmaceutiquement acceptable de celui-ci.

**9.** Composant $B_1$ d'antibiotique A54145 selon la revendication 1, dans lequel dans la formule 1 $R^2$ représente H, X

représente Ile, Y représente 3-MG et R représente un groupe 8-méthylnonanoyle, ou un sel pharmaceutiquement acceptable de celui-ci.

**10.** Composant C d'antibiotique A54145 selon la revendication 1, dans lequel dans la formule 1 $R^2$ représente H, X représente Val, Y représente 3-MG et R représente un groupe 8-méthyldécanoyle, ou un sel pharmaceutiquement acceptable de celui-ci.

**11.** Composant D d'antibiotique A54145 selon la revendication 1, dans lequel dans la formule 1 $R^2$ représente H, X représente Ile, Y représente Glu et R représente un groupe 8-méthyldécanoyle, ou un sel pharmaceutiquement acceptable de celui-ci.

**12.** Composant E d'antibiotique A54145 selon la revendication 1, dans lequel dans la formule 1 $R^2$ représente H, X représente Ile, Y représente 3-MG et R représente un groupe 8-méthyldécanoyle, ou un sel pharmaceutiquement acceptable de celui-ci.

**13.** Composant F d'antibiotique A54145 selon la revendication 1, dans lequel dans la formule 1 $R^2$ représente H, X représente Val, Y représente Glu et R représente un groupe 8-méthylnonanoyle, ou un sel pharmaceutiquement acceptable de celui-ci.

**14.** Composition pharmaceutique comprenant comme ingrédient actif un composé selon l'une quelconque des revendications 1 à 2 ou 4 à 13, associé à un ou plusieurs supports pharmaceutiquement acceptables pour celui-ci.

**15.** Composé selon l'une quelconque des revendications 1 à 2 ou 4 à 13 pour une utilisation dans une thérapeutique d'infections bactériennes.

**16.** Prémélange d'aliment pour animaux comprenant comme ingrédient actif un composé selon l'une quelconque des revendications 1 à 2 ou 4 à 13, associé à un ou plusieurs supports agronomiquement acceptables pour celui-ci.

**17.** Procédé pour préparer un composé répondant à la formule 1 selon l'une quelconque des revendications 1 et 3 à 13, qui comprend :

A) la culture de Streptomyces fradiae NRRL 18158, NRRL 18159 ou NRRL 18160, ou d'un mutant produisant du A54145 de ceux-ci, dans un milieu de culture contenant des sources assimilables de carbone, d'azote et de sels inorganiques dans des conditions de fermentation aérobie immergée, facultativement suivies par la séparation de l'antibiotique à partir du milieu de fermentation et/ou la salification de l'antibiotique s'il ne se trouve pas sous forme saline de façon à produire un composé répondant à la formule 1 dans laquelle :

$R^2$ et $R^1$ représentent un atome d'hydrogène; et
R représente un groupe méthylnonanoyle, un groupe 8-méthyldécanoyle ou un groupe n-décanoyle;

pour autant que : lorsque X représente Val et Y représente Glu, R doit être un groupe 8-méthylnonanoyle; et lorsque X représente Val et Y représente 3-MG, R doit être un groupe 8-méthyldécanoyle;
B) la protection facultative de l'antibiotique avec un groupe amino-protecteur de façon à préparer un composé répondant à la formule 1 dans laquelle :

$R^2$ et $R^1$ représentent un groupe amino-protecteur; et
R représente un groupe 8-méthylnonanoyle, un groupe 8-méthyldécanoyle ou un groupe n-décanoyle;

pour autant que : lorsque X représente Val et Y représente Glu, R doit être un groupe 8-méthylnonanoyle; et lorsque X représente Val et Y représente 3-MG, R doit être un groupe 8-méthyldécanoyle;
C) la désacylation enzymatique du produit de l'étape (A) ou (B) de façon à préparer un composé répondant à la formule 1 dans laquelle :

R représente un atome d'hydrogène; et
$R^1$ et $R^2$ représentent un atome d'hydrogène ou un groupe amino-protecteur, et/ou la salification du composé s'il ne se trouve pas sous forme saline; et facultativement

D)

i) la réacylation du produit préparé à l'étape C avec la chaîne latérale alcanoyle ou alcénoyle souhaitée de façon à préparer un composé répondant à la formule 1 dans laquelle :

R représente un groupe alcanoyle en $C_8$-$C_{18}$ ou alcénoyle en $C_8$-$C_{18}$ facultativement substitué; et $R^1$ et $R^2$ représentent un atome d'hydrogène ou un groupe amino-protecteur, pour autant que R' ne puisse pas être un atome d'hydrogène:

1) lorsque X = Ile, Y = Glu ou 3-MG et R représente un groupe 8-méthylnonanoyle, un groupe 8-méthyldécanoyle ou un groupe n-décanoyle;
2) lorsque X = Val, Y = Glu et R = un groupe n-décanoyle; et
3) lorsque X = Val, Y = 3-MG et R = un groupe 8-méthyldécanoyle; ou

ii) la condensation du groupe amino primaire du tryptophane du composé préparé à l'étape B avec un aldéhyde approprié ou une cétone appropriée de façon à préparer le composé répondant à la formule 1 dans laquelle R et $R^2$, pris ensemble, représentent un groupe alkylidène en $C_8$-$C_{18}$; et
iii) la réduction facultative des composés de l'étape (C)(ii) de façon à préparer un composé répondant à la formule 1 dans laquelle R représente un groupe alkyle en $C_8$-$C_{18}$;
et

F) le déblocage facultatif du composé de l'étape C de façon à préparer le composé répondant à la formule 1 dans laquelle $R^1$ et $R^2$ représentent un atome d'hydrogène.

**18.** Procédé pour préparer un composant A54145 répondant à la formule :

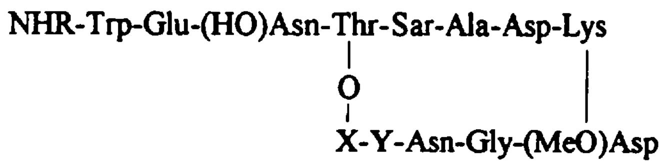

dans laquelle R est choisi parmi les groupes n-décanoyle, 8-méthyldécanoyle et 8-méthylnonanoyle;

X représente Ile ou Val; et
Y représente Glu ou 3-MG;

pour autant que : 1) lorsque X = Val et Y = 3-MG, R doit représenter un groupe 8-méthyldécanoyle; et 2) lorsque X = Val et Y = Glu, R doit représenter un groupe 8-méthylnonanoyle;
qui comprend l'addition d'un acide ou d'un alcool alcanoïque ou alcénoïque en $C_4$-$C_{18}$, ou d'un ester ou d'un sel de celui-ci, à une culture produisant du A54145, après l'initiation de l'étape de production de la fermentation, en continuant jusqu'à ce qu'une quantité récupérable de composant A54145 a été produite.

**19.** Procédé pour préparer un dérivé peptidique cyclique A54145 répondant à la formule :

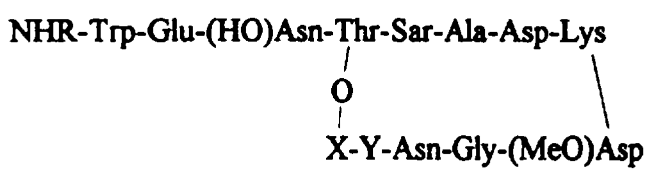

dans laquelle :

R représente un groupe alcanoyle en $C_6$-$C_{10}$;
X représente Ile ou Val; et
Y représente Glu ou 3-MG;

pour autant que :

1) lorsque X = Ile et Y = Glu ou 3-MG, R ne peut pas être un groupe 8-méthylnonanoyle, un groupe 8-méthyldécanoyle ou un groupe n-décanoyle;

2) lorsque X = Val et Y = 3-MG, R ne peut pas être un groupe 8-méthyldécanoyle; et
3) lorsque X = Val et Y = Glu, R ne peut pas être un groupe 8-méthylnonanoyle;

qui comprend l'addition d'un acide ou d'un alcool alcanoïque ou alcénoïque en $C_6$-$C_{10}$, ou d'un ester ou d'un sel de celui-ci, à une culture produisant du A54145, après l'initiation de l'étape de production de la fermentation, en continuant jusqu'à ce qu'une quantité récupérable du dérivé A54145 a été produite.

**20.** Procédé pour préparer un composé A54145 répondant à la formule :

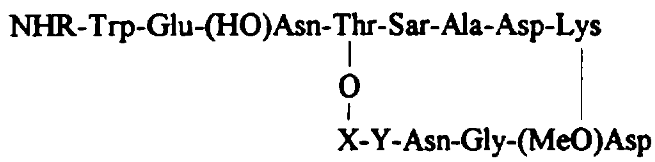

dans laquelle :

R représente un groupe alcanoyle en $C_6$-$C_{10}$;
X représente Ile ou Val; et
Y représente Glu ou 3-MG;

qui comprend l'alimentation de glucose à une culture produisant du A54145, après l'initiation de l'étape de production de la fermentation, en continuant jusqu'à ce qu'une quantité récupérable de composé A54145 a été produite.

**21.** Procédé pour préparer un composé A54145 répondant à la formule :

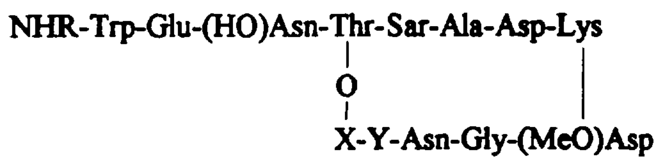

dans laquelle :

R représente un groupe alcanoyle en $C_6$-$C_{10}$;
X représente Ile ou Val; et
Y représente Glu ou 3-MG;

qui comprend l'alimentation de soja digéré par voie enzymatique à une culture produisant du A54145, après l'initiation de l'étape de production de la fermentation, en continuant jusqu'à ce qu'une quantité récupérable de composé A54145 a été produite.

**22.** Culture purifiée par voie biologique de Streptomyces fradiae NRRL 18158, NRRL 18159 ou NRRL 18160, ou d'un mutant produisant du A54145 de celle-ci.

**Revendications pour l'Etat contractant suivant : ES**

**1.** Procédé pour préparer un composé répondant à la formule 1 :

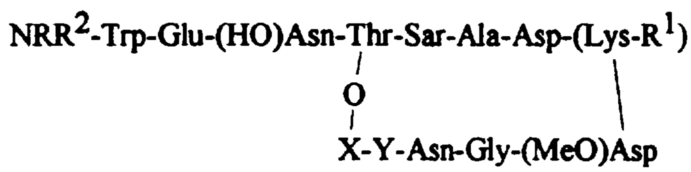

1

dans laquelle :

R représente un atome d'hydrogène, un groupe alkyle en $C_8$-$C_{18}$, un groupe alcanoyle en $C_8$-$C_{18}$ ou alcénoyle

en $C_8$-$C_{18}$ facultativement substitué;
(Lys-$R^1$) représente un groupe $-NH(CH_2)_4CH(NHR^1)CO-$;
$R^1$ et $R^2$ représentent un atome d'hydrogène ou un groupe amino-protecteur; ou
R et $R^2$, pris ensemble, représentent un groupe alkylidène en $C_8$-$C_{18}$;
X représente Ile ou Val; et
Y représente Glu ou 3-MG;

ou un sel pharmaceutiquement acceptable de celui-ci;
qui comprend :

A) la culture de <u>Streptomyces</u> <u>fradiae</u> NRRL 18158, NRRL 18159 ou NRRL 18160, ou d'un mutant produisant du A54145 de ceux-ci, dans un milieu de culture contenant des sources assimilables de carbone, d'azote et de sels inorganiques dans des conditions de fermentation aérobie immergée, facultativement suivies par la séparation de l'antibiotique à partir du milieu de fermentation et/ou la salification de l'antibiotique s'il ne se trouve pas sous forme saline de façon à produire un composé répondant à la formule <u>1</u> dans laquelle :

$R^2$ et $R^1$ représentent un atome d'hydrogène; et
R représente un groupe méthylnonanoyle, un groupe 8-méthyldécanoyle ou un groupe n-décanoyle;

pour autant que : lorsque X représente Val et Y représente Glu, R doit être un groupe 8-méthylnonanoyle; et lorsque X représente Val et Y représente 3-MG, R doit être un groupe 8-méthyldécanoyle;
B) la protection facultative de l'antibiotique avec un groupe amino-protecteur de façon à préparer un composé répondant à la formule <u>1</u> dans laquelle :

$R^2$ et $R^1$ représentent un groupe amino-protecteur; et
R représente un groupe 8-méthylnonanoyle, un groupe 8-méthyldécanoyle ou un groupe n-décanoyle;

pour autant que : lorsque X représente Val et Y représente Glu, R doit être un groupe 8-méthylnonanoyle; et lorsque X représente Val et Y représente 3-MG, R doit être un groupe 8-méthyldécanoyle;
C) la désacylation enzymatique du produit de l'étape (A) ou (B) de façon à préparer un composé répondant à la formule <u>1</u> dans laquelle :

R représente un atome d'hydrogène; et
$R^1$ et $R^2$ représentent un atome d'hydrogène ou un groupe amino-protecteur; et/ou la salification du composé s'il ne se trouve pas sous forme saline; et facultativement

D)

i) la réacylation du produit préparé à l'étape C avec la chaîne latérale alcanoyle ou alcénoyle souhaitée de façon à préparer un composé répondant à la formule <u>1</u> dans laquelle :

R représente un groupe alcanoyle en $C_8$-$C_{18}$ ou alcénoyle en $C_8$-$C_{18}$ facultativement substitué; et $R^1$ et $R^2$ représentent un atome d'hydrogène ou un groupe amino-protecteur; pour autant que $R^1$ ne puisse pas être un atome d'hydrogène :

1) lorsque X = Ile, Y = Glu ou 3-MG et R représente un groupe 8-méthylnonanoyle, un groupe 8-méthyldécanoyle ou un groupe n-décanoyle;
2) lorsque X = Val, Y = Glu et R = un groupe n-décanoyle; et
3) lorsque X = Val, Y = 3-MG et R = un groupe 8-méthyldécanoyle; ou

ii) la condensation du groupe amino primaire du tryptophane du composé préparé à l'étape B avec un aldéhyde approprié ou une cétone appropriée de façon à préparer le composé répondant à la formule <u>1</u> dans laquelle R et $R^2$, pris ensemble, représentent un groupe alkylidène en $C_8$-$C_{18}$; et
iii) la réduction facultative des composés de l'étape (C)(ii) de façon à préparer un composé répondant à la formule <u>1</u> dans laquelle R représente un groupe alkyle en $C_8$-$C_{18}$;
et

F) le déblocage facultatif du composé de l'étape C de façon à préparer le composé répondant à la formule <u>1</u>

dans laquelle $R^1$ et $R^2$ représentent un atome d'hydrogène.

**2.** Procédé pour préparer un composant A54145 répondant à la formule :

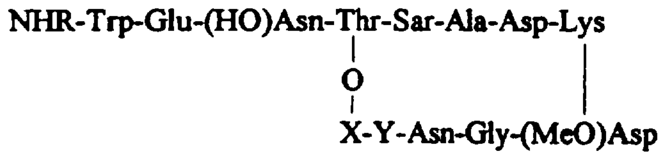

dans laquelle R est choisi parmi les groupes n-décanoyle, 8-méthyldécanoyle et 8-méthylnonanoyle;

X représente Ile ou Val; et
Y représente Glu ou 3-MG;

pour autant que : 1) lorsque X = Val et Y = 3-MG, R doit représenter un groupe 8-méthyldécanoyle; et 2) lorsque X = Val et Y = Glu, R doit représenter un groupe 8-méthylnonanoyle;
qui comprend l'addition d'un acide ou d'un alcool alcanoïque ou alcénoïque en $C_4$-$C_{18}$, ou d'un ester ou d'un sel de celui-ci, à une culture produisant du A54145, après l'initiation de l'étape de production de la fermentation, en continuant jusqu'à ce qu'une quantité récupérable de composant A54145 a été produite.

**3.** Procédé pour préparer un dérivé peptidique cyclique A54145 répondant à la formule :

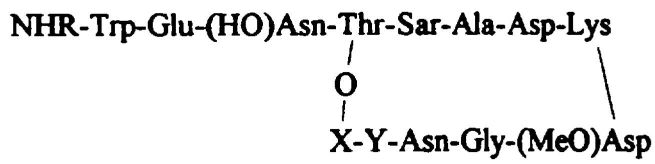

dans laquelle :

R représente un groupe alcanoyle en $C_6$-$C_{10}$;
X représente Ile ou Val; et
Y représente Glu ou 3-MG;

pour autant que :

1) lorsque X = Ile et Y = Glu ou 3-MG, R ne peut pas être un groupe 8-méthylnonanoyle, un groupe 8-méthyldécanoyle ou un groupe n-décanoyle;
2) lorsque X = Val et Y = 3-MG, R ne peut pas être un groupe 8-méthyldécanoyle; et
3) lorsque X = Val et Y = Glu, R ne peut pas être un groupe 8-méthylnonanoyle; qui comprend l'addition d'un acide ou d'un alcool alcanoïque ou alcénoïque en $C_6$-$C_{10}$, ou d'un ester ou d'un sel de celui-ci, à une culture produisant du A54145, après l'initiation de l'étape de production de la fermentation, en continuant jusqu'à ce qu'une quantité récupérable du dérivé A54145 a été produite.

**4.** Procédé pour préparer un composé A54145 répondant à la formule :

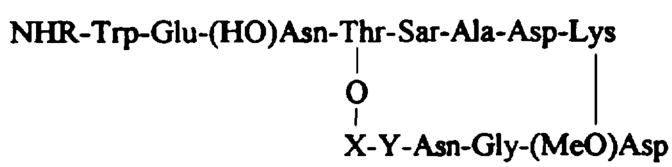

dans laquelle :

R représente un groupe alcanoyle en $C_6$-$C_{10}$;
X représente Ile ou Val; et
Y représente Glu ou 3-MG;

qui comprend l'alimentation de glucose à une culture produisant du A54145, après l'initiation de l'étape de production

de la fermentation, en continuant jusqu'à ce qu'une quantité récupérable de composé A54145 a été produite.

5. Procédé pour préparer un composé A54145 répondant à la formule :

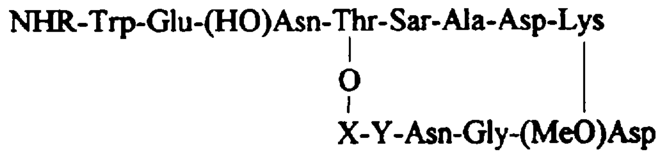

dans laquelle :

R représente un groupe alcanoyle en $C_6$-$C_{10}$;
X représente Ile ou Val; et
Y représente Glu ou 3-MG;

qui comprend l'alimentation de soja digéré par voie enzymatique à une culture produisant du A54145, après l'initiation de l'étape de production de la fermentation, en continuant jusqu'à ce qu'une quantité récupérable de composé A54145 a été produite.

6. Procédé pour préparer une formulation vétérinaire comprenant le mélange d'un composé selon l'une quelconque des revendications 1 à 2 ou 4 à 14 avec un ou plusieurs supports agronomiquement acceptables pour oelui-ci.

7. Culture purifiée par voie biologique de Streptomyces fradiae NRRL 18158, NRRL 18159 ou NRRL 18160, ou d'un mutant produisant du A54145 de celle-ci.

**Revendications pour l'Etat contractant suivant : ES**

1. Composé répondant à la formule 4 :

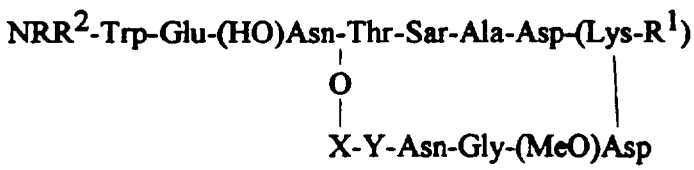

4

dans laquelle :

R est choisi parmi le groupe constitué d'un atome d'hydrogène, d'un groupe amino-protecteur, du groupe 8-méthylnonanoyle, du groupe 8-méthyldécanoyle et du groupe n-décanoyle;
(Lys-$R^1$) représente un groupe $-NH(CH_2)_4CH(NHR^1)CO-$;
$R^1$ représente un atome d'hydrogène ou un groupe amino-protecteur;
$R^2$ représente un atome d'hydrogène;
X représente Ile ou Val; et
Y représente Glu ou 3-MG;

pour autant que :

1) lorsque $R^1$ = H, X = Ile et Y = Glu ou 3-MG, R ne peut pas être un groupe 8-méthylnonanoyle, un groupe 8-méthyldécanoyle ou un groupe n-décanoyle;
2) lorsque $R^1$ = H, X = Val et Y = 3-MG, R ne peut pas être un groupe 8-méthyldécanoyle; et
3) lorsque $R^1$ = H, X = Val et Y = Glu, R ne peut pas être un groupe 8-méthylnonanoyle

ou un sel pharmaceutiquement acceptable de celui-ci.

2. Procédé pour préparer un composé répondant à la formule 1 :

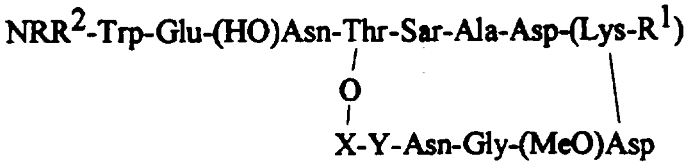

**1**

dans laquelle :

R représente un atome d'hydrogène, un groupe alkyle en $C_8$-$C_{18}$, un groupe alcanoyle en $C_8$-$C_{18}$ ou alcénoyle en $C_8$-$C_{18}$ facultativement substitué;
(Lys-$R^1$) représente un groupe $-NH(CH_2)_4CH(NHR^1)CO-$;
$R^1$ et $R^2$ représentent un atome d'hydrogène ou un groupe amino-protecteur; ou
R et $R^2$, pris ensemble, représentent un groupe alkylidène en $C_8$-$C_{18}$;
X représente Ile ou Val; et
Y représente Glu ou 3-MG;

ou un sel pharmaceutiquement acceptable de celui-ci;
qui comprend :

A) la culture de Streptomyces fradiae NRRL 18158, NRRL 18159 ou NRRL 18160, ou d'un mutant produisant du A54145 de ceux-ci, dans un milieu de culture contenant des sources assimilables de carbone, d'azote et de sels inorganiques dans des conditions de fermentation aérobie immergée, facultativement suivies par la séparation de l'antibiotique à partir du milieu de fermentation et/ou la salification de l'antibiotique s'il ne se trouve pas sous forme saline de façon à produire un composé répondant à la formule 1 dans laquelle :

$R^2$ et $R^1$ représentent un atome d'hydrogène; et
R représente un groupe méthylnonanoyle, un groupe 8-méthyldécanoyle ou un groupe n-décanoyle;

pour autant que : lorsque X représente Val et Y représente Glu, R doit être un groupe 8-méthylnonanoyle; et lorsque X représente Val et Y représente 3-MG, R doit être un groupe 8-méthyldécanoyle;
B) la protection facultative de l'antibiotique avec un groupe amino-protecteur de façon à préparer un composé répondant à la formule 1 dans laquelle :

$R^2$ et $R^1$ représentent un groupe amino-protecteur; et
R représente un groupe 8-méthylnonanoyle, un groupe 8-méthyldécanoyle ou un groupe n-décanoyle;

pour autant que : lorsque X représente Val et Y représente Glu, R doit être un groupe 8-méthylnonanoyle; et lorsque X représente Val et Y représente 3-MG, R doit être un groupe 8-méthyldécanoyle;
C) la désacylation enzymatique du produit de l'étape (A) ou (B) de façon à préparer un composé répondant à la formule 1 dans laquelle :

R représente un atome d'hydrogène; et
$R^1$ et $R^2$ représentent un atome d'hydrogène ou un groupe amino-protecteur; et/ou la salification du composé s'il ne se trouve pas sous forme saline; et facultativement

D)

i) la réacylation du produit préparé à l'étape C avec la chaîne latérale alcanoyle ou alcénoyle souhaitée de façon à préparer un composé répondant à la formule 1 dans laquelle :

R représente un groupe alcanoyle en $C_8$-$C_{18}$ ou alcénoyle en $C_8$-$C_{18}$ facultativement substitué; et $R^1$ et $R^2$ représentent un atome d'hydrogène ou un groupe amino-protecteur; pour autant que $R^1$ ne puisse pas être un atome d'hydrogène :

1) lorsque X = Ile, Y = Glu ou 3-MG et R représente un groupe 8-méthylnonanoyle, un groupe 8-méthyldécanoyle ou un groupe n-décanoyle;

2) lorsque X = Val, Y = Glu et R = un groupe n-décanoyle; et
3) lorsque X = Val, Y = 3-MG et R = un groupe 8-méthyldécanoyle; ou

ii) la condensation du groupe amino primaire du tryptophane du composé préparé à l'étape B avec un aldéhyde approprié ou une cétone appropriée de façon à préparer le composé répondant à la formule 1 dans laquelle R et $R^2$, pris ensemble, représentent un groupe alkylidène en $C_8$-$C_{18}$; et
iii) la réduction facultative des composés de l'étape (C)(ii) de façon à préparer un composé répondant à la formule 1 dans laquelle R représente un groupe alkyle en $C_8$-$C_{18}$;
et

F) le déblocage facultatif du composé de l'étape C de façon à préparer le composé répondant à la formule 1 dans laquelle $R^1$ et $R^2$ représentent un atome d'hydrogène.

**3.** Procédé pour préparer un composant A54145 répondant à la formule :

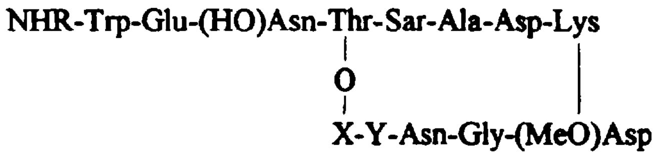

dans laquelle R est choisi parmi les groupes n-décanoyle, 8-méthyldécanoyle et 8-méthylnonanoyle;

X représente Ile ou Val; et
Y représente Glu ou 3-MG;

pour autant que : 1) lorsque X = Val et Y = 3-MG, R doit représenter un groupe 8-méthyldécanoyle; et 2) lorsque X = Val et Y = Glu, R doit représenter un groupe 8-méthylnonanoyle;
qui comprend l'addition d'un acide ou d'un alcool alcanoïque ou alcénoïque en $C_4$-$C_{18}$, ou d'un ester ou d'un sel de celui-ci, à une culture produisant du A54145, après l'initiation de l'étape de production de la fermentation, en continuant jusqu'à ce qu'une quantité récupérable de composant A54145 a été produite.

**4.** Procédé pour préparer un dérivé peptidique cyclique A54145 répondant à la formule :

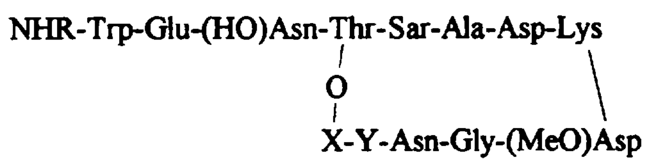

dans laquelle :

R représente un groupe alcanoyle en $C_6$-$C_{10}$;
X représente Ile ou Val; et
Y représente Glu ou 3-MG;

pour autant que :

1) lorsque X = Ile et Y = Glu ou 3-MG, R ne peut pas être un groupe 8-méthylnonanoyle, un groupe 8-méthyldécanoyle ou un groupe n-décanoyle;
2) lorsque X = Val et Y = 3-MG, R ne peut pas être un groupe 8-méthyldécanoyle; et
3) lorsque X = Val et Y = Glu, R ne peut pas être un groupe 8-méthylnonanoyle;

qui comprend l'addition d'un acide ou d'un alcool alcanoïque ou alcénoïque en $C_6$-$C_{10}$, ou d'un ester ou d'un sel de celui-ci, à une culture produisant du A54145, après l'initiation de l'étape de production de la fermentation, en continuant jusqu'à ce qu'une quantité récupérable du dérivé A54145 a été produite.

**5.** Procédé pour préparer un composé A54145 répondant à la formule :

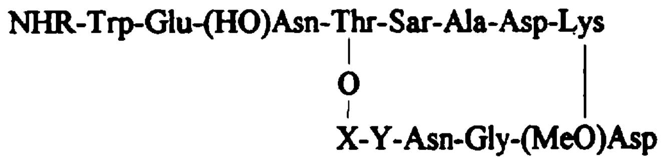

dans laquelle :

R représente un groupe alcanoyle en $C_6$-$C_{10}$;
X représente Ile ou Val; et
Y représente Glu ou 3-MG;

qui comprend l'alimentation de glucose à une culture produisant du A54145, après l'initiation de l'étape de production de la fermentation, en continuant jusqu'à ce qu'une quantité récupérable de composé A54145 a été produite.

**6.** Procédé pour préparer un composé A54145 répondant à la formule :

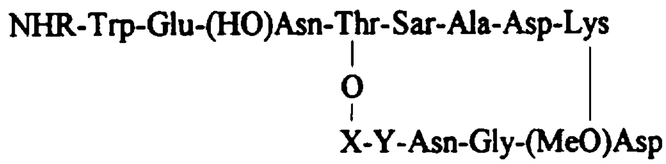

dans laquelle :

R représente un groupe alcanoyle en $C_6$-$C_{10}$;
X représente Ile ou Val; et
Y représente Glu ou 3-MG;

qui comprend l'alimentation de soja digéré par voie enzymatique à une culture produisant du A54145, après l'initiation de l'étape de production de la fermentation, en continuant jusqu'à ce qu'une quantité récupérable de composé A54145 a été produite.

**7.** Procédé pour préparer une formulation vétérinaire comprenant le mélange d'un composé répondant à la formule 1 avec un ou plusieurs supports agronomiquement acceptables pour celui-ci.

**8.** Culture purifiée par voie biologique de <u>Streptomyces</u> <u>fradiae</u> NRRL 18158, NRRL 18159 ou NRRL 18160, ou d'un mutant produisant du A54145 de celles-ci.

FIG. 1

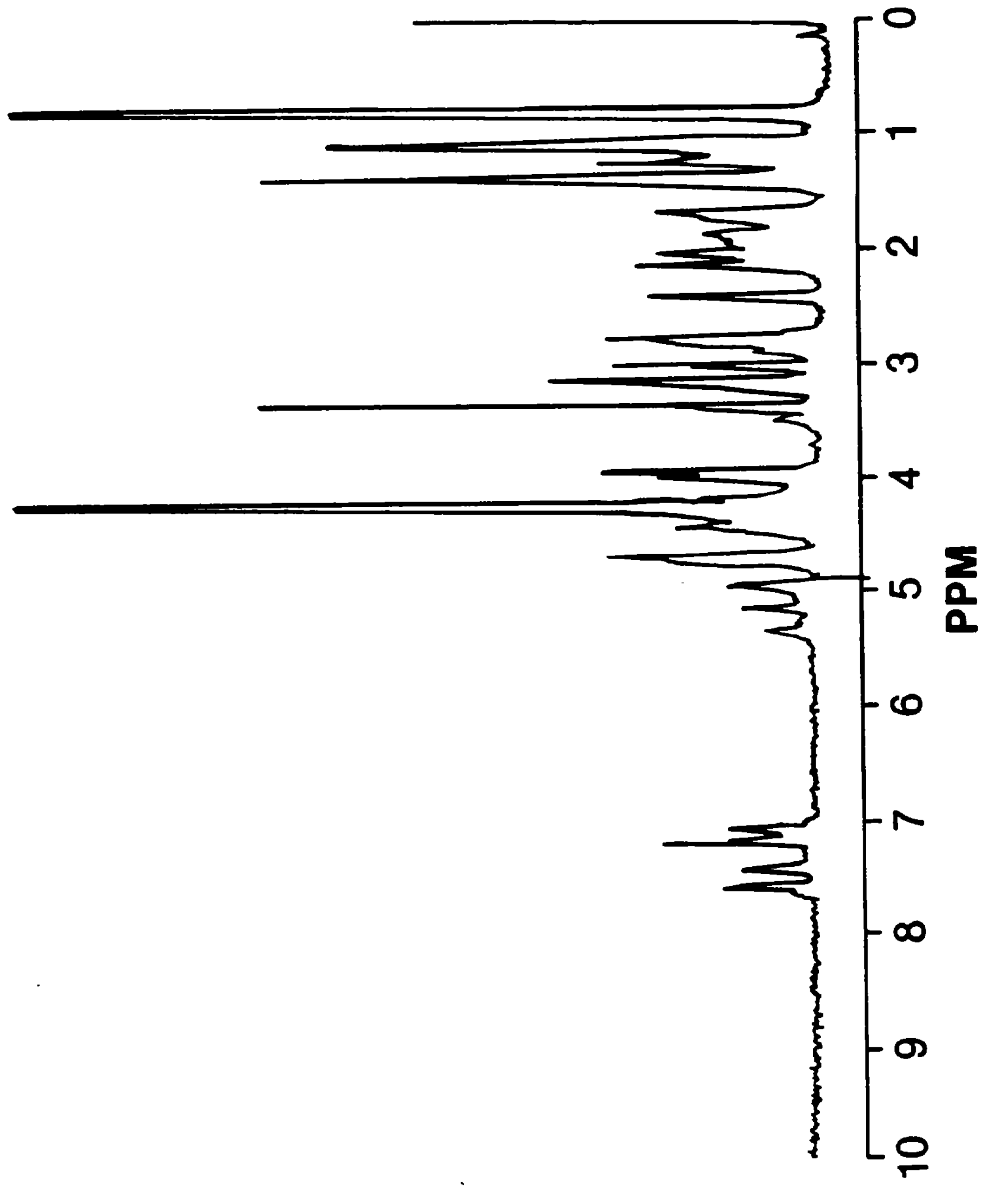

FIG.2

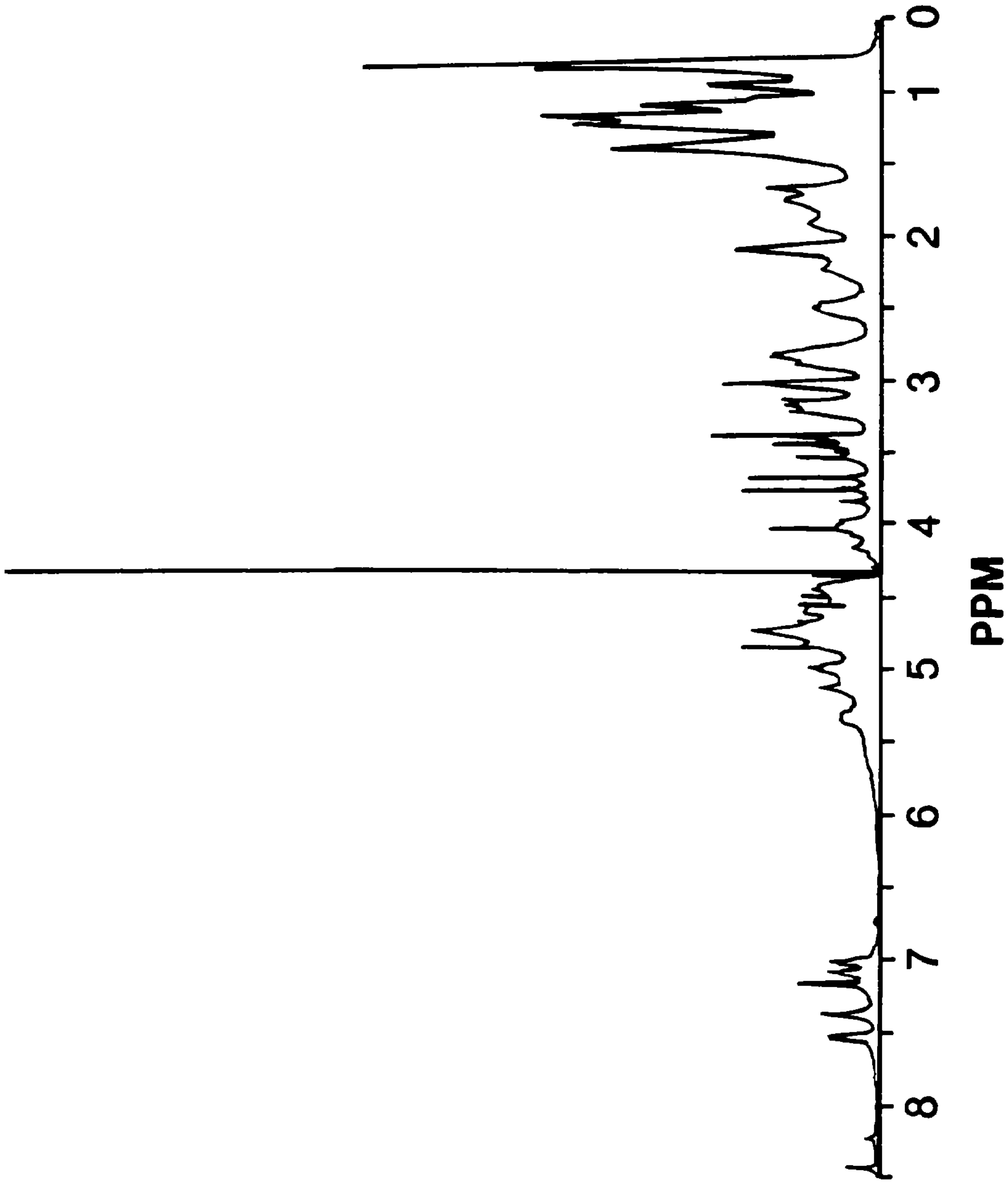

FIG.3

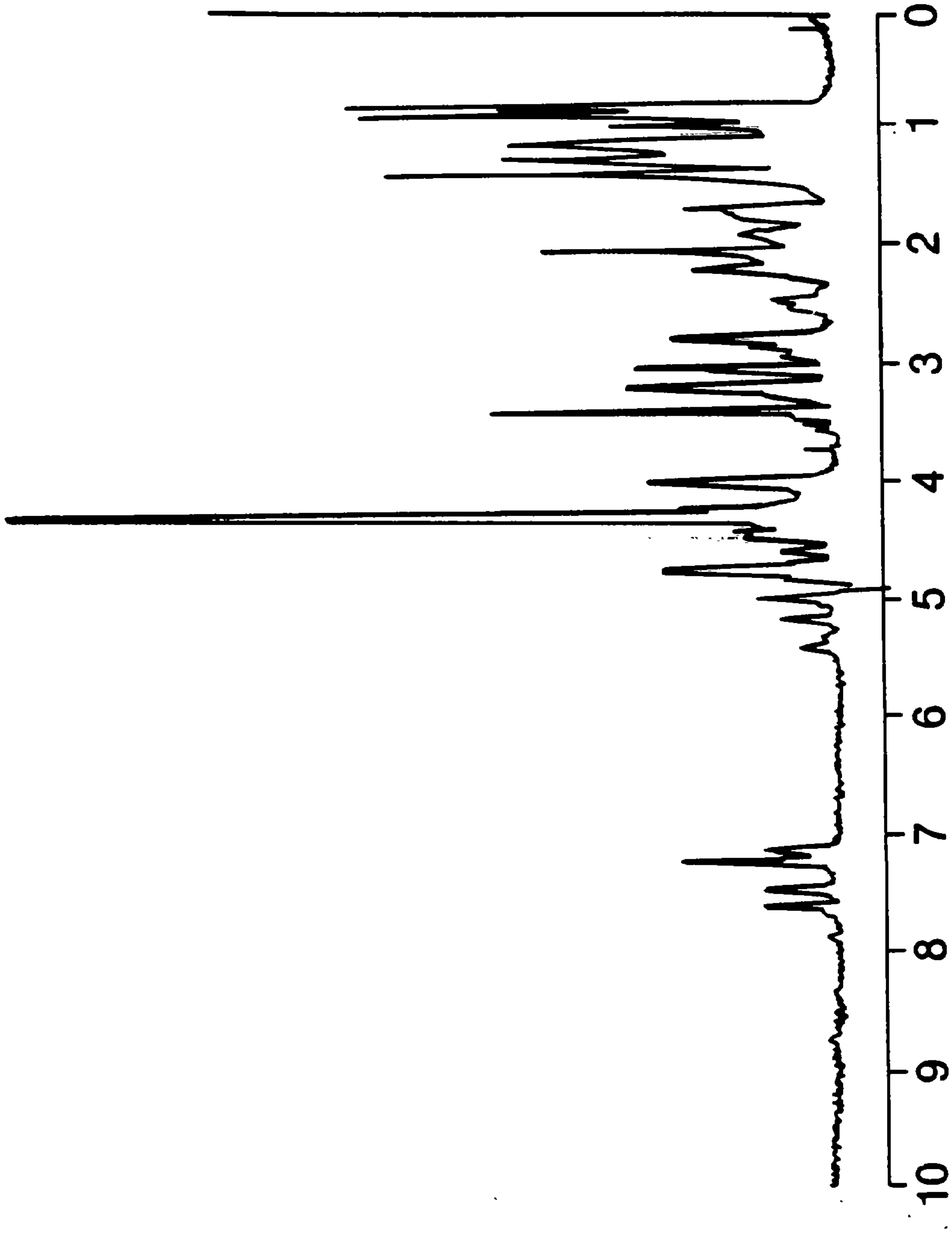

FIG.4

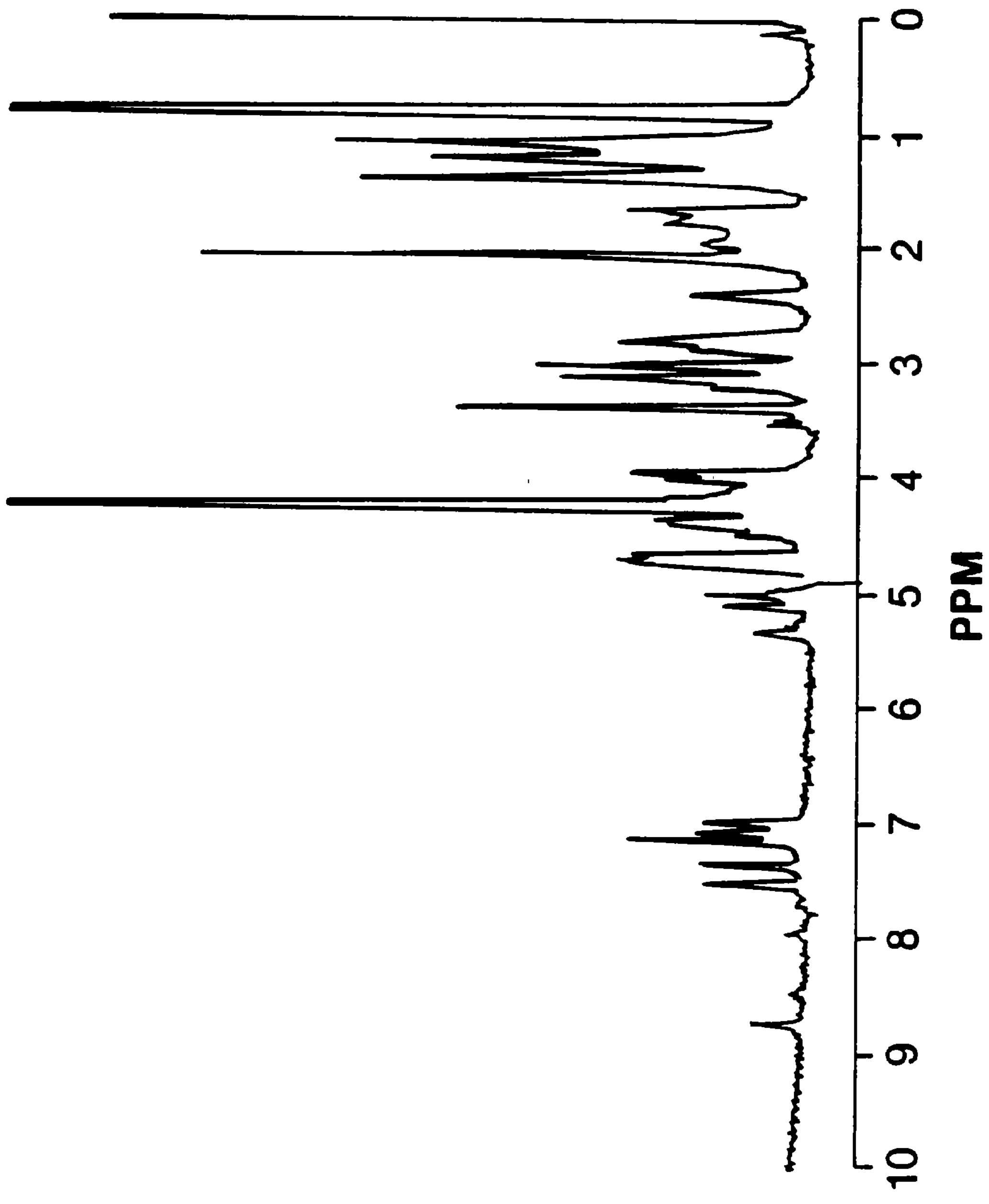

FIG.5

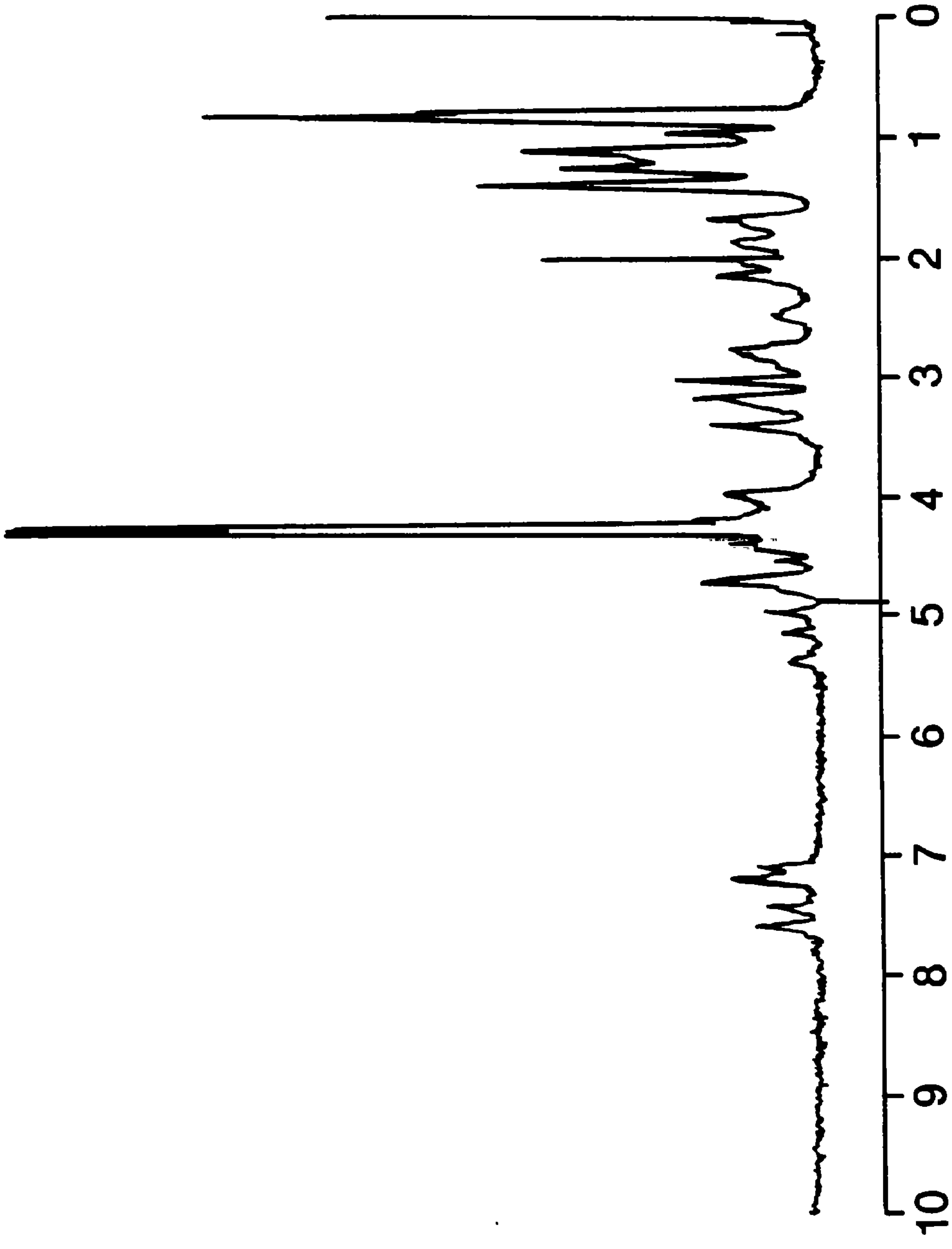

FIG.6

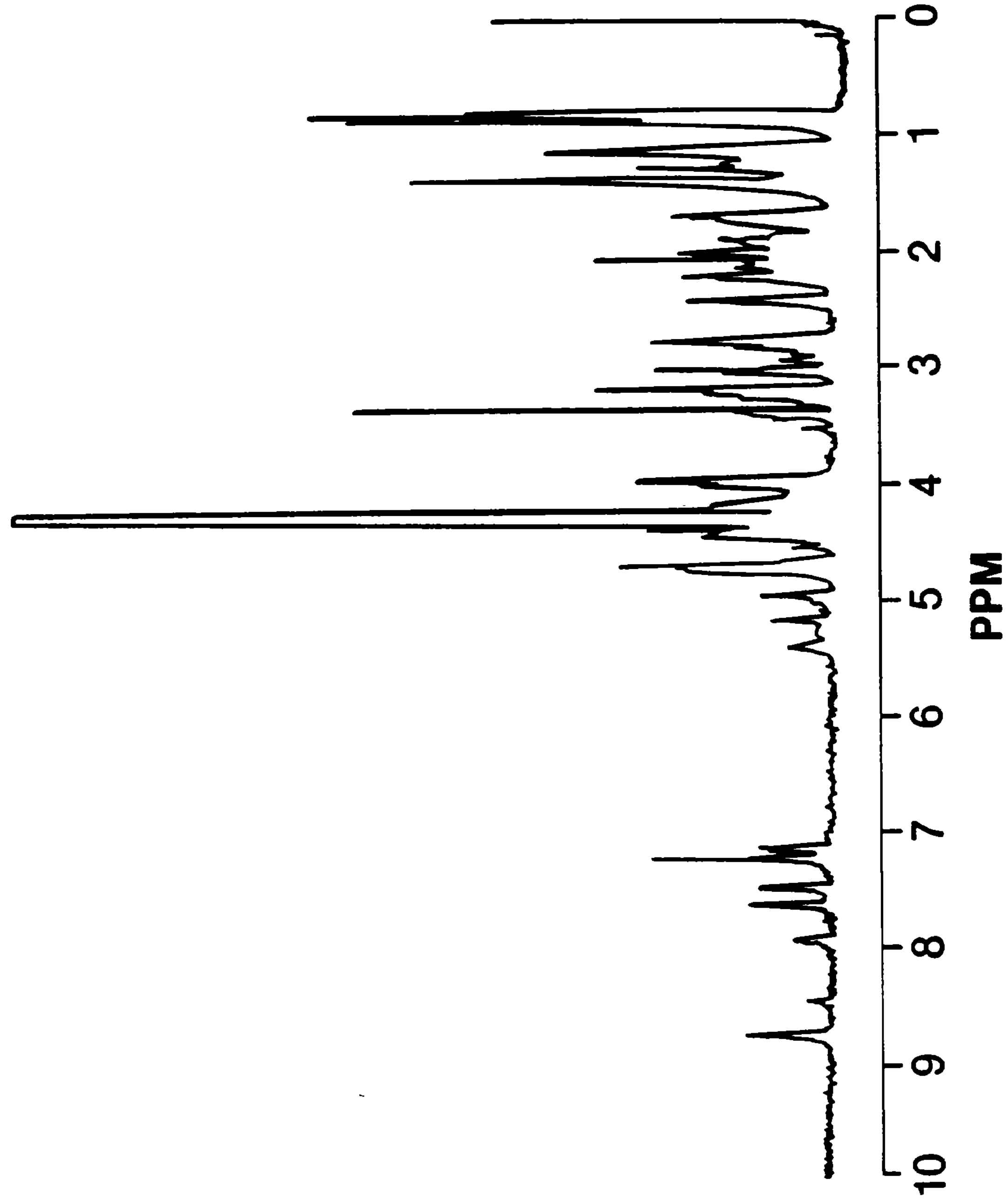

FIG. 7

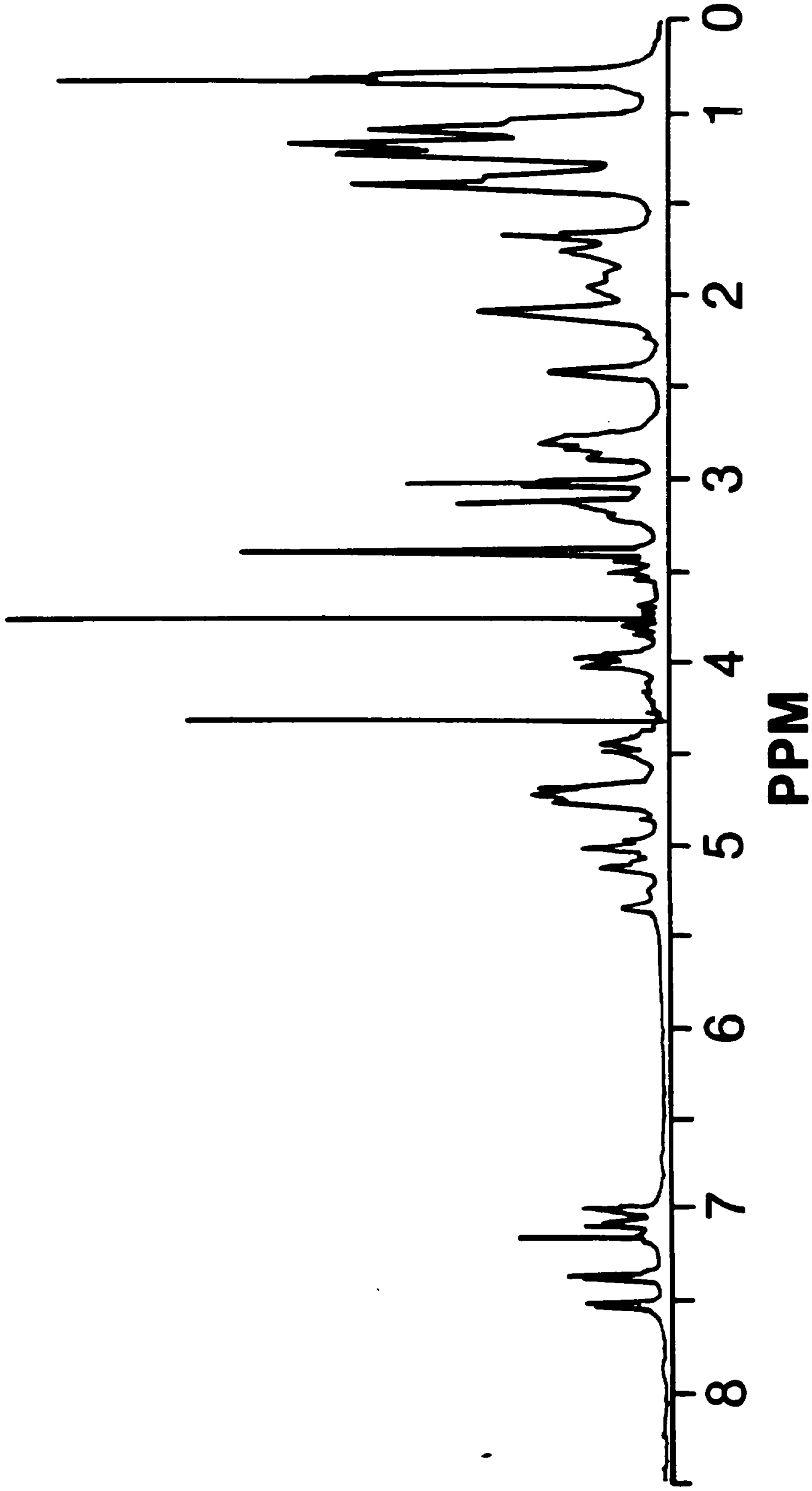

FIG.8

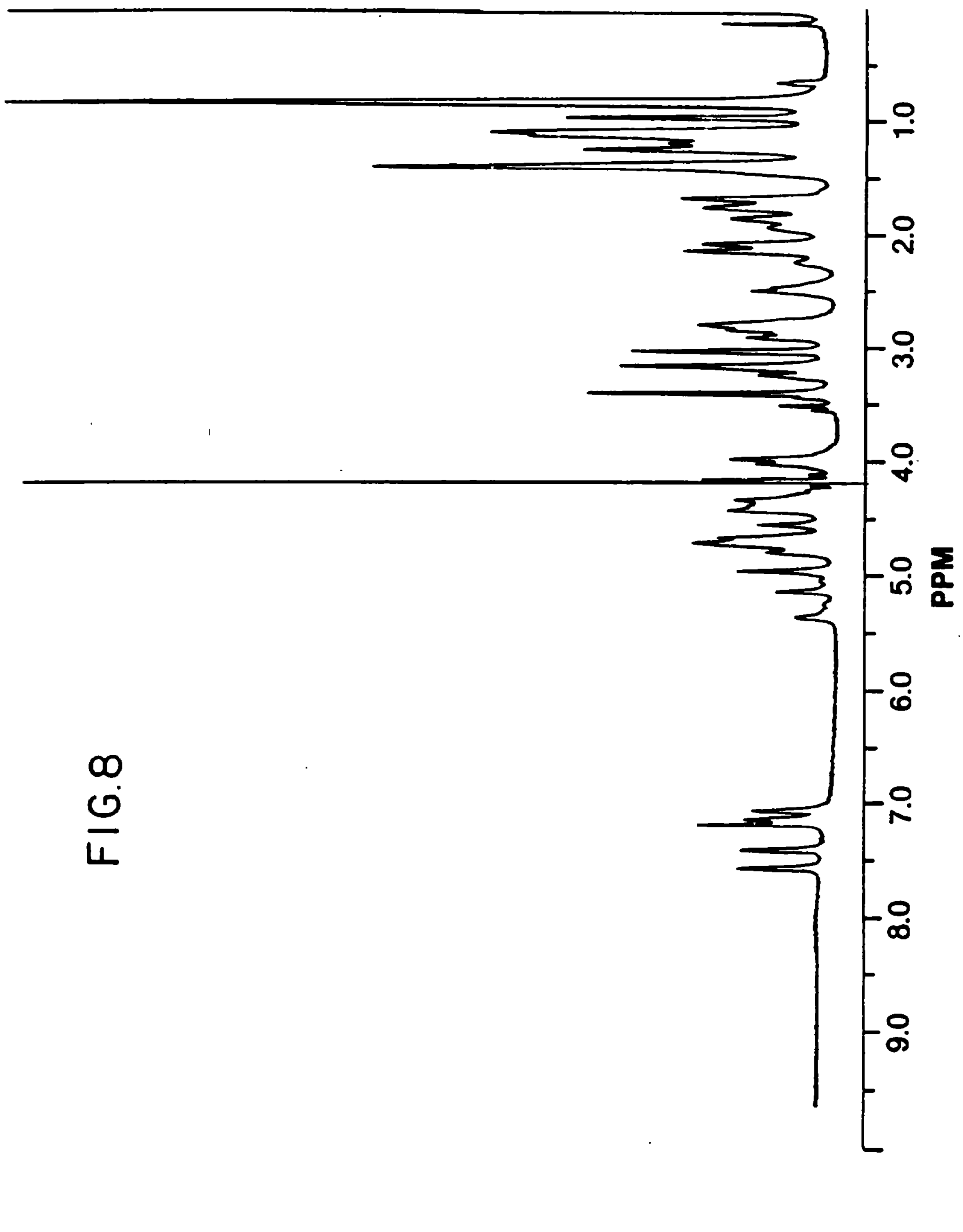

FIG.9

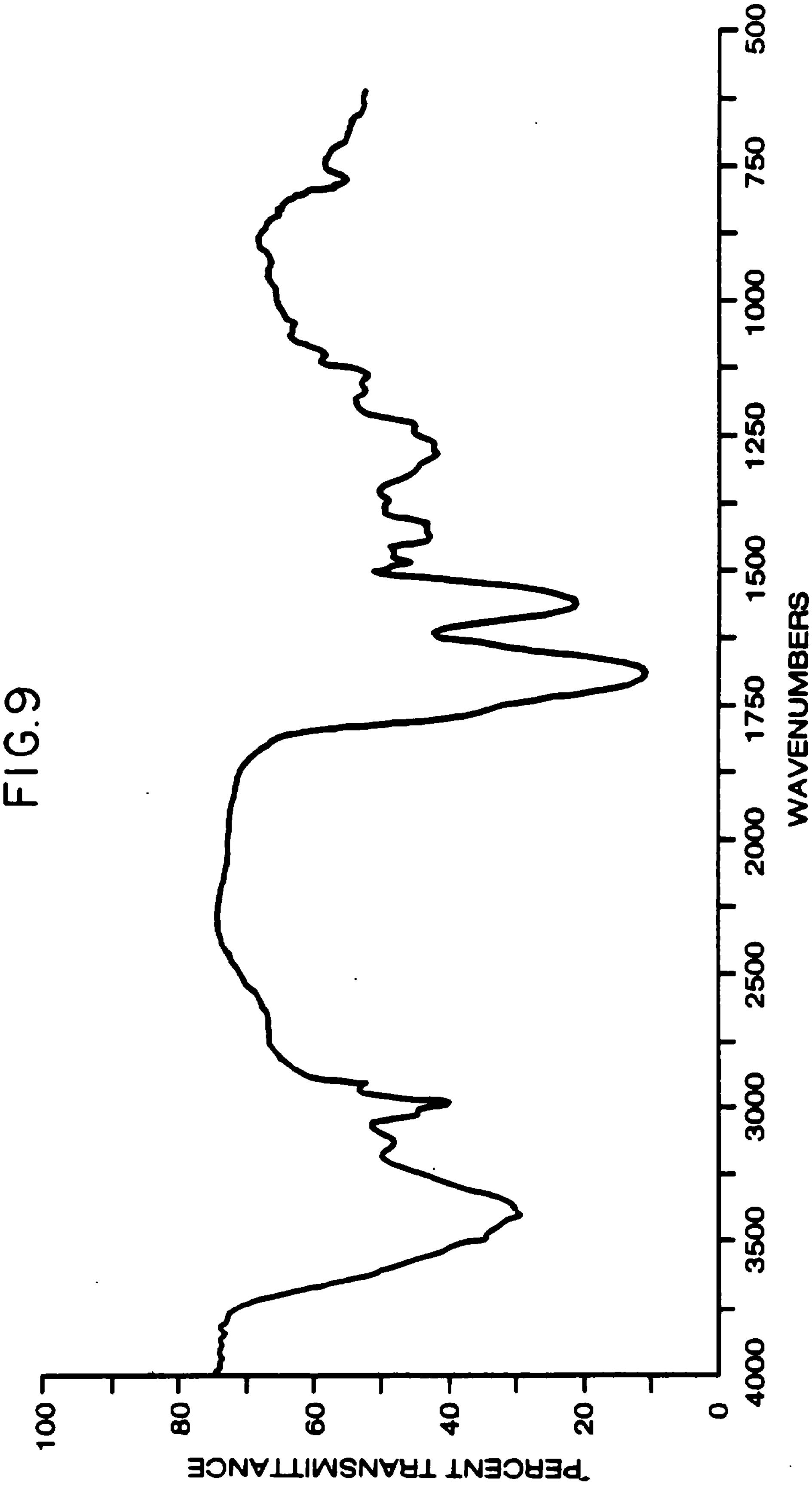
PERCENT TRANSMITTANCE
100
80
60
40
20
0
WAVENUMBERS
4000
3500
3000
2500
2000
1750
1500
1250
1000
750
500

FIG. 10

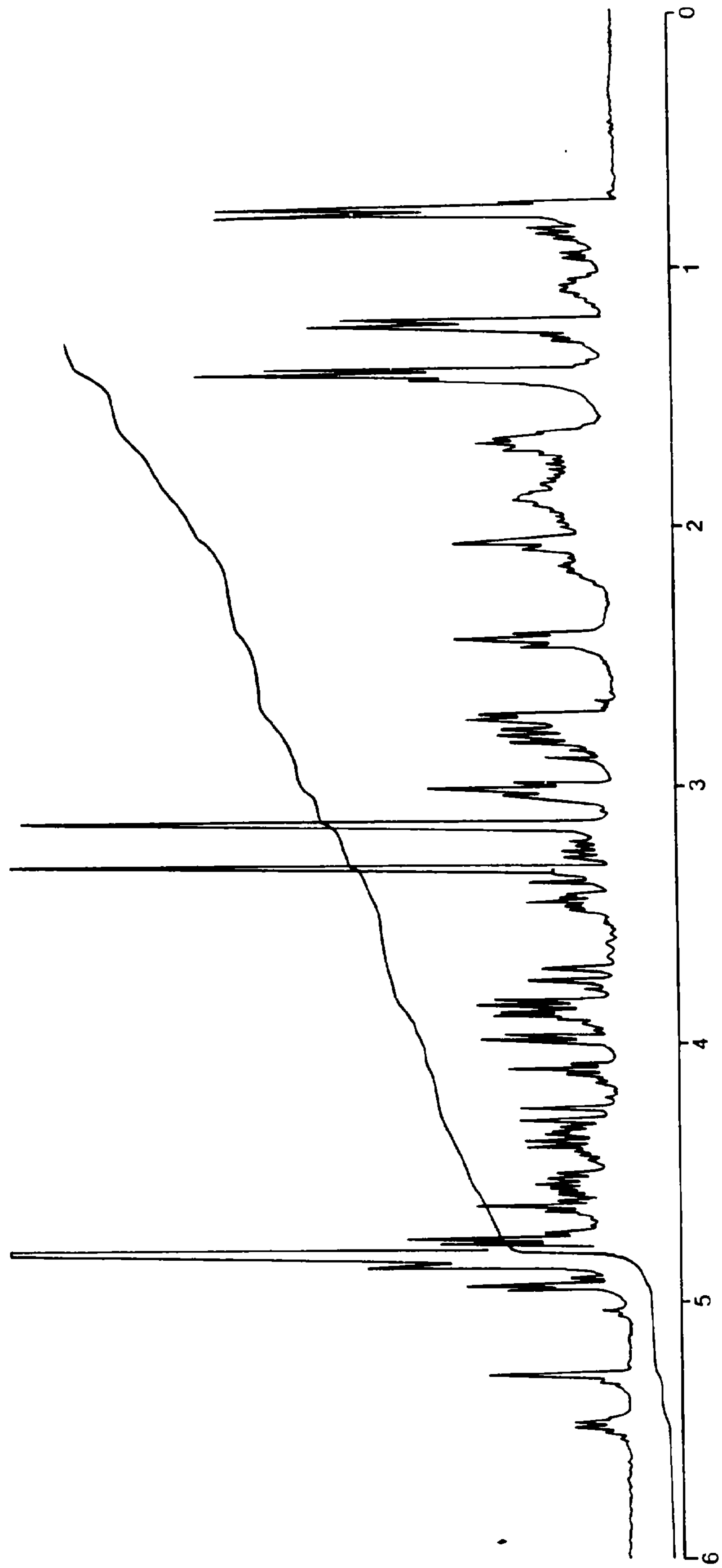

FIG.11

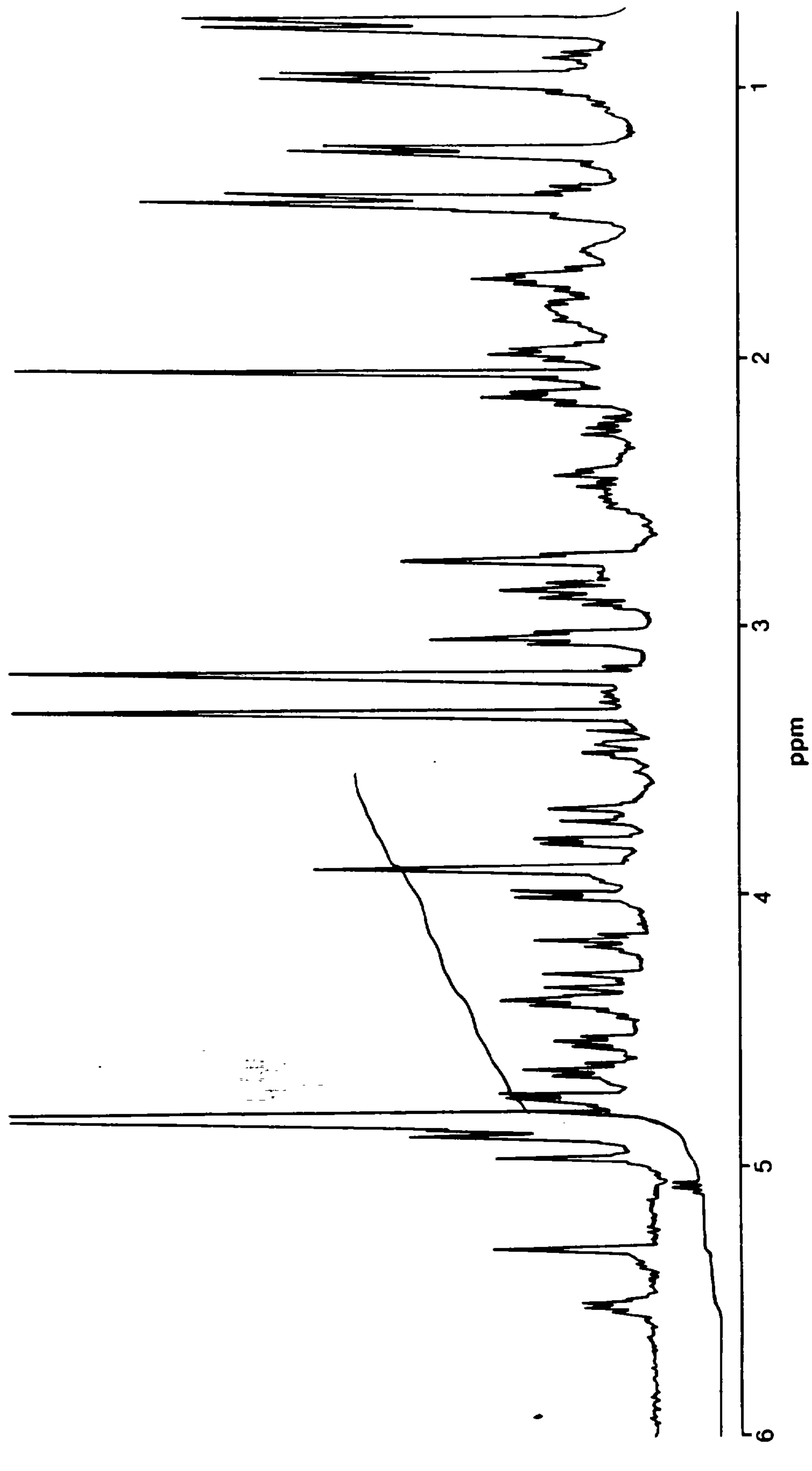

FIG.12

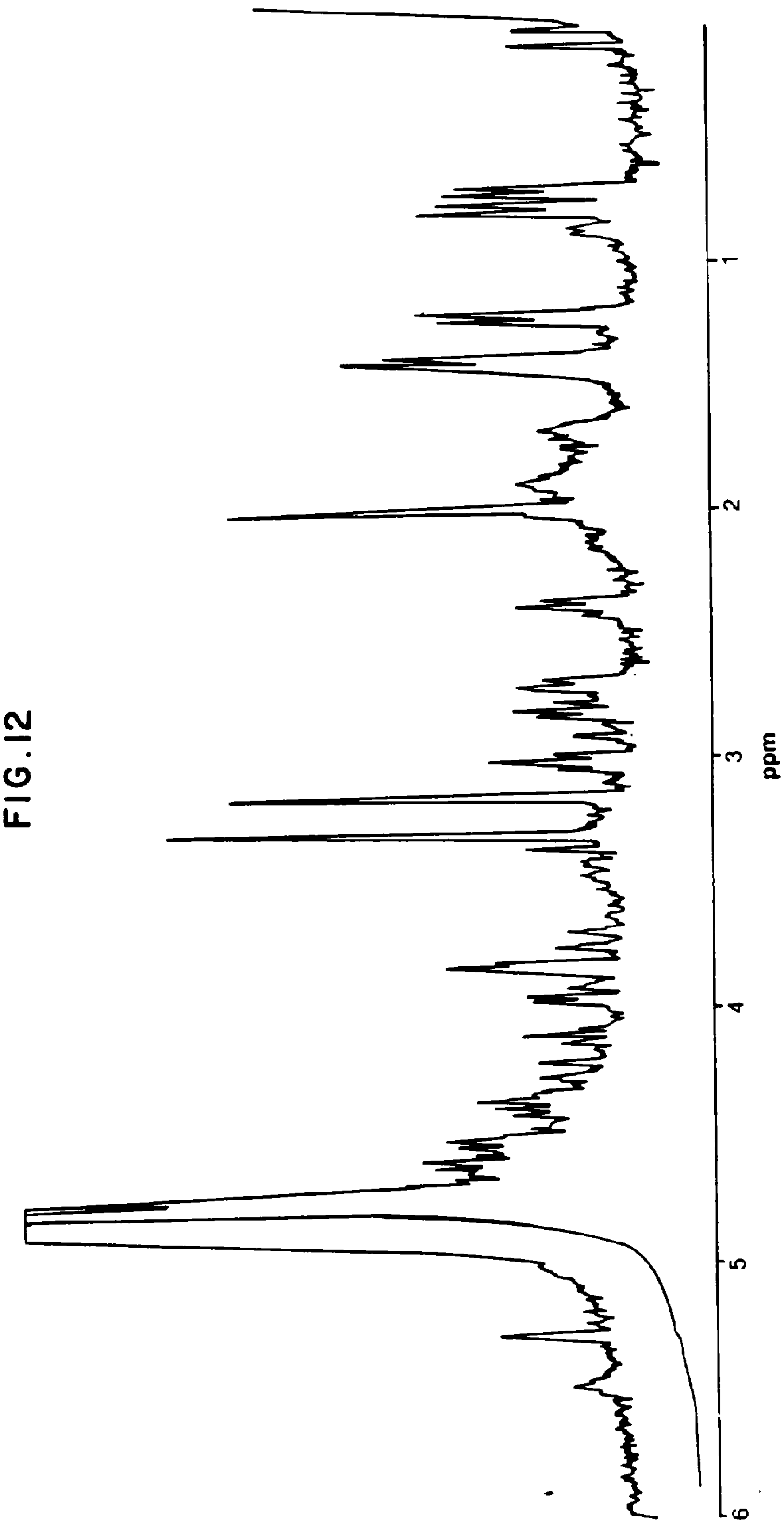

FIG.13

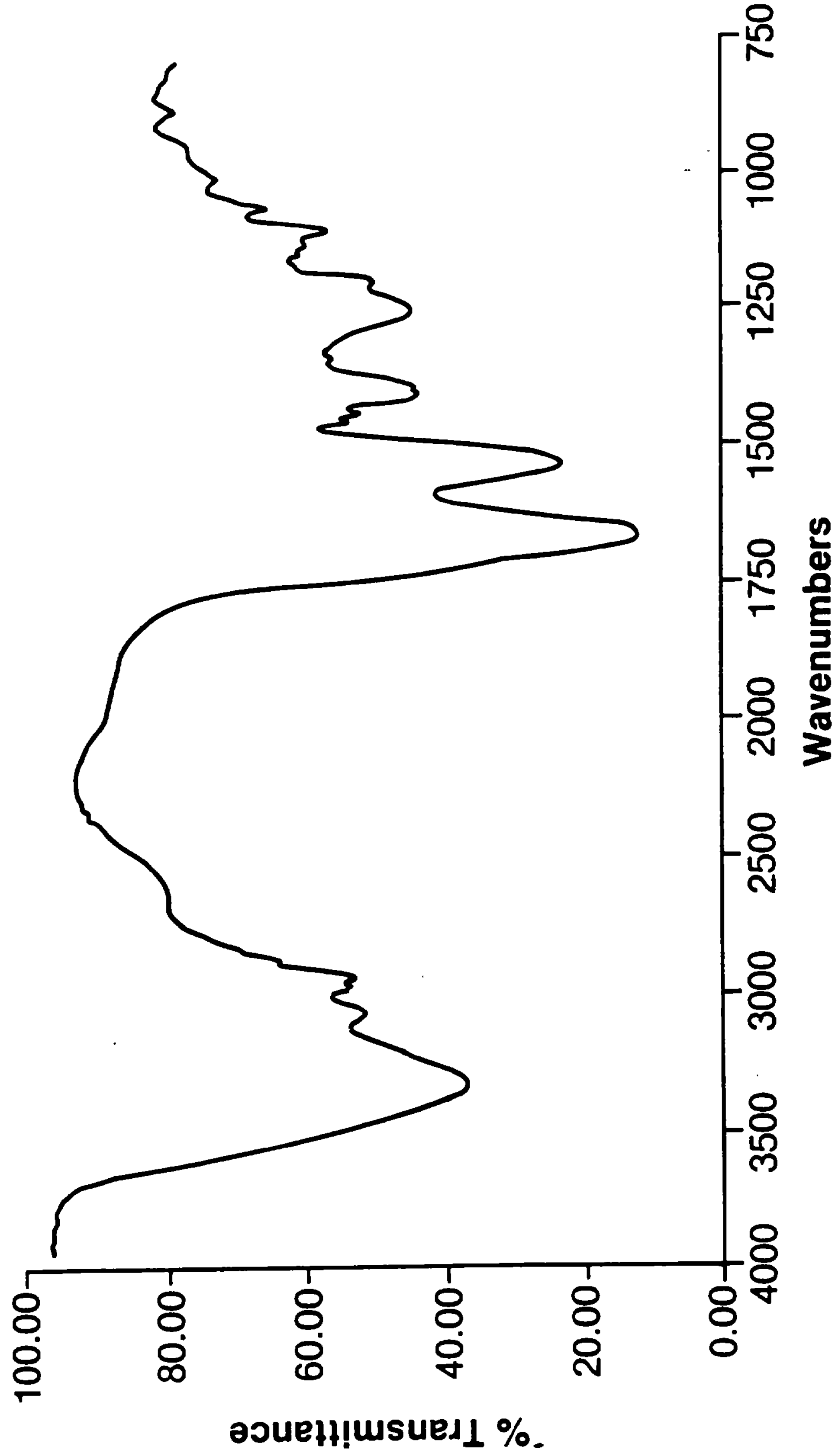

% Transmittance
100.00
80.00
60.00
40.00
20.00
0.00
4000
3500
3000
2500
2000
1750
1500
1250
1000
750
Wavenumbers